# EUROPEAN PATENT SPECIFICATION

(11) **EP 1 646 400 B1**
(45) Date of publication and mention of the grant of the patent: **21.07.2010**
(21) Application number: 04722319.3
(22) Date of filing: 22.03.2004
(51) Int. Cl.: A61K 39/00, C07K 14/47, A61P 37/06, A61P 37/08

(54) **TREATMENT OF ALLERGIC DISEASES USING A MODULATOR OF THE NOTCH SIGNALING PATHWAY**
BEHANDLUNG VON ALLERGISCHEN ERKRANKUNGEN UNTER VERWENDUNG EINES MODULATORS DES NOTCH SIGNALING PATHWAY
TRAITEMENT DE MALADIES ALLERGIQUES UTILISANT UN MODULATEUR DE LA VOIE DE SIGNALISATION NOTCH

(30) Priority: 21.03.2003 GB 0306583; 21.03.2003 GB 0306582; 22.03.2003 GB 0306621; 22.03.2003 GB 0306622; 22.03.2003 GB 0306626; 22.03.2003 GB 0306624; 22.03.2003 GB 0306640; 22.03.2003 GB 0306644; 22.03.2003 GB 0306650; 22.03.2003 GB 0306651; 22.03.2003 GB 0306654; 04.04.2003 WO PCT/GB03/01525; 24.05.2003 GB 0312062; 01.08.2003 WO PCT/GB03/03285; 03.10.2003 GB 0323130; 07.01.2004 WO PCT/GB2004/000046; 23.01.2004 WO PCT/GB2004/000263
(43) Date of publication of application: 19.04.2006
(73) Proprietor: Celldex Therapeutics Limited, Suite 54 Cambridge CB4 0EY (GB)
(72) Inventor: BODMER, Mark William, Lorantis Limited, Cambridge CB4 0PE (GB); BRIEND, Emmanuel Cyrille Pascal, Lorantis Limited, Cambridge CB4 OPE (GB); CHAMPION, Brian Robert, Lorantis Limited, Cambridge CB4 0PE (GB); LENNARD, Andrew Christopher, Lorantis Limited, Cambridge CB4 0PE (GB); MCKENZIE, Grahame James, Lorantis Limited, Cambridge CB4 0PE (GB); RAGNO, Silvia, Lorantis Limited, Cambridge CB4 0PE (GB); TUGAL, Tamara, Lorantis Limited, Cambridge CB4 0PE (GB); WARD, George Albert, Lorantis Limited, Cambridge CB4 OPE (GB); YOUNG, Lesley Lynn, Lorantis Limited, Cambridge CB4 0PE (GB)
(74) Representative: Mallalieu, Catherine Louise
(86) International application number: PCT/GB2004/001252
(87) International publication number: WO 2004/082710

(56) References cited:
- WO-A-00/36089
- WO-A-98/20142
- HOYNE G F ET AL: "SERRATE-1-INDUCED NOTCH SIGNALLING REGULATES THE DECISION BETWEEN IMMUNITY AND TOLERANCE MADE BY PERIPHERAL CD4+ T CELLS" INTERNATIONAL IMMUNOLOGY, OXFORD UNIVERSITY PRESS, GB, vol. 12, no. 2, 2000, pages 177-185, XP000929552 ISSN: 0953-8178
- WONG K K ET AL: "Notch ligation by Delta1 inhibits peripheral immune responses to transplantation antigens by a CD8+ cell-dependent mechanism" JOURNAL OF CLINICAL INVESTIGATION, NEW YORK, NY, US, vol. 112, no. 11, December 2003 (2003-12), pages 1741-1750, XP002268827 ISSN: 0021-9738
- ZLOBIN A ET AL: "TOWARD THE RATIONAL DESIGN OF CELL FATE MODIFIERS: NOTCH SIGNALING AS A TARGET FOR NOVEL BIOPHARMACEUTICALS" CURRENT PHARMACEUTICAL BIOTECHNOLOGY, BENTHAM SCIENCE PUBLISHERS, BOCA RATON,FL, US, vol. 1, no. 1, July 2000 (2000-07), pages 83-106, XP008004456 ISSN: 1389-2010
- OSBORNE B ET AL: "Notch and the immune system" IMMUNITY, CELL PRESS, US, vol. 11, no. 6, December 1999 (1999-12), pages 653-663, XP002217474 ISSN: 1074-7613
- HICKS C ET AL: "A SECRETED DELTA1-FC FUSION PROTEIN FUNCTIONS BOTH AS AN ACTIVATOR AND INHIBITOR OF NOTCH1 SIGNALING" JOURNAL OF NEUROSCIENCE RESEARCH, WILEY-LISS, US, vol. 68, no. 6, 15 June 2002 (2002-06-15), pages 655-667, XP009013890 ISSN: 0360-4012

## Description

### Field of the invention

The present invention relates to the modulation of immune function.

### Background of the invention

International Patent Publication No WO 98/20142 describes how manipulation of the Notch signalling pathway can be used in immunotherapy and in the prevention and/or treatment of T-cell mediated diseases. In particular, allergy, autoimmunity, graft rejection, tumour induced aberrations to the T-cell system and infectious diseases caused, for example, by Plasmodium species, Microfilariae, Helminths, Mycobacteria, HIV, Cytomegalovirus, Pseudomonas, Toxoplasma, Echinococcus, Haemophilus influenza type B, measles, Hepatitis C or Toxicara, may be targeted.

It has also been shown that it is possible to generate a class of regulatory T cells which are able to transmit antigen-specific tolerance to other T cells, a process termed infectious tolerance (WO98/20142). The functional activity of these cells can be mimicked by over-expression of a Notch ligand protein on their cell surfaces or on the surface of antigen presenting cells. In particular, regulatory T cells can be generated by over-expression of a member of the Delta or Serrate family of Notch ligand proteins.

A description of the Notch signalling pathway and conditions affected by it may be found, for example, in our published PCT Applications as follows:
PCT/GB97/03058 (filed on 6 November 1997 and published as WO 98/20142; claiming priority from GB 9623236.8 filed on 7 November 1996, GB 9715674.9 filed on 24 July 1997 and GB 9719350.2 filed on 11 September 1997);
PCT/GB99/04233 (filed on 15 December 1999 and published as WO 00/36089; claiming priority from GB 9827604.1 filed on 15 December 1999);
PCT/GB00/04391 (filed on 17 November 2000 and published as WO 0135990; claiming priority from GB 9927328.6 filed on 18 November 1999);
PCT/GB01/03503 (filed on 3 August 2001 and published as WO 02/12890; claiming priority from GB 0019242.7 filed on 4 August 2000);
PCT/GB02/02438 (filed on 24 May 2002 and published as WO 02/096952; claiming priority from GB 0112818.0 filed on 25 May 2001);
PCT/GB02/03381 (filed on 25 July 2002 and published as WO 03/012111; claiming priority from GB 0118155.1 filed on 25 July 2001);
PCT/GB02/03397 (filed on 25 July 2002 and published as WO 03/012441; claiming priority from GB0118153.6 filed on 25 July 2001, GB0207930.9 filed on 5 April 2002, GB 0212282.8 filed on 28 May 2002 and GB 0212283.6 filed on 28 May 2002);
PCT/GB02/03426 (filed on 25 July 2002 and published as WO 03/011317; claiming priority from GB0118153.6 filed on 25 July 2001, GB0207930.9 filed on 5 April 2002, GB 0212282.8 filed on 28 May 2002 and GB 0212283.6 filed on 28 May 2002);
PCT/GB02/04390 (filed on 27 September 2002 and published as WO 03/029293; claiming priority from GB 0123379.0 filed on 28 September 2001);
PCT/GB02/05137 (filed on 13 November 2002 and published as WO 03/041735; claiming priority from GB 0127267.3 filed on 14 November 2001, PCT/GB02/03426 filed on 25 July 2002, GB 0220849.4 filed on 7 September 2002, GB 0220913.8 filed on 10 September 2002 and PCT/GB02/004390 filed on 27 September 2002);
PCT/GB02/05133 (filed on 13 November 2002 and published as WO 03/042246; claiming priority from GB 0127271.5 filed on 14 November 2001 and GB 0220913.8 filed on 10 September 2002).

Reference is made also to Hoyne G.F. (1999) Int Arch Allergy Immunol 118:122-124; Hoyne et al. (2000) Immunology 100:281-288; Hoyne G.F. et al (2000) Intl Immunol 12:177-185; Hoyne, G. et al. (2001) Immunological Reviews 182:215-227),.

The present invention seeks to provide further methods of modulating the immune system and in particular immune responses, particularly in the prevention and/or treatment of allergy.

### Statements of the Invention

According to a first aspect of the invention there is provided a product comprising i) a modulator of the Notch signalling pathway and ii) an allergen or antigenic determinant thereof, or a polynucleotide coding for an allergen or antigenic determinant thereof; as a combined preparation for simultaneous, contemporaneous, separate or sequential use for modulation of the immune system, wherein the modulator of the Notch signalling pathway comprises a Notch ligand comprising an N-terminal domain, a DSL domain and the first 3 only EGF repeats of Deltal, or a polynucleotide coding therefor.

Generally, the term "allergen" as used herein may also include allergen "bystander antigens".

According to a further aspect of the invention there is provided a combination of a modulator of the Notch signalling pathway and an allergen or antigenic determinant thereof or a polynucleotide coding for an allergen or antigenic determinant thereof; for simultaneous, contemporaneous, separate or sequential use in modulating the immune system, wherein the modulator of the Notch signalling pathway comprises a Notch ligand comprising an N-terminal domain, a DSL domain and the first 3 only EGF repeats of Deltal, or a polynucleotide coding therefor.

According to a further aspect of the invention there is provided a modulator of the Notch signalling pathway for use in modulating the immune system in simultaneous, contemporaneous, separate or sequential combination with an allergen or antigenic determinant thereof or a polynucleotide coding for an allergen or antigenic determinant thereof, wherein the modulator of the Notch signalling pathway comprises a Notch ligand comprising an N-terminal domain, a DSL domain and the first 3 only EGF repeats of Deltal, or a polynucleotide coding therefor.

According to a further aspect of the invention there is provided the use of a combination of a modulator of the Notch signalling pathway and an allergen or antigenic determinant thereof or a polynucleotide coding for an allergen or antigenic determinant thereof; in the manufacture of a medicament for modulation of the immune system, wherein the modulator of the Notch signalling pathway comprises a Notch ligand comprising an N-terminal domain, a DSL domain and the first 3 only EGF repeats of Deltal, or a polynucleotide coding therefor.

According to a further aspect of the invention there is provided the use of a modulator of the Notch signalling pathway in the manufacture of a medicament for modulation of the immune system in simultaneous, contemporaneous, separate or sequential combination with an allergen or antigenic determinant thereof or a polynucleotide coding for an allergen or antigenic determinant thereof, wherein the modulator of the Notch signalling pathway comprises a Notch ligand comprising an N-terminal domain, a DSL domain and the first 3 only EGF repeats of Deltal, or a polynucleotide coding therefor.

According to a further aspect of the invention there is provided a pharmaceutical kit comprising a modulator of the Notch signalling pathway and an allergen or antigenic determinant thereof or a polynucleotide coding for an allergen or antigenic determinant thereof, wherein the modulator of the Notch signalling pathway comprises a Notch ligand comprising an N-terminal domain, a DSL domain and the first 3 only EGF repeats of Deltal, or a polynucleotide coding therefor.

Preferably the modulation of the immune system comprises immunotherapy.

Preferably the modulation of the immune system comprises reducing the immune response to an allergen or antigenic determinant thereof.

Preferably the modulation of the immune system comprises modulation of T-cell activity, preferably peripheral T-cell activity, preferably Tr or Th cell activity.

Suitably the modulation of the immune system may comprise generating regulatory T-cells (Tregs) or increasing Treg activity in relation to an allergen or antigenic determinant thereof. Tregs may, for example, be Tr1 or Th3 regulatory T-cells.

Preferably the modulator of the Notch signalling pathway is an agent which activates the Notch signalling pathway (Notch signalling agonist), or a polynucleotide which codes for such an agent.

Preferably the modulator of the Notch signalling pathway is an agent which activates a Notch receptor, preferably a human Notch receptor (eg human Notch1, human Notch2, human Notch3 or human Notch4; ie preferably a Notch receptor agonist which term includes partial agonists), or a polynucleotide which codes for such an agent Preferably the Notch receptor is activated in immune cells, preferably T-cells, B-cells or APCs.

For example the modulator of Notch signalling may be an agent for Notch signalling transduction or an agent for Notch signalling activation.

In one embodiment the modulator of the Notch signalling pathway is not a Notch IC protease, and in in one embodiment may not be a modulator of presenilin-dependent gamma secretase activity. In a preferred embodiment the modulator of the Notch signalling pathway is not a cytokine.

Suitably the modulator of the Notch signalling pathway may comprise a fusion protein or a polynucleotide which codes for a fusion protein. For example, the modulator may be a fusion protein comprising a segment of a *Notch* ligand extracellular domain and an immunoglobulin F_{c} segment or a polynucleotide encoding such a fusion protein.

Suitably an activator of Notch signalling may be be in a multimerised form, and may preferably comprise a construct comprising at least 3, preferably at least 5, preferably at least 10, at least 20 or at least 30 modulators of Notch signalling, or in some embodiments as many as 50 or 100 or 1000 or more modulators of Notch signalling, which may each be the same or different.

For example, modulators of Notch signalling in the form of Notch ligand proteins/polypeptides coupled to particulate supports such as beads are described in WO 03/011317 (Lorantis;). and in Lorantis' co-pending International (PCT) application PCT/GB2003/001525 ( filed on 4 April 2003), (eg see in particular Examples 17, 18, 19 of PCT/GB2003/001525). Reference is made also to the applicant's co-pending International (PCT) Application No PCT/GB2004/000046 filed on 7 January 2004, in particular Example 26 therein disclosing Notch ligand fragment/microbead constructs.

Modulators of Notch signalling in the form of Notch ligand proteins/polypeptides coupled to polymer supports are described in Lorantis Ltd's co-pending PCT application PCT/GB2003/003285 (filed on 1 August 2003 claiming priority from GB 0218068.5), published as WO04/013179; (eg see in particular Example 5 therein disclosing a dextran conjugate).

Suitably the modulator of Notch signalling may be administered in a multimerised form. For example, in one embodiment the modulator of Notch signalling may be bound to a membrane or support. Suitably a plurality or multiplicity of modulators (for example at least 5) will be bound to the membrane or support. Such a membrane or support can be selected from those known in the art. In a preferred embodiment, the support is a particulate support matrix. In an even more preferred embodiment, the support is a bead. The bead may be, for example, a magnetic bead (e.g. as available under the trade name "Dynal") or a polymeric bead such as a Sepharose bead.

Suitably a modulator of Notch signalling for use in the present invention may comprise a protein or polypeptide comprising:
i) a Notch ligand DSL domain;
ii) 3 Notch ligand EGF domains;
iii) optionally all or part of a Notch ligand N-terminal domain; and
iv) optionally one or more heterologous amino acid sequences; or a polynucleotide coding therefor.

Suitably the protein or polypeptide may have at least 50%, preferably at least 70%, preferably at least 90%, for example at least 95% amino acid sequence similarity (or preferably sequence identity) to the following sequence, preferably along the entire length of the latter:

GVFELKLQEFVNKKGLLGNRNCCRGGAGPPPCACRTFFRVCLKHYQASVSPEPPCTYGS
AVTPVLGVDSFSLPDGGGADSAFSNPIRFPFGFTWPGTFSLIIEALHTDSPDDLATENP
ERLISRLATQRHLTVGEEWSQDLHSSGRTDLKYSYRFVCDEHYYGEGCSVFCRPRDDAF
GHFTCGERGEKVCNPGWKGPYCTEPICLPGCDEQHGFCDKPGECKCRVGWQGRYCDECI
RYPGCLHGTCQQPWQCNCQEGWGGLFCNQDLNYCTHHKPCKNGATCTNTGQGSYTCSCR
PGYTGATCELGIDEC

Where reference is made to % homology, similarity or identity, this preferably means that the relevant % homology, similarity or identity occurs over over a region of at least 50 nucleic acid bases or amino acids, preferably over a region of at least 100 nucleic acid bases or amino acids, and preferably over the entire length of the reference sequence.

In addition the modulator of the Notch signalling pathway may comprise, for example, Notch intracellular domain (Notch IC) or a fragment, derivative, homologue, analogue or allelic variant thereof, or a polynucleotide sequence which codes for Notch intracellular domain or a fragment, derivative, homologue, analogue or allelic variant thereof.

Suitably the modulator of the Notch signalling pathway comprises Delta1 (suitably human Delta-1) or a fragment, derivative, homologue, analogue or allelic variant thereof or a polynucleotide encoding Delta1 or a fragment, derivative, homologue, analogue or allelic variant thereof.

In addition a modulator of the Notch signalling pathway may comprise Serrate/Jagged (suitably human Jagged-1 or Jagged-2) or a fragment, derivative, homologue, analogue or allelic variant thereof or a polynucleotide encoding Serrate/Jagged or a fragment, derivative, homologue, analogue or allelic variant thereof.

In addition a modulator of the Notch signalling pathway may comprise Notch or a fragment, derivative, homologue, analogue or allelic variant thereof or a polynucleotide encoding Notch or a fragment, derivative, homologue, analogue or allelic variant thereof.

In addition a modulator of the Notch signalling pathway may comprise a dominant negative version of a Notch signalling repressor, or a polynucleotide which codes for a dominant negative version of a Notch signalling repressor.

In addition a modulator of the Notch signalling pathway may comprise a polypeptide capable of upregulating the expression or activity of a Notch ligand or a downstream component of the Notch signalling pathway, or a polynucleotide which codes for such a polypeptide.

In one embodiment a modulator of the Notch signalling pathway may comprise an antibody, antibody fragment or antibody derivative or a polynucleotide which codes for an antibody, antibody fragment or antibody derivative.

According to a further aspect of the invention there is provided the use of the modulator of the Notch signalling pathway in the manufacture of a medicament for the treatment of allergy.

According to a further aspect of the invention there is provided a method for producing a lymphocyte or antigen presenting cell (APC) capable of promoting tolerance to an allergen or antigenic determinant thereof which method comprises incubating a lymphocyte or APC obtained from a human or animal patient with (i) the modulator of the Notch signalling pathway and (ii) an allergen or antigenic determinant thereof or a polynucleotide coding for an allergen or antigenic determinant thereof.

Suitably the method comprises incubating a lymphocyte or APC obtained from a human or animal patient with an APC in the presence of (i) the modulator of the Notch signalling pathway and (ii) an allergen or antigenic determinant thereof or a polynucleotide coding for an allergen or antigenic determinant thereof.

According to a further aspect of the invention there is provided a method for producing an APC capable of inducing in a T cell tolerance to an allergen or antigenic determinant thereof which method comprises contacting an APC with (i) the modulator of the Notch signalling pathway and (ii) an allergen or antigenic determinant thereof or a polynucleotide coding for an allergen or antigenic determinant thereof.

According to a further aspect of the invention there is provided a method for producing a T cell capable of promoting tolerance to an allergen or antigenic determinant thereof which method comprises incubating an antigen presenting cell (APC) simultaneously or sequentially, in any order, with:
(i) an allergen or antigenic determinant thereof or a polynucleotide coding for an allergen or antigenic determinant thereof;
(ii) the modulator of the Notch signalling pathway; and
(iii) a T cell obtained from a human or animal patient.

According to a further aspect of the invention there is provided a method for producing a lymphocyte or APC capable of promoting tolerance to an allergen or antigenic determinant thereof which method comprises incubating a lymphocyte or APC obtained from a human or animal patient with a lymphocyte or APC produced as described above.

Suitably in such methods the lymphocyte or APC may be incubated *ex-vivo*.

According to a further aspect of the invention there is provided a method of promoting tolerance to an allergen or antigenic determinant thereof which method comprises administering to the patient a lymphocyte or APC produced by a method as described above.

The term "APC" as used herein, includers any vehicle capable of presenting the desired *Notch*-ligand to the T cell population Examples of suitable APCs include dendritic cells, L cells, hybridomas, lymphomas, macrophages, B cells or synthetic APCs such as lipid membranes.

When the APCs are transfected with a gene capable of expressing a *Notch*-ligand, the transfection may be brought about by a virus such as a retrovirus or adenovirus, or by any other vehicle or method capable of delivering a gene to the cells. These include any vehicles or methods shown to be effective in gene therapy and include retroviruses, liposomes, electroporation, other viruses such as adenovirus, adeno-associated virus, herpes virus, vaccinia, calcium phosphate precipitated DNA, DEAE dextran assisted transfection, microinjection, polyethylene glycol, protein-DNA complexes.

Using such vehicles or methods alone or in combination it is possible to site-direct the gene delivery to a particular population of cells, thus enabling the method of the present invention to be performed in vivo. For example, a virus may be used in combination with liposomes in order to increase the efficiency of DNA uptake. The site specificity of the delivery may be achieved by the inclusion of specific proteins (eg a single chain antibody reactive with CD11c for dendritic cells/macrophages) in the viral coat or liposome.

In one emdodiment of the present invention the allergen may be a pollen allergen or antigenic determinant thereof, or bystander antigen or antigenic determinant, or a polynucleotide coding therefor may be used.

In one emdodiment of the present invention the allergen may be a mite allergen, such as a dust mite allergen or antigenic determinant thereof, or bystander antigen or antigenic determinant, or a polynucleotide coding therefor may be used.

In one emdodiment of the present invention the allergen may be an animal dander allergen or antigenic determinant thereof, or bystander antigen or antigenic determinant, or a polynucleotide coding therefor may be used.

In one emdodiment of the present invention the allergen may be a latex allergen or antigenic determinant thereof, or bystander antigen or antigenic determinant, or a polynucleotide coding therefor may be used.

### Detailed description

Various preferred features and embodiments of the present invention will now be described in more detail by way of non-limiting example and with reference to the accompanying Figures, in which:
Figure 1 shows a schematic representation of the Notch signalling pathway;

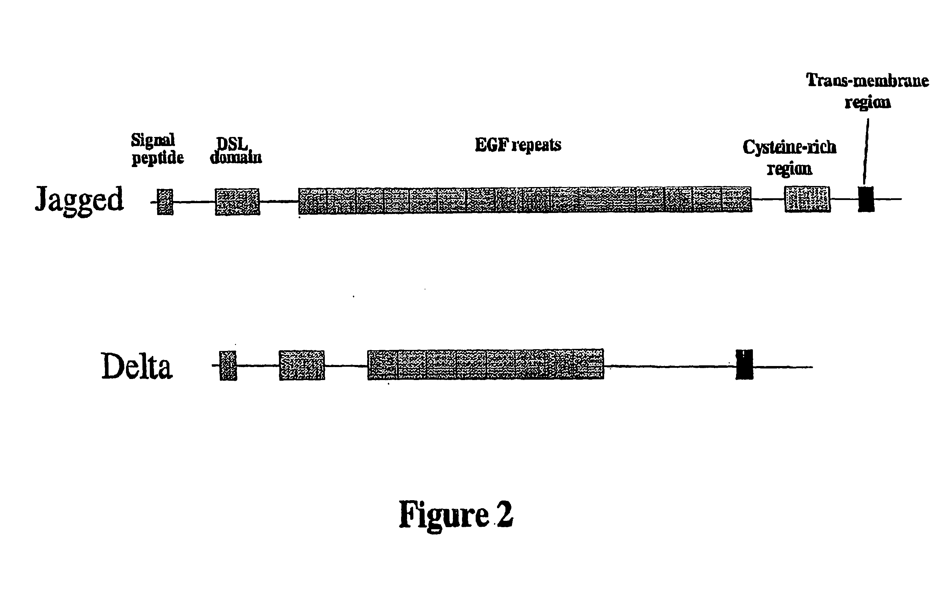

Figure 2 shows schematic representations of the Notch ligands Jagged and Delta;
Figure 3 shows an example of a nucleotide vector according to one embodiment of the present invention;
Figure 4 shows aligned amino acid sequences of DSL domains from various Drosophila and mammalian Notch ligands;

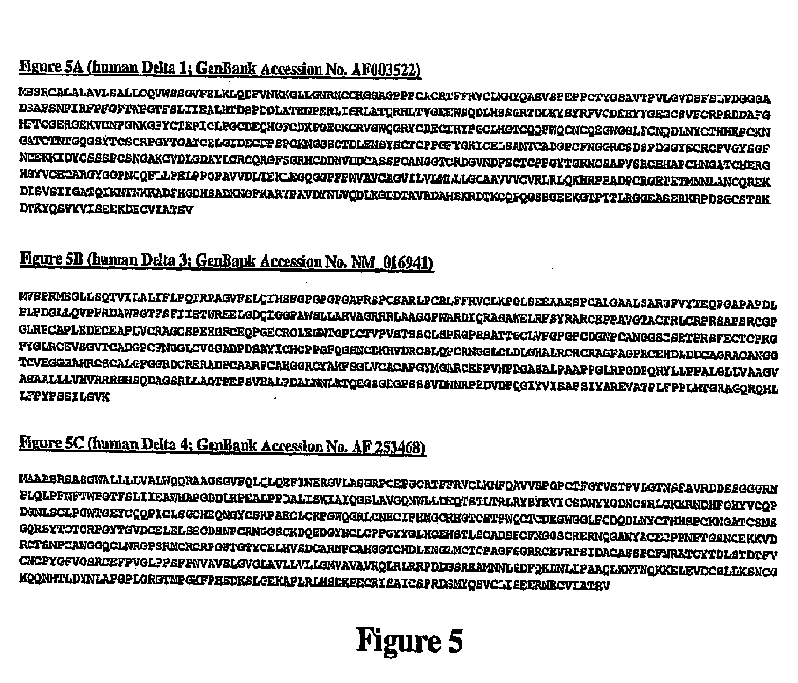

Figure 5 shows amino acid sequences of human Delta-1, Delta-2 and Delta-3; and

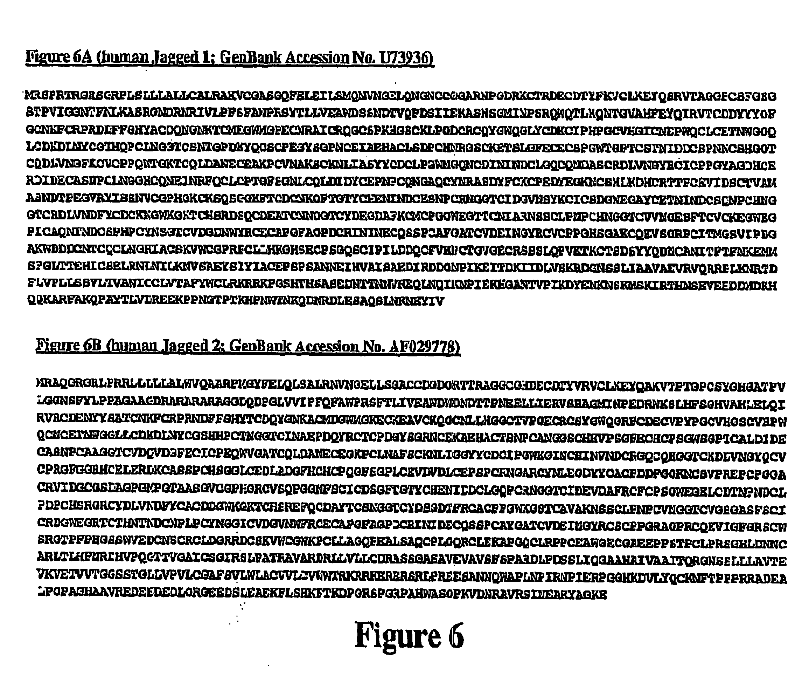

Figure 6 shows amino acid sequences of human Jagged-1 and Jagged-2;
Figure 7 shows schematic representations of fusion proteins which may be used in the present invention.
Figure 8 shows results from Example 9;
Figure 9 shows results from Example 11; and
Figure 10 shows results from Example 12.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and J. E. Coligan, A. M. Kruisbeek, D. H. Margulies, E. M. Shevach and W. Strober (1992 and periodic supplements; Current Protocols in Immunology, John Wiley & Sons, New York, NY).

For the avoidance of doubt, Drosophila and vertebrate names for genes and proteins are used interchangeably and all homologues are included within the scope of the invention.

### Allergens and antigenic determinants thereof

The term "allergen" as used herein means any substance which can induce an allergic response, especially a type I hypersensitive response. Typical allergens include, but are not limited to, pollens, molds, foods, animal danders or their excretions, smuts and insects, their venoms or their excretions. Allergens may, for example, be natural or synthetic organic molecules such as peptides/proteins, polysaccharides or lipids. They may be administered singly or as a mixture. Allergens may be chemically or physically modified. Such modified allergens, or allergen derivatives, are known in the art. Examples include, but are not limited to, peptide fragments, conjugates or polymerized allergen derivatives. Thus, the term "allergen" as used herein includes naturally occurring (native) allergens as well as any biologically active fragment, derivative, homologue or variant thereof or any antigenic determinant or epitope (especially immunodominant epitope) thereof or any polynucleotide coding for an allergen (including any biologically active fragment, derivative, homologue or variant) or antigenic determinant or epitope (especially immunodominant epitope) thereof.

The amount of allergen to be administered can be determined empirically and depends on the sensitivity of the individual as well as the desired clinical result. Generally, a regimen of desensitization initially involves the periodic administration of smaller amounts of allergen, which level is increased over the course of the regimen until a predetermined (planned) upper limit is reached or the individual can tolerate exposure to such allergen without a significant adverse allergic response. The particular regimen often is tailored to individual patient needs. The embodiment and potential advantage of the present invention is that it may be possible to meaningfully decrease the level of allergens administered and/or the number of injections and, thereby, the length of the desensitization regimen. Further, with a meaningful decrease of the level (dose) of allergen administered to particularly sensitive individuals, there is a possible diminished risk of severe allergic reaction to the administration of the allergen.

The progress of immunotherapy can be monitored by any clinically acceptable diagnostic tests. Such tests are well known in the art and include symptom levels and requirement levels for ancillary therapy recorded in a daily diary, as well as skin testing and in vitro serological tests for specific IgE antibody and/or specific IgG antibody.

The antigen or antigenic determinant may also be a "bystander antigen" or antigenic determinant thereof.

The term "allergen bystander antigen" herein preferably means an antigen presented as part of an immune disease process, preferably being presented in an affected disease locus (eg organ or tissue) or lymphatic tissues draining this locus, together with a target antigen, whether or not the bystander antigen contributes significantly to an unwanted or overly severe immune response.

In one embodiment the "bystander antigen" is not the or a primary causative antigen of the relevant allergic state and may not itself contribute significantly to unwanted or overly severe immune response, but is frequently present at the site of that response (disease locus) as a "bystander".

Alternatively, the bystander antigen may be an exogenous (foreign) antigen or antigenic determinant (eg KLH or any other suitable exogenous antigen) that is delivered to the affected target tisse (eg by direct physical introduction, such as by injection or other such means, or targeted with an agent which concentrates it at the requires site, such as an antibody specific for an antigen present at the target site) to trigger suppressive immune cells (preferably T-cells, preferably regulatory T-cells) in the target tissue or lymphatic tissues draining this tissue.

The term "bystander antigen" as used herein thus includes any substance capable of eliciting an immune response, including proteins, protein fragments, polypeptides, peptides, glycoproteins, nucleic acids, polysaccharides or any other immunogenic substance that is present in the disease locus in an allergic condition.

The present invention may be used for preventing and treating all forms of allergy and allergic disorder, including without limitation: ophthalmic allergic disorders, including allergic conjunctivitis, vernal conjunctivitis, vernal keratoconjunctivitis, and giant papillary conjunctivitis; nasal allergic disorders, including allergic rhinitis and sinusitis; otic allergic disorders, including eustachian tube itching; allergic disorders of the upper and lower airways, including intrinsic and extrinsic asthma; allergic disorders of the skin, including dermatitis, eczema and urticaria; and allergic disorders of the gastrointestinal tract.

Any form of allergen (including any biologically active fragment, derivative, homologue or variant) or antigenic determinant or epitope (especially immunodominant epitope) thereof or any polynucleotide coding for an allergen (including any biologically active fragment, derivative, homologue or variant) or antigenic determinant or epitope (especially immunodominant epitope) thereof may be used, including but not limited to pollen allergens, mite allergens, animal dander allergens, latexes, food allergens, insect allergens (eg mite or cockroach allergens), fungal allergens, drug allergens and venom allergens and antigenic determinants or epitopes (especially immunodominant epitopes) thereof, for example:

### Pollen allergens and antigenic determinants thereof

### 1. Grass Pollen Antigens and Antigenic Determinants

### a. Ryegrass pollen antigens and antigenic determinants

For example, Lol p 1 (eg GenBank Accession No M57474), Lol p 1b (eg GenBank Accession No M59163), Lo1 p 2 (eg GenBank Accession No X73363), Lol p 2a (eg SwissProt Accession No P14947), Lol p 2b (eg PIR Accession No A48595), Lol p 3 (eg SwissProt Accession No P14948), Lo1 p 4 (eg PIR Accession No A60737), Lol p 5 (eg PIR Accession No S38288), Lol p 9 (eg GenBank Accession No L13083) or Lol p 11 (eg PIR Accession No A54002); and antigenic determinants thereof.

For example, an amino acid sequence for Lo1 p 1 is reported as follows (GenBank Accession No M57474):

MASSSSVLLVVALFAVFLGSAHGIAKVPPGPNITAEYGDKWLDAKSTWYGKPTGAGPKDNGGACGYKNVDK
APFNGMTGCGNTPIFKDGRGCGSCFEIKCTKPESCSGEAVTVTITDDNEEPIAPYHFDLSGHAFGSMAKKG
EEQNVRSAGELELQFRRVKCKYPDDTKPTFHVEKASNPNYLAILVKYVDGDGDVVAVDIKEKGKDKWTELK
ESWGAVWRIDTPDKLTGPFTVRYTTEGGTKSEFEDVIPEGWKADTSYSAK

Ryegrass (Lo1 p 1) allergens are also described in US Patents Nos 5,710,126; 6,180,368 B1; 6,239,269 B1; 6,197,313 B1; 6,451,324 B1; and 6,265,566 B1.

Ryegrass (Lo1 p 5/9) allergens are also described in US Patents Nos 5,840,316; 6,277,383 B1; 5,869,333; 5,721,119; 5,965,455; and 5,736,362.

### b. Timothy Grass pollen antigens and antigenic determinants

For example, Phl p 1 (eg GenBank Accession No X78813), Phl p 2 (eg GenBank Accession No X75925), Phl p 5 (eg GenBank Accession No Z27083), Phl p 5a (eg GenBank Accession No X70942), Phl p 5b (eg GenBank Accession No Z27083), Phl p 6 (eg GenBank Accession No Z27082), Phl p 11 (eg GenBank Accession No X77583), Phl p 32K (eg PIR Accession No S38294) and Phl p 38K (eg PIR Accession No S38293); and antigenic determinants thereof.

For example, an amino acid sequence for Phl p 1 is reported as follows (GenBank Accession No X78813):

MASSSSVLLVVVLFAVFLGSAYGIPKVPPGPNITATYGDKWLDAKSTWYGKPTGAGPKDNGGACGYKDVDK
PPFSGMTGCGNTPIFKSGRGCGSCFEIKCTKPEACSGEPVVVHITDDNEEPIAPYHFDLSGHAFGAMAKKG

DEQKLRSAGELELQFRRVKCKYPEGTKVTFHVEKGSNPNYLALLVKYVNGDGDVVAVDIKEKGKDKWIELK
ESWGAIWRIDTPDKLTGPFTVRYTTEGGTKTEAEDVIPEGWKADTSYESK

### c. Bent Grass pollen antigens and antigenic determinants

For example, Agr a 1 (eg PIR Accession No E37396); and antigenic determinants thereof.

### d. Bermuda Grass pollen antigens and antigenic determinants

For example, Cyn d 1 (eg PIR Accession No A61226) and Cyn d 2 (eg GenBank Accession No AJ131335); and antigenic determinants thereof. See also antigens described in US Patents Nos 6,441,157 B1 and 6,214,358 B1.

### e. Blue Grass pollen antigens and antigenic determinants

For example, Poa p 1 (eg PIR Accession No F37396), Poa p 2 (eg GenBank Accession No AJ131337) and Poa p 9 (eg GenBank Accession No M38342); and antigenic determinants thereof.

### f. Canary Grass pollen antigens and antigenic determinants

For example, Pha a 1 (eg SwissProt Accession No Q41260) Pha a 5.1 (eg SwissProt Accession No P56154) Pha a 5.2 (eg SwissProt Accession No P56165) Pha a 5.3 (eg SwissProt Accession No P56166) and Pha a 5.4 (eg SwissProt Accession No P56167); and antigenic determinants thereof.

### g. Orchard Grass pollen antigens and antigenic determinants

For example, Dac g 1 (eg PIR Accession No D58493) Dac g 2 (eg GenBank Accession No S45354) Dac g 3 (eg PIR Accession No A60359); and antigenic determinants thereof.

### h. Johnson Grass pollen antigens and antigenic determinants

For example, as described in US Patents Nos 5,480,972; 5,736,149; and 5,691,167.

### 2. Tree pollen antigens and antigenic determinants

### a. Birch pollen antigens and antigenic determinants

For example, Bet v 1a (eg GenBank Accession No X1 5877), Bet v 1b (eg GenBank Accession No X77200), Bet v 1c (eg GenBank Accession No X77265), Bet v 1d (eg GenBank Accession No X77266), Bet v 1e (eg GenBank Accession No X77267), Bet v 1f (eg GenBank Accession No X77268), Bet v 1g (eg GenBank Accession No X77269), Bet v 1j (eg (GenBank Accession No X77271), Bet v 1k (eg (GenBank Accession No X77272), Bet v 1L (eg GenBank Accession No X77273), Bet v 1m (eg GenBank Accession No X81972), Bet v 2 (eg GenBank Accession No M65179), Bet v 3 (eg GenBank Accession No X79267), or Bet v 4 (eg GenBank Accession No Y12560); and antigenic determinants thereof.

For example, an amino acid sequence for Bet v 1a is reported as follows (GenBank Accession No X15877):

MGVFNYETETTSVIPAARLFKAFILDGDNLFPKVAPQAISSVENIEGNGGPGTIKKISFPEGFPFKYVKDR
VDEVDHTNFKYNYSVIEGGPIGDTLEKISNEIKIVATPDGGSILKISNKYHTKGDHEVKAEQVKASKEMGE
TLLRAVESYLLAHSDAYN

### b. Chestnut tree pollen antigens and antigenic determinants

For example, Cas s 1 (eg PIR Accession No PC2001); and antigenic determinants thereof.

### c. Hornbeam tree pollen antigens and antigenic determinants

For example, Car b 1 (eg GenBank Accession No X66932); and antigenic determinants thereof.

### d. Oak tree pollen antigens and antigenic determinants

For example, Que a 1 (eg PIR Accession No D53288); and antigenic determinants thereof.

### e. Olive Tree pollen antigens and antigenic determinants

For example, Ole e 1 (eg GenBank Accession No S75766), Ole e 3 (eg GenBank Accession No AF015810), Ole e 4 (eg SwissProt Accession No P80741) Ole e 5 (eg SwissProt Accession No P80740) Ole e 6 (eg GenBank Accession No U86342); and antigenic determinants thereof.

### f. Japanese Cedar Pollen antigens and antigenic determinants

For as described in US Patent No 6,090,386.

### 3. Weed pollen antigens and antigenic determinants

### a. Ragweed (A. artemisiifloria)

For example, Amb a 1.1 (eg GenBank Accession No M80558), Amb a 1.2 (eg GenBank Accession No M80559), Amb a 1.3 (eg GenBank Accession No M62961), Amb a 1.4 (eg GenBank Accession No M80562), Amb a 2 (eg GenBank Accession No M80561) Amb a 3 (eg GenBank Accession No P00304) and Amb a 5 (eg SwissProt Accession No P02878); and antigenic determinants thereof.

For example, an amino acid sequence for Amb a 1.1 is reported as follows (GenBank Accession No M80558):

MGIKHCCYILYFTLALVTLLQPVRSAEDLQEILPVNETRRLTTSGAYNIIDGCWRGKADWAENRKALADCA
QGFGKGTVGGKDGDIYTVTSELDDDVANPKEGTLRFGAAQNRPLWIIFERDMVIRLDKEMVVNSDKTIDGR
GAKVEIINAGFTLNGVKNVIIHNINMHDVKVNPGGLIKSNDGPAAPRAGSDGDAISISGSSQIWIDHCSLS
KSVDGLVDAKLGTTRLTVSNSLFTQHQFVLLFGAGDENIEDRGMLATVAFNTFTDNVDQRMPRCRHGFFQV
VNNNYDKWGSYAIGGSASPTILSQGNRFCAPDERSKKNVLGRHGEAAAESMKWNWRTNKDVLENGAIFVAS
GVDPVLTPEQSAGMIPAEPGESALSLTSSAGVLSCQPGAPC

### b. Ragweed (A. psilostachya)

For example, Amb p 5 (eg GenBank Accession No L24465); and antigenic determinants thereof.

### c. Ragweed (A. trifida)

For example, Amb t 5 (eg GenBank Accession No M38782); and antigenic determinants thereof.

Ragweed antigens and antigenic determinants are also described in US Patents Nos 5,698,204; 6,335,020 B1; 6,335,019 B1; and 5,776,761.

### 4. Crop pollen antigens and antigenic determinants

### a. Brassica (Rape)

For example, Bra n 1 (eg GenBank Accession No D63151) and Bra n 2 (eg GenBank Accession No D63152); and antigenic determinants thereof.

For example, an amino acid sequence for Bra n 1 is reported as follows (GenBank Accession No D63151):

MADAEHERIFKKFDTDGDGKISAAELEEALKKLGSVTPDDVTRMMAKIDTDGDGNISFQEFTEFASANPGL
MKDVAKVF

### b. Maize pollens

For example, Zea m 1 (eg GenBank Accession No L14271); and antigenic determinants thereof.

### c. Rice pollens

For example, Ory s 1(eg GenBank Accession No U31771); and antigenic determinants thereof.

### Mite allergens

Mite allergens include all types of allergens found in mites, especially dust mites. Common types of mite allergen include, for example, enzymes such as proteases (eg trypsin, chymotrypsin) amylase, and glutathione transferase or structural proteins such as tropomyosin. Suitably the mite allergen is a dust mite allergen.

Any form of mite antigen or antigenic determinant or any polynucleotide coding for a mite antigen or antigenic determinant (including any biologically active fragment, derivative, homologue or variant) may be used, including but not limited to epitopic polypeptide or polynucleotide sequences of the following:
antigens or antigenic determinants from dust mites such as Dermatophagoides farinae such as Der f 1 (eg SwissProt Accession Nos P16311, Q9GYY0), Der f 2 (eg SwissProt Accession Nos Q00855, Q9BIX2), Der f 3 (eg SwissProt Accession Nos P49275+, Q94508, Q9TWV8), Der f 6 (eg SwissProt Accession No P49276), Der f 7 (eg SwissProt Accession No Q26456), Der f mag (eg SwissProt Accession No P39673), Der f mag29 (eg SwissProt Accession No P39674), Der f mag3 (eg SwissProt Accession No Q94507), Der f 15 (eg SwissProt Accession No Q9U6R7); antigens or antigenic determinants from mites such as Glycyphagus domesticus, such as Gly d 2.02 (eg SwissProt Accession No Q9NFQ4);
antigens or antigenic determinants from dust mites such as Dermatophagoides pteronyssinus such as Der p 1 (eg SwissProt Accession No P08176), Der p 2 (eg SwissProt Accession No P49278), Der p 3 (eg SwissProt Accession No P39675), Der p 4 (eg SwissProt Accession Nos P49274, Q9Y197), Der p 5 (eg SwissProt Accession No P14004) Der p 6 (eg SwissProt Accession No P49277), Der p 7 (eg SwissProt Accession No P49273). Der p 10 (eg SwissProt Accession No O18416), Der p 8 (eg SwissProt Accession No P46419);
antigens or antigenic determinants from dust mites such as Dermatophagoides microceras, such as Der m 1 (eg SwissProt Accession No P 16312);
antigens or antigenic determinants from mites such as Euroglyphus, such as Eur m 1 (eg SwissProt Accession No P25780), Eur m 2.0101 (eg SwissProt Accession No Q9TZZ2) or Eur m 3.0101 (eg SwissProt Accession No 097370);
antigens or antigenic determinants from mites such as Lepidoglyphus, such as Lep d 1 (eg SwissProt Accession No P80384+), Lep d 5 (eg SwissProt Accession No Q9U5P2), Lep d 7 (eg SwissProt Accession No Q9U1G2), Lep d 10 (eg SwissProt Accession No Q9NFZ4), Lep d 13 (eg SwissProt Accession No Q9U5P1);

For example, an amino acid sequence for Der p I is reported as follows (SwissProt Accession No P08176):

1 mkivlaiasl lalsavyarp ssiktfeeyk kafnksyatf edeeaarknf lesvkyvqsn
61 ggainhlsdl sldefknrfl msaeafehlk tqfdlnaetn acsingnapa eidlrqmrtv
121 tpirmqggcg scwafsgvaa tesaylayrn qsldlaeqel vdcasqhgch gdtiprgiey
181 iqhngvvqes yyryvareqs crrpnaqrfg isnycqiypp nvnkireala qthsaiavii
241 gikdldafrh ydgrtiiqrd ngyqpnyhav nivgysnaqg vdywivrnsw dtnwgdngyg
301 yfaanidlmm ieeypyvvil

For example, an amino acid sequence for Der f I is reported as follows (eg SwissProt Accession No P16311):

1   mkfvlaiasl lvlstvyarp asiktfeefk kafnknyatv eeeevarknf leslkyvean
61  kgainhlsdl sldefknryl msaeafeqlk tqfdlnaets acrinsvnvp seldlrslrt
121 vtpirmqggc gscwafsgva atesaylayr ntsldlseqe lvdcasqhgc hgdtiprgie
181 yiqqngvvee rsypyvareq rcrrpnsqhy gisnycqiyp pdvkqireal tqthtaiavi
241 igikdlrafq hydgrtiiqh dngyqpnyha vnivgygstq gddywivrns wdttwgdsgy
301 gyfqagnnlm mieqypyvvi m

Dust mite antigens and antigenic determinants are also described in US Patent Nos 6,147,201; 6,086,897; 6,060,057; 6,423,837 B1; 5,972,352; 6,071,522; 6,132,734; 5,973,132; 6,077,518; 5,433,948; 5,770,202; 5,552,142; 5,773,002; 5,820,862; 6,268,491 B1; 5,968,526; 6,180,771 B1; 6,413,738 B1; 6,077,517; 5,547,669.

### Animal Food Allergens

Animal food allergens include all types of allergens found in foods originating with animals, such as milk, eggs and fish/seafoods. Common types of animal food allergen include, for example antigens or antigenic determinants from tropomyosins, parvalbumins, ovomucoids, ovalbumins, ovotransferrins, lysozymes, vitellogenins, apovitellenins, serum albumins (such as Bovine Serum Albumin; BSA), beta-lactoglobulins, alpha-lactalbumins and caseins (such as Casein, alpha-S1 Casein and Alpha-S2 Casein).

Any form of animal food antigen or antigenic determinant or any polynucleotide coding for an animal food antigen or antigenic determinant (including any biologically active fragment, derivative, homologue or variant) may be used, including but not limited to polypeptide or polynucleotide sequences of the following:

### Fish allergens such as those from Carp (eg Cyc p 1.02 and Cyc p 1.01)

Cod (eg Gad c1; eg SwissProt Accession No P02622), Mackerel (eg Tra j 1; eg SwissProt Accession No Q91482) and Salmon (eg Sal s 1; eg SwissProt Accession No Q91482);

Marine mollusc allergens such as those from Crab (eg Cha f 1; eg SwissProt Accession No Q9N2R3), Lobster (eg Hom a 1; eg SwissProt Accession No 044119), Shrimp (eg Met e1; eg SwissProt Accession No Q25456) and Spiny Lobster (eg Pan s 1; eg SwissProt Accession No 061379);

Egg allergens such as ovomucoids (eg Ga1 d1; eg SwissProt Accession No P01005), ovalbumins (eg Gal d2; eg SwissProt Accession No P01014), ovotransferrins (eg Gal d3; eg SwissProt Accession No P02789), lysozymes (eg Gal d4; eg SwissProt Accession No P00698), vitellogenins, apovitellenins and tropomyosins (eg Hom a 1);

Milk allergens such as those from cow milk, such as BSA (eg Bos d 6; eg SwissProt Accession No P02769), beta-lactoglobulins, alpha-lactalbumins, alpha-S1 caseins and alpha-S2 caseins.

### Plant Food Allergens

Plant food allergens include all types of allergens found in plant matter used as food. Common examples include, for example plant enzymes such as papains, pectate lyases, superoxide dismutases, glyoxalases, beta-fructofuranosidases and phosphate isomerases; plant enzyme inhibitors such as amylase inhibitors and trypsin inhibitors; plant profilins, patatins, actinidins, glycinins, beta-conglycinins, agglutinins and gliadins

Any form of plant food allergen or antigenic determinant or any polynucleotide coding for a plant food allergen or antigenic determinant (including any biologically active fragment, derivative, homologue or variant) may be used in the present invention, including but not limited to polypeptide or polynucleotide sequences of the following:

Avocado allergens and antigenic determinants such as those from Prs a 1 (eg SwissProt Accession No P93680);

Apple allergens and antigenic determinants such as those from Mal d 1 (eg SwissProt Accession No P43211), Mal d 4 (eg SwissProt Accession No Q9XF42), Mal d 3 (eg SwissProt Accession No Q9M5X7);

Apricot allergens and antigenic determinants such as those from Pru ar 3 (eg SwissProt Accession No P81651), Pru ar 1 (eg SwissProt Accession No 050001);

Barley allergens and antigenic determinants such as those from Hor v 1 (eg SwissProt Accession No P16968) and Hor v 9 (eg SwissProt Accession No Q9S8H1);

Buckwheat allergens and antigenic determinants such as those from Fag ag 1 (eg SwissProt Accession No Q9XFM4);

Carrot allergens and antigenic determinants such as those from Dau c 1 (eg SwissProt Accession No 004298);

Castor Bean allergens and antigenic determinants such as those from Ric c 1 (eg SwissProt Accession No P01089);

Celery allergens and antigenic determinants such as those from Api g 1 (eg SwissProt Accession No P49372) Api g 5 (eg SwissProt Accession No P81943) and Api g 1.0201 1 (eg SwissProt Accession No P92918), Api g 3 (eg SwissProt Accession No P92919) Api g 4 (eg SwissProt Accession No Q9XF37);

Cherry allergens and antigenic determinants such as those from Pru a 1 (eg SwissProt Accession No 024248), Pru a 2 (eg SwissProt Accession No P50694), Pru av 3 (eg SwissProt Accession No Q9M5X8), Pru av 4 (eg SwissProt Accession No Q9XF39); Kidney Bean allergens and antigenic determinants such as those from PR Protein (eg SwissProt Accession Nos P25985+ and P25986);

Kiwi allergens and antigenic determinants such as those from Act c 1 (eg SwissProt Accession No P00785);

Maize allergens and antigenic determinants such as those from Zea m 14 (eg SwissProt Accession No P19656), Profilin (eg SwissProt Accession No 022655), Zea m 1 (eg SwissProt Accession No Q07154);

Mustard leaf allergens and antigenic determinants such as those from Bra j 1 L (eg SwissProt Accession No P80215);

Mustard white allergens and antigenic determinants such as those from Sin a 1 (eg SwissProt Accession No Q41196);

Olive allergens and antigenic determinants such as those from Ole e 1 (eg SwissProt Accession No P 19963), Ole e 3 (eg SwissProt Accession No 081092), Ole e 4 (eg SwissProt Accession No P80741), Ole e 5 (eg SwissProt Accession No P80740), Ole e 6 (eg SwissProt Accession No 024172), Ole e 7 (eg SwissProt Accession No P81430), Ole e 8 (eg SwissProt Accession No Q9M7R0), Ole e 9 (eg Entez Accession No AAK58515), Ole e 2 (eg SwissProt Accession No 024169);

Papaya allergens and antigenic determinants such as those from papain (eg SwissProt Accession No P00784);

Peach allergens and antigenic determinants such as those from Pru p 1 (eg SwissProt Accession No P81402);

Pear allergens and antigenic determinants such as those from Pyr c 1 (eg SwissProt Accession No 065200), Pyr c 3 (eg SwissProt Accession No Q9M5X6), Pyr c 4 (eg SwissProt Accession No Q9XF38);

Pineapple allergens and antigenic determinants such as those from pineapple profilin (eg Entrez Accession No AAK54835);

Plantain allergens and antigenic determinants such as those from Pla 11 (eg SwissProt Accession No P82242), Pla 11.0101 (eg SwissProt Accession No CAC41633), Pla 1 1.0102 (eg SwissProt Accession No CAC41634), Pla 11.0103 (eg SwissProt Accession No CAC41635);

Plum allergens and antigenic determinants such as those from Pru d 3 (eg SwissProt Accession No P82534);

Potato allergens and antigenic determinants such as those from patatins (eg SwissProt Accession Nos P07745, P15476, P15477, P11768 and P15478) Sol a t 2 (eg SwissProt Accession No P16348) and Sol a t 4 (eg SwissProt Accession No P30941);

Rice allergens and antigenic determinants such as those from RA 1 (eg SwissProt Accession No Q01884), RA 2 (eg SwissProt Accession No Q01885), RA 5 (eg SwissProt Accession No Q01881), RA 14 (eg SwissProt Accession No Q01882), RA 17 (eg SwissProt Accession No Q01883) Glyoxalase (eg SwissProt Accession No Q9ZWJ2), Ory s 1 (eg SwissProt Accession No Q40638);

Soybean allergens and antigenic determinants such as those from AlaBx (eg SwissProt Accession No P04776), A2B1a (eg SwissProt Accession No P04405), A3B4 (eg SwissProt Accession No P04347) A5A4B3 (eg SwissProt Accession No P02858), Gy3 (eg SwissProt Accession No P11828), Gy4 (eg SwissProt Accession No Q43452), A5A4B3 (eg SwissProt Accession No Q39921), Beta -Conglycinin (eg SwissProt Accession No P 13916), Lectin (eg SwissProt Accession No P05046), Trypsin Inhibitor (eg SwissProt Accession Nos Q39869, Q39898 and Q39899), Gly m 1 (eg SwissProt Accession No P22895), Gly m 1A (eg SwissProt Accession No Q9S8F3), Gly m 2 (eg SwissProt Accession No Q39802), Gly m 3 (eg SwissProt Accession No 065809), Gly m 3 (eg SwissProt Accession No 065810), Gly m bd (eg SwissProt Accession No Q9AVK8);

Tomato allergens and antigenic determinants such as those from Lyc e 1 (eg SwissProt Accession No P13447);

Turnip allergens and antigenic determinants such as those from Bra r 2 (eg SwissProt Accession No P81729);

Wheat allergens and antigenic determinants such as those from agglutinins (eg SwissProt Accession Nos P10968, P02876 and P 10969), alpha amylase and trypsin inhibitors (eg SwissProt Accession Nos P01084, P10846, P01083, P01085, P16852, P16159, P17314, P16851, P16850 and Q41540), gliadins (eg SwissProt Accession Nos P04728, P04726, P02863, Q41546, P04727, P04721, P04722, P04723, P04724, P04725, P18573, P02863, P21292, P08453, P06659, P04729 , P04730, P08079, P02865, Q41548 and Q41543), phosphate isomerases (eg SwissProt Accession No Q9FS79), glutenins (eg SwissProt Accession Nos P08488, P10386, P10387, P02861, P02862, P08489, P10388, P10385, P16315, Q03872, Q41603, Q03871, Q41549, Q41550, Q41551, Q41552 and Q9S8D7), Tri a 2 (CAA10349), Tri a 3 (eg SwissProt Accession No Q41576) and profilins (eg SwissProt Accession Nos P49232, P49233 and P49234).

### Nut allergens

Nut allergens include all types of allergens found in nuts. Common examples include, for example albumins, profilins, vicilins, agglutinins, arachins, glycinins and profilins.

Any form of nut allergen or antigenic determinant or any polynucleotide coding for a nut allergen or antigenic determinant (including any biologically active fragment, derivative, homologue or variant) may be used in the present invention, including but not limited to polypeptide or polynucleotide sequences of the following:
allergens or antigenic determinants from peanut such as Ara h 1 (eg SwissProt Accession Nos P43238, P43237), Ara h 2 (eg ENTREZ Accession No AAK96887), Ara h 3 (eg SwissProt Accession No 082580), Ara h 4 (eg SwissProt Accession No Q9SQH7), Ara h 5 (eg SwissProt Accession No Q9SQI9), Ara h 6 (eg SwissProt Accession No Q9SQG5), Ara h 7 (eg SwissProt Accession No Q9SQH1);
allergens or antigenic determinants from brazil nut such as Ber e 1 (eg SwissProt Accession No P04403);
allergens or antigenic determinants from chestnut (eg Castanea sativa) such as Cas s 1 (eg SwissProt Accession No Q9S8Q4); and
allergens or antigenic determinants from hazel nut such as Cor a 1-5 (eg SwissProt Accession No P43216), Cor a 1 (eg SwissProt Accession Nos Q08407, Q39454, Q39453), Cor a 1.0401 (eg SwissProt Accession No Q9SWR4), Cor a 1.0402 (eg SwissProt Accession No Q9FPK4), Cor a 1.0403 (eg SwissProt Accession No Q9FPK3), Cor a 1.0404 (eg SwissProt Accession No Q9FPK2), Cor a 9 (eg ENTREZ Accession No AAL73404).

### Animal allergens

Animal allergens include all types of allergens generated by animals. Common examples include, for example lipocalins, serum albumins and protease inhibitors, which are commonly present, for example, in animal danders.

Any form of animal antigen or antigenic determinant or any polynucleotide coding for an animal antigen or antigenic determinant (including any biologically active fragment, derivative, homologue or variant) may be used, including but not limited to polypeptide or polynucleotide sequences of the following:
antigens or antigenic determinants from cat danders such as Fel d 1 (eg SwissProt Accession No P30440), Fel d 2 (eg SwissProt Accession No P49064) and Fel d 3 (eg Entrez Accession No AAL49391);
antigens or antigenic determinants from cow danders such as Bos d 2 (eg PIR Accession No B59225);
antigens or antigenic determinants from dog danders such as Can f 1 (eg SwissProt Accession No 018873), Can f 2 (eg SwissProt Accession No 018874) or Can f 3 (eg SwissProt Accession No P49822); and
antigens or antigenic determinants from horse danders such as Equ c1 (eg SwissProt Accession No Q95182), Equ c 2.0101 (eg SwissProt Accession No P81216) and Equ c 2.0102 (eg SwissProt Accession No P81217).

### Cockroach allergens

Any form of cockroach antigen or antigenic determinant or any polynucleotide coding for a cockroach antigen or antigenic determinant (including any biologically active fragment, derivative, homologue or variant) may be used, such as allergens from Blatella and Periplanta, including but not limited to polypeptide or polynucleotide sequences of: Blag 1.0101 (eg SwissProt Accession No Q9UAM5), B1a g 1.02 (eg SwissProt Accession No 096522), Bla g 2 (eg SwissProt Accession No P54958), Bla g 4 (eg SwissProt Accession No P54962), B1a g 5 (eg SwissProt Accession No 018598) Per a 1.0104 (eg SwissProt Accession No 018528), Per a 1.02 (eg SwissProt Accession No O18527), Per a 1.0101 (eg SwissProt Accession No Q9TZR6), Per a 3 (eg SwissProt Accession No Q25641), Per a 1.0102 (eg SwissProt Accession No O18535), Per a 1 (eg SwissProt Accession No 018530) and Per a 7 (eg SwissProt Accession No Q9UB83).

### Venom allergens

Venom allergens include all types of allergens found in venoms, especially insect venoms. Common types of venom allergen include, for example enzyme inhibitors such as melittin and venom enzymes such as phospholipases, hyaluronidases, and diphosphatases.

Any form of venom antigen or antigenic determinant or any polynucleotide coding for a venom antigen or antigenic determinant (including any biologically active fragment, derivative, homologue or variant) may be used, including but not limited to polypeptide or polynucleotide sequences of the following: Bee venom allergens such as allergens from the honey bee and bumble bee, for example Api m 1 (eg SwissProt Accession No P00630), Api m 2 (eg SwissProt Accession No Q08169), Api m 3 (eg SwissProt Accession No P01501) and Bom t1 (eg SwissProt Accession No P82971);

Hornet venom allergens from hornets such as, for example, the European Hornet, D. arenaria, D. maculata, Vespa crabro and Vespa mandarinia, for example Ves c 5.01 (eg SwissProt Accession No P35781), Ves c 5.02 (eg SwissProt Accession No P35782), Dol a 5 (eg SwissProt Accession No Q05108), Dol m 1.01 (eg SwissProt Accession No Q06478), Dol m 1.02 (eg SwissProt Accession No P53357), Dol m 2 (eg SwissProt Accession No P49371), Dol m 5.01 (eg SwissProt Accession No P10736), Dol m 5.02 (eg SwissProt Accession No P10737), Vesp c 5.01 (eg SwissProt Accession No P35781), Vesp c 5.02 (eg SwissProt Accession No P35782),Vesp m 5 (eg SwissProt Accession No P81657);

Ant venom allergens from ants such as, for example, common ants and the fire ants S. invicta, S. richteri and S. geminata , for example Myr p1 (eg SwissProt Accession No Q07932), Myr p 2 (eg SwissProt Accession No Q26464), Sol i 2 (eg SwissProt Accession No P35775), Sol i 3 (eg SwissProt Accession No P35778), Sol i 4 (eg SwissProt Accession No P35777), Sol j 4 (eg Entrez Accession No AAC97369), Sol r 2 (eg SwissProt Accession No P35776), Sol r 3 (eg SwissProt Accession No P35779), Sol g 4 (eg SwissProt Accession No Q9NH75).

Mosquito venom allergens from mosquitos such as Aedes aegypti, for example Aed a 1(eg SwissProt Accession No P50635), Aed a 2 (eg SwissProt Accession No P18153) and Aed a 3 (eg SwissProt Accession No 001949).

Wasp venom allergens from wasps such as P. annularis, P. dominulus, P. exclamans and P. fascatus, such as Pol a 5 (eg SwissProt Accession No Q05109), Pol a 1 (eg SwissProt Accession No Q9U6W0 Pol a 2 (eg SwissProt Accession No Q9U6V9), Pol d 5 (eg SwissProt Accession No P81656), Pol e 5 (eg SwissProt Accession No P35759) and Pol f 5 (eg SwissProt Accession No P35780);

Yellow jacket venom allergens from yellow jackets such as V. flavopilosa, V. germanica, V. maculifrons, V. pensylvanica, V. squamosa, V. vidua and V. vulgaris, such as V es f 5 (eg SwissProt Accession No P35783), Ves g 5 (eg SwissProt Accession No P35784), Ves ml (eg SwissProt Accession No P51528), Ves m 5 (eg SwissProt Accession No P35760), Ves p 5 (eg SwissProt Accession No P35785), Ves s 5 (eg SwissProt Accession No P35786), Ves vi 5 (eg SwissProt Accession No P35787), Ves v 1 (eg SwissProt Accession No P49369), Ves v2 (eg SwissProt Accession No P49370) and Ves v 5 (eg SwissProt Accession No Q05110).

### Fungal allergens

Fungal allergens include all types of allergens originating with fungi. Common examples include, for example, ribosomal proteins, heat shock proteins and enzymes (such as proteases, enolases, alcohol dehydrogenases and superoxide dismutases (SODs)). Fungi may include, for example, strains of Alternaria, Aspergillus, Candida, Cladosporium, Fusarium, Penicillium and Trichophyton.

Any form of fungal antigen or antigenic determinant or any polynucleotide coding for a fungal antigen or antigenic determinant (including any biologically active fragment, derivative, homologue or variant) may be used in the present invention, including but not limited to polypeptide or polynucleotide sequences of the following:
antigens or antigenic determinants from Alternaria altemata such as Alt a 1 (eg SwissProt Accession Nos P79085, Q00021), Alt a 2 (eg SwissProt Accession No 094095), Alt a 3 (eg SwissProt Accession No P78983), Alt a 6 (eg SwissProt Accession No P42037) Alt a 7 (eg SwissProt Accession No P42058), Alt a 10 (eg SwissProt Accession No P42041), Alt a 11 (eg SwissProt Accession No Q9HDT3), Alt a 12 (eg SwissProt Accession No P49148);
antigens or antigenic determinants from Aspergillus mitogillin such as Asp f 1 (eg SwissProt Accession Nos P04389, P82261, O60023, Q9P4F0), Asp f 2 (eg SwissProt Accession Nos P79017, P82262), Asp f 3 (eg SwissProt Accession Nos 043099, P82263, 043099), Asp f 4 (eg SwissProt Accession No 060024), Asp f 6 (eg SwissProt Accession No Q92450), Asp f 7 (eg SwissProt Accession No 042799), Asp f 8 (eg SwissProt Accession No Q9UUZ6), Asp f 9 (eg SwissProt Accession No 042800), Asp f 13 (eg SwissProt Accession No 060022), Asp 11 (eg SwissProt Accession No P82257), Asp f 11 (eg SwissProt Accession No Q9Y7F6), Asp 12 (eg SwissProt Accession No P82258), Asp 13 (eg SwissProt Accession No P82259) or Asp f1 1 (eg SwissProt Accession No Q9UVU3);
antigens or antigenic determinants from Candida such as Can a 1 (eg SwissProt Accession No P43067);
antigens or antigenic determinants from Cladosporium such as C1a h 6 (eg SwissProt Accession No P42040), Cla h 3 (eg SwissProt Accession No P40108), Cla h 4 (eg SwissProt Accession No P42039), C1a h 5 (eg SwissProt Accession No P42059) or Cla h 12 (eg SwissProt Accession No P50344);
antigens or antigenic determinants from Penicillium citrinum such as Pen c 19 (eg SwissProt Accession No Q92260) or Pen c 2 (eg SwissProt Accession No Q9Y755);
antigens or antigenic determinants from Penicillium notatum such as Pen n 13 (eg PIR Accession No JC7208);
antigens or antigenic determinants from Penicillium oxalicum such as Pen o 18 (eg ENTREZ Accession No AAG44478);
antigens or antigenic determinants from Trichophyton such as Tri r 4 (eg SwissProt Accession No Q9UW98) or Tri r 2 (eg SwissProt Accession No Q9UW97).

### Drug allergens

Drugs or drug-like agents capable of causing allergic reactions (drug allergens) include for example:
Antibiotics such as penicillins, sulphonamides, chloramphenicol, cephalosporins, neomycin, streptomycin, bacitracin, clindamycin, dapsone, cephalosporins and vancomycin; cardiovascular agents such as ACE inhibitors, quinidine, amiodarone and methyldopa; anaesthetic drugs and muscle relaxants such as thiopentone and halothane; analgesic agents, for example morphine derivatives such as morphine, pethidine and codeine; anti-inflammatory drugs such as diclofenac, ibuprofen and indomethacin; cancer chemotherapy drugs such as cisplatin, cyclophosphamide, methotrexate, bleomycin and cytarabine; antiseptics such as chlorhexidine, iodine and mercurochrome; solvents such as cremophor; vaccines such as tetanus toxoid and diphtheria vaccine; preservatives such as parabens, sulphites and benzalkonium chlorides; biological therapeutics such as erythropoietins (EPO), insulins, blood factors such as Factor VIII, therapeutic antibodies (eg anti-TNF antibodies) and therapeutic enzymes (eg chymopapain and streptokinase); dyes such as erythrosine and tartrazine; diagnostic agents such as fluoroscein and iodine contrast reagents; hormones such as ACTH, calcitonin, glucocorticoids, and insulins; antivenoms; serum albumins such as human serum albumin; and allergy immunotherapy vaccines.

It will be appreciated that combinations of such allergens and antigenic determinants and/or polynucleotide sequences coding for them may also be used as appropriate.

In addition, it will be appreciated that modulation of an immune response to one particular antigen or antigenic determinant may also modulate responses to other similar antigens and antigenic determinants by operation of a "bystander effect" and/or by so-called epitope spreading or linked suppression.

An antigen suitable for use in the present invention may be any substance that can be recognised by the immune system, and is generally recognised by an antigen (T-cell) receptor. Preferably the antigen used in the present invention is an immunogen.

The immune response to antigen is generally either cell mediated (T cell mediated killing) or humoral (antibody production via recognition of whole antigen). The pattern of cytokine production by TH cells involved in an immune response can influence which of these response types predominates: cell mediated immunity (TH1) is characterised by high IL-2 and IFNγ but low IL-4 production, whereas in humoral immunity (TH2) the pattern is low IL-2 and IFNγ but high IL-4, IL-5 and IL-13. Since the secretory pattern is modulated at the level of the secondary lymphoid organ or cells, then pharmacological manipulation of the specific TH cytokine pattern can influence the type and extent of the immune response generated.

The TH1-TH2 balance refers to the relative representation of the two different forms of helper T cells. The two forms have large scale and opposing effects on the immune system. If an immune response favours TH1 cells, then these cells will drive a cellular response, whereas TH2 cells will drive an antibody-dominated response. The type of antibodies responsible for some allergic reactions is induced by TH2 cells.

The antigen used in the present invention may be a peptide, polypeptide, carbohydrate, protein, glycoprotein, or more complex material containing multiple antigenic epitopes such as a protein complex, cell-membrane preparation, whole cells (viable or non-viable cells), bacterial cells or virus/viral component.

The antigen moiety may be, for example, a synthetic MHC-peptide complex i.e. a fragment of the MHC molecule bearing the antigen groove bearing an element of the antigen. Such complexes have been described in Altman et al. (1996) Science 274: 94-96.

### Modulators of Notch signaling

The term "modulate" as used herein refers to a change or alteration in the biological activity of the Notch signalling pathway or a target signalling pathway thereof. The term "modulator" may refer to antagonists or inhibitors of Notch signalling, i.e. compounds which block, at least to some extent, the normal biological activity of the Notch signalling pathway. Conveniently such compounds may be preferred to herein as inhibitors or antagonists. Alternatively, the term "modulator" may refer to agonists of Notch signalling, i.e. compounds which stimulate or upregulate, at least to some extent, the normal biological activity of the Notch signalling pathway. Conveniently such compounds may be referred to as upregulators or agonists. Preferably the modulator is an agonist of Notch signalling, and preferably an agonist of the Notch receptor (eg an agonist of the Notch1, Notch2, Notch3 and/or Notch4 receptor) in immune cells such as T-cells.

The active agent of the present invention may be an organic compound or other chemical. In one embodiment, a modulator will be an organic compound comprising two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain hetero atoms. Suitable hetero atoms will be apparent to those skilled in the art and include, for instance, sulphur, nitrogen and oxygen. The candidate modulator may comprise at least one cyclic group. The cyclic group may be a polycyclic group, such as a non-fused polycyclic group. For some applications, the agent comprises at least the one of said cyclic groups linked to another hydrocarbyl group.

In one preferred embodiment, the modulator will be an amino acid sequence or a chemical derivative thereof, or a combination thereof. In another preferred embodiment, the modulator will be a nucleotide sequence - which may be a sense sequence or an anti-sense sequence. The modulator may also be an antibody.

The term "antibody" includes intact molecules as well as fragments thereof, such as Fab, F(ab')2, Fv and scFv which are capable of binding the epitopic determinant. These antibody fragments retain some ability to selectively bind with its antigen or receptor and include, for example:
(i) Fab, the fragment which contains a monovalent antigen-binding fragment of an antibody molecule can be produced by digestion of whole antibody with the enzyme papain to yield an intact light chain and a portion of one heavy chain;
(ii) Fab', the fragment of an antibody molecule can be obtained by treating whole antibody with pepsin, followed by reduction, to yield an intact light chain and a portion of the heavy chain; two Fab' fragments are obtained per antibody molecule;
(iii) F(ab')₂, the fragment of the antibody that can be obtained by treating whole antibody with the enzyme pepsin without subsequent reduction; F(ab')₂ is a dimer of two Fab' fragments held together by two disulfide bonds;
(iv) scFv, including a genetically engineered fragment containing the variable region of a heavy and a light chain as a fused single chain molecule.

General methods of making these fragments are known in the art. (See for example, Harlow and Lane, Antibodies: A Laboratory Manual, Cold Spring Harbor Laboratory, New York (1988)).

Modulators may be synthetic compounds or natural isolated compounds.

In one form the modulator of the Notch signalling pathway may be a protein for Notch signalling transduction.

By a protein which is for Notch signalling transduction is meant a molecule which participates in signalling through Notch receptors including activation of Notch, the downstream events of the Notch signalling pathway, transcriptional regulation of downstream target genes and other non-transcriptional downstream events (e.g. post-translational modification of existing proteins). More particularly, the protein is a domain that allows activation of target genes of the Notch signalling pathway, or a polynucleotide sequence which codes therefor.

A very important component of the Notch signalling pathway is Notch receptor/Notch ligand interaction. Thus Notch signalling may involve changes in expression, nature, amount or activity of Notch ligands or receptors or their resulting cleavage products. In addition, Notch signalling may involve changes in expression, nature, amount or activity of Notch signalling pathway membrane proteins or G-proteins or Notch signalling pathway enzymes such as proteases, kinases (e.g. serine/threonine kinases), phosphatases, ligases (e.g. ubiquitin ligases) or glycosyltransferases. Alternatively the signalling may involve changes in expression, nature, amount or activity of DNA binding elements such as transcription factors.

In a preferred form of the invention the signalling may be specific signalling, meaning that the signal results substantially or at least predominantly from the Notch signalling pathway, and preferably from Notch/Notch ligand interaction, rather than any other significant interfering or competing cause, such as cytokine signalling. Thus, in a preferred embodiment, Notch signalling excludes cytokine signalling. The Notch signalling pathway is described in more detail below.

Key targets for Notch-dependent transcriptional activation are genes of the *Enhancer of split* complex (E[spl]). Moreover these genes have been shown to be direct targets for binding by the Su(H) protein and to be transcriptionally activated in response to Notch signalling. By analogy with EBNA2, a viral coactivator protein that interacts with a mammalian Su(H) homologue CBF1 to convert it from a transcriptional repressor to a transcriptional activator, the Notch intracellular domain, perhaps in association with other proteins may combine with Su(H) to contribute an activation domain that allows Su(H) to activate the transcription of *E(spl)* as well as other target genes. It should also be noted that Su(H) is not required for all Notch-dependent decisions, indicating that Notch mediates some cell fate choices by associating with other DNA-binding transcription factors or by employing other mechanisms to transduce extracellular signals.

According to one aspect of the present invention the active agent may be Notch or a fragment thereof which retains the signalling transduction ability of Notch or an analogue of Notch which has the signalling transduction ability of Notch.

As used herein the term "analogue of Notch" includes variants thereof which retain the signalling transduction ability of Notch. By "analogue" we include a protein which has Notch signalling transduction ability, but generally has a different evolutionary origin to Notch. Analogues of Notch include proteins from the Epstein Barr virus (EBV), such as EBNA2, BARF0 or LMP2A.

By a protein which is for Notch signalling activation we mean a molecule which is capable of activating Notch, the Notch signalling pathway or any one or more of the components of the Notch signalling pathway.

In a particular embodiment, the active agent will be capable of inducing or increasing Notch or Notch ligand expression. Such a molecule may be a nucleic acid sequence capable of inducing or increasing Notch or Notch ligand expression.

In one embodiment, the active agent will be capable of upregulating expression of the endogenous genes encoding Notch or Notch ligands in target cells. In particular, the molecule may be an immunosuppressive cytokine capable of upregulating the expression of endogenous Notch or Notch ligands in target cells, or a polynucleotide which encodes such a cytokine. Immunosuppressive cytokines include IL-4, IL-10, IL-13, TGF-β and SLIP3 (FLT3) ligand.

Preferably, the active agent will be a polypeptide selected from Noggin, Chordin, Follistatin, Xnr3, fibroblast growth factors and derivatives, fragments, variants and homologues thereof, or a polynucleotide encoding any one or more of the above.

Suitably, the active agent may be a Notch ligand, or a polynucleotide encoding a Notch ligand. Notch ligands of use in the present invention include endogenous Notch ligands which are typically capable of binding to a Notch receptor polypeptide present in the membrane of a variety of mammalian cells, for example hemapoietic stem cells.

The term "Notch ligand" as used herein means an agent capable of interacting with a Notch receptor to cause a biological effect. The term as used herein therefore includes naturally occurring protein ligands such as Delta and Serrate/Jagged and their biologically active fragments as well as antibodies to the Notch receptor, peptidomimetics and small molecules which have corresponding biological effects to the natural ligands. Preferably the Notch ligand interacts with the Notch receptor by binding.

Particular examples of mammalian Notch ligands identified to date include the Delta family, for example Delta or Delta-like 1 (Genbank Accession No. AF003522 - *Homo sapiens*)*,* Delta-3 (Genbank Accession No. AF084576 - *Rattus norvegicus*) and Delta-like 3 (*Mus musculus*) (Genbank Accession No. NM_016941 - *Homo sapiens*) and US 6121045 (Millennium), Delta-4 (Genbank Accession Nos. AB043894 and AF 253468 - *Homo sapiens*) and the Serrate family, for example Serrate-1 and Serrate-2 (WO97/01571, WO96/27610 and WO92/19734), Jagged-1 (Genbank Accession No. U73936 - *Homo sapiens*) and Jagged-2 (Genbank Accession No. AF029778 - *Homo sapiens*)*,* and LAG-2. Homology between family members is extensive.

For example, an exemplary human Delta 4 is contained in a plasmid which was deposited with the American Type Culture Collection (ATCC) on March 5, 1997, and has been assigned ATCC accession number 98348 (eg see US 6121045; Millennium)

A transformant in which vector pUCDL-1F, which reportedly contains cDNA coding total amino acid sequence of human Delta-1, is transformed into E. coli JM109, has been deposited in the National Institute of Bioscience and Human-Technology, Agency of Industrial Science and Technology, MITI, of 1-1-3, Higasi, Tsukuba-shi, Ibaragi-ken, Japan, as E. coli: JM109-pUCDL-1F. Date of deposit was Oct. 28, 1996, and deposition No. is FBRM BP-5728. A transformant in which vector pUCSR-1, which reportedly contains cDNA coding total amino acid sequence of human Serrate,-1, is transformed into E. coli JM109, has been deposited in the National Institute of Bioscience and Human-Technology, Agency of industrial Science and Technology, MITI, of 1-1-3, Higasi, Tsukuba-shi, Ibaragi-ken, Japan, as E. coli: JM109-pUCSR-1. Date of deposit was October 28, 1996, and deposition No. is FBRPM BP-5726 (See US 6337387).

In an alternative embodiment, an activator will be a constitutively active Notch receptor or Notch intracellular domain, or a polynucleotide encoding such a receptor or intracellular domain.

In a further alternative embodiment, an activator of Notch signalling will act downstream of the Notch receptor. Thus, for example, the activator of Notch signalling may be a constitutively active Deltex polypeptide or a polynucleotide encoding such a polypeptide. Other downstream components of the Notch signalling pathway of use in the present invention include the polypeptides involved in the Ras/MAPK cascade catalysed by Deltex, polypeptides involved in the proteolytic cleavage of Notch such as Presenilin and polypeptides involved in the transcriptional regulation of Notch target genes, preferably in a constitutively active form.

By polypeptide for Notch signalling activation is also meant any polypeptides expressed as a result of Notch activation and any polypeptides involved in the expression of such polypeptides, or polynucleotides coding for such polypeptides.

Activation of Notch signalling may also be achieved by repressing inhibitors of the Notch signalling pathway. As such, polypeptides for Notch signalling activation will include molecules capable of repressing any Notch signalling inhibitors. Preferably the molecule will be a polypeptide, or a polynucleotide encoding such a polypeptide, that decreases or interferes with the production or activity of compounds that are capable of producing an decrease in the expression or activity of Notch, Notch ligands, or any downstream components of the Notch signalling pathway. In a preferred embodiment, the molecules will be capable of repressing polypeptides of the Toll-like receptor protein family, cytokines such as IL-12, IFN-γ, TNF-α, and growth factors such as the bone morphogenetic protein (BMP), BMP receptors and activins, derivatives, fragments, variants and homologues thereof.

By a protein which is for Notch signalling inhibition or a polynucleotide encoding such a protein, we mean a molecule which is capable of inhibiting Notch, the Notch signalling pathway or any one or more of the components of the Notch signalling pathway.

In a particular embodiment, the molecule will be capable of reducing or preventing Notch or Notch ligand expression. Such a molecule may be a nucleic acid sequence capable of reducing or preventing Notch or Notch ligand expression.

For example the nucleic acid sequence may encode a polypeptide selected from Toll-like receptor protein family, a cytokine such as IL-12, IFN-γ, TNF-α, or a growth factor such as a bone morphogenetic protein (BMP), a BMP receptor and activins. Suitably the agent is a polypeptide, or a polynucleotide encoding such a polypeptide, that decreases or interferes with the production of compounds that are capable of producing an increase in the expression of Notch ligand, such as Noggin, Chordin, Follistatin, Xnr3, fibroblast growth factors and derivatives, fragments, variants and homologues thereof.

Alternatively, the nucleic acid sequence may be an antisense construct derived from a sense nucleotide sequence encoding a polypeptide selected from a Notch ligand and a polypeptide capable of upregulating Notch ligand expression, such as Noggin, Chordin, Follistatin, Xnr3, fibroblast growth factors and derivatives, fragments, variants and homologues thereof.

In another embodiment a modulator of Notch signalling may be a molecule which is capable of modulating Notch-Notch ligand interactions. A molecule may be considered to modulate Notch-Notch ligand interactions if it is capable of inhibiting the interaction of Notch with its ligands, preferably to an extent sufficient to provide therapeutic efficacy.

The molecule may also be a polypeptide, or a polynucleotide encoding such a polypeptide, selected from a Toll-like receptor, a cytokine such as IL-12, IFN-γ, TNF-α, or a growth factor such as a BMP, a BMP receptor and activins. Preferably the polypeptide decreases or interferes with the production of an agent that is capable of producing an increase in the expression of Notch ligand, such as Noggin, Chordin, Follistatin, Xnr3, fibroblast growth factors and derivatives, fragments, variants, homologues and analogues thereof.

Preferably when the inhibitor is a receptor or a nucleic acid sequence encoding a receptor, the receptor is activated. Thus, for example, when the agent is a nucleic acid sequence, the receptor is preferably constitutively active when expressed.

Inhibitors of Notch signalling also include downstream inhibitors of the Notch signalling pathway, compounds that prevent expression of Notch target genes or induce expression of genes repressed by the Notch signalling pathway. Examples of such proteins include Dsh and Numb and dominant negative versions of Notch IC and Deltex. Proteins for Notch signalling inhibition will also include variants of the wild-type components of the Notch signalling pathway which have been modified in such a way that their presence blocks rather than transduces the signalling pathway. An example of such a compound would be a Notch receptor which has been modified such that proteolytic cleavage of its intracellular domain is no longer possible.

Any one or more of appropriate targets - such as an amino acid sequence and/or nucleotide sequence - may be used for identifying a compound capable of modulating the Notch signalling pathway and/or a targeting molecule in any of a variety of drug screening techniques. The target employed in such a test may be free in solution, affixed to a solid support, borne on a cell surface, or located intracellularly.

Techniques for drug screening may be based on the method described in Geysen, European Patent No. 0138855, published on September 13, 1984. In summary, large numbers of different small peptide candidate modulators or targeting molecules are synthesized on a solid substrate, such as plastic pins or some other surface. The peptide test compounds are reacted with a suitable target or fragment thereof and washed. Bound entities are then detected - such as by appropriately adapting methods well known in the art. A purified target can also be coated directly onto plates for use in drug screening techniques. Plates of use for high throughput screening (HTS) will be multi-well plates, preferably having 96, 384 or over 384 wells/plate. Cells can also be spread as "lawns". Alternatively, non-neutralising antibodies can be used to capture the peptide and immobilise it on a solid support. High throughput screening, as described above for synthetic compounds, can also be used for identifying organic candidate modulators and targeting molecules.

This invention also contemplates the use of competitive drug screening assays in which neutralising antibodies capable of binding a target specifically compete with a test compound for binding to a target.

Techniques are well known in the art for the screening and development of agents such as antibodies, peptidomimetics and small organic molecules which are capable of binding to components of the Notch signalling pathway. These include the use of phage display systems for expressing signalling proteins, and using a culture of transfected E. coli or other microorganism to produce the proteins for binding studies of potential binding compounds (see, for example, G. Cesarini, FEBS Letters, 307(1):66-70 (July 1992); H. Gram et al., J. Immunol. Meth., 161:169-176 (1993); and C. Summer et al., Proc. Natl. Acad. Sci., USA, 89:3756-3760 (May 1992)). Further library and screening techniques are described, for example, in US 6281344 (Phylos).

### Polypeptides, Proteins and Amino Acid Sequences

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "protein".

"Peptide" usually refers to a short amino acid sequence that is 10 to 40 amino acids long, preferably 10 to 35 amino acids.

The amino acid sequence may be prepared and isolated from a suitable source, or it may be made synthetically or it may be prepared by use of recombinant DNA techniques.

Within the definitions of "proteins" useful in the present invention, the specific amino acid residues may be modified in such a manner that the protein in question retains at least one of its endogenous functions, such modified proteins are referred to as "variants". A variant protein can be modified by addition, deletion and/or substitution of at least one amino acid present in the naturally-occurring protein.

Typically, amino acid substitutions may be made, for example from 1, 2 or 3 to 10 or 20 substitutions provided that the modified sequence retains the required target activity or ability to modulate Notch signalling. Amino acid substitutions may include the use of non-naturally occurring analogues.

The protein used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the target or modulation function is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

For ease of reference, the one and three letter codes for the main naturally occurring amino acids (and their associated codons) are set out below:

| Symbol | 3-letter | Meaning | Codons |
|---|---|---|---|
| | | | |
| A | Ala | Alanine | GCT,GCC,GCA,GCG |
| B | Asp,Asn | Aspartic, Asparagine | GAT,GAC,AAT,AAC |
| C | Cys | Cysteine | TGT,TGC |
| D | Asp | Aspartic | GAT,GAC |
| B | Glu | Glutamic | GAA,GAG |
| F | Phe | Phenylalanine | TTT,TTC |
| G | Gly | Glycine | GGT,GGC,GGA,GGG |
| H | His | Histidine | CAT,CAC |
| I | Ile | Isoleucine | ATT,ATC,ATA |
| K | Lys | Lysine | AAA,AAG |
| L | Leu | Leucine | TTG,TTA,CTT,CTC,CTA,CTG |
| M | Met | Methionine | ATG |
| N | Asn | Asparagine | AAT,AAC |
| P | Pro | Proline | CCT,CCC,CCA,CCG |
| Q | Gln | Glutamine | CAA,CAG |
| R | Arg | Arginine | CGT,CGC,CGA,CGG,AGA,AGG |
| S | Ser | Serine | TCT,TCC,TCA,TCG,AGT,AGC |
| T | Thr | Threonine | ACT,ACC,ACA,ACG |
| V | Val | Valine | GTT,GTC,GTA,GTG |
| W | Trp | Tryptophan | TGG |
| X | Xxx | Unknown | |
| Y | Tyr | Tyrosine | TAT, TAC |
| Z | Glu,Gln | Glutamic, Glutamine | GAA,GAG,CAA,CAG |
| * | End | Terminator | TAA, TAG, TGA |

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function. A peptide useful in the invention will at least have a target or signalling modulation capability. "Fragments" are also variants and the term typically refers to a selected region of the protein that is of interest in a binding assay and for which a binding partner is known or determinable. "Fragment" thus refers to an amino acid sequence that is a portion of a full-length polypeptide, suitably between about 8 and about 1500 amino acids in length, for example between about 8 and about 745 amino acids in length, preferably about 8 to about 300, more preferably about 8 to about 200 amino acids, for example about 10 to about 50 or 100 amino acids in length. "Peptide" refers to a short amino acid sequence that is 10 to 40 amino acids long, preferably 10 to 35 amino acids.

Proteins or polypeptides may be in the form of the "mature" protein or may be a part of a larger protein such as a fusion protein or precursor. For example, it is often advantageous to include an additional amino acid sequence which contains secretory or leader sequences or pro-sequences (such as a HIS oligomer, immunoglobulin Fc, glutathione S-transferase, FLAG etc) to aid in purification. Likewise such an additional sequence may sometimes be desirable to provide added stability during recombinant production. In such cases the additional sequence may be cleaved (eg chemically or enzymatically) to yield the final product. In some cases, however, the additional sequence may also confer a desirable pharmacological profile (as in the case of IgFc fusion proteins) in which case it may be preferred that the additional sequence is not removed so that it is present in the final product as administered.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

Variants of the nucleotide sequence may also be made. Such variants will preferably comprise codon optimised sequences. Codon optimisation is known in the art as a method of enhancing RNA stability and therefore gene expression. The redundancy of the genetic code means that several different codons may encode the same amino-acid. For example, leucine, arginine and serine are each encoded by six different codons. Different organisms show preferences in their use of the different codons. Viruses such as HIV, for instance, use a large number of rare codons. By changing a nucleotide sequence such that rare codons are replaced by the corresponding commonly used mammalian codons, increased expression of the sequences in mammalian target cells can be achieved. Codon usage tables are known in the art for mammalian cells, as well as for a variety of other organisms.

### Nucleotide Sequences

Where the modulator of Notch signalling or antigen/antigenic determinant comprises a nucleotide sequence it may suitably be codon optimised for expression in mammalian cells. In a preferred embodiment, such sequences are optimised in their entirety.

"Polynucleotide" refers to a polymeric form of nucleotides of at least 10 bases in length and up to 10,000 bases or more, either ribonucleotides or deoxyribonucleotides or a modified form of either type of nucleotide. The term includes single and double stranded forms of DNA and also derivatised versions such as protein nucleic acid (PNA).

These may be constructed using standard recombinant DNA methodologies. The nucleic acid may be RNA or DNA and is preferably DNA. Where it is RNA, manipulations may be performed via cDNA intermediates. Generally, a nucleic acid sequence encoding the first region will be prepared and suitable restriction sites provided at the 5' and/or 3' ends. Conveniently the sequence is manipulated in a standard laboratory vector, such as a plasmid vector based on pBR322 or pUC19 (see below). Reference may be made to Molecular Cloning by Sambrook et al. (Cold Spring Harbor, 1989) or similar standard reference books for exact details of the appropriate techniques.

Nucleic acid encoding the second region may likewise be provided in a similar vector system.

Sources of nucleic acid may be ascertained by reference to published literature or databanks such as GenBank. Nucleic acid encoding the desired first or second sequences may be obtained from academic or commercial sources where such sources are willing to provide the material or by synthesising or cloning the appropriate sequence where only the sequence data are available. Generally this may be done by reference to literature sources which describe the cloning of the gene in question.

Alternatively, where limited sequence data are available or where it is desired to express a nucleic acid homologous or otherwise related to a known nucleic acid, exemplary nucleic acids can be characterised as those nucleotide sequences which hybridise to the nucleic acid sequences known in the art.

It will be understood by a skilled person that numerous different nucleotide sequences can encode the same protein used in the present invention as a result of the degeneracy of the genetic code. In addition, it is to be understood that skilled persons may, using routine techniques, make nucleotide substitutions that do not affect the protein encoded by the nucleotide sequence of the present invention to reflect the codon usage of any particular host organism in which the target protein or protein for Notch signalling modulation of the present invention is to be expressed.

In general, the terms "variant", "homologue" or "derivative" in relation to the nucleotide sequence used in the present invention includes any substitution of, variation of, modification of, replacement of, deletion of or addition of one (or more) nucleic acid from or to the sequence providing the resultant nucleotide sequence codes for a target protein or protein for T cell signalling modulation.

As indicated above, with respect to sequence homology, preferably there is at least 75%, more preferably at least 85%, more preferably at least 90% homology to the reference sequences. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is - 3 for each nucleotide.

Homology comparisons can be conducted by eye, or more usually, with the aid of readily available sequence comparison programs. These commercially available computer programs can calculate % homology between two or more sequences.

Percent homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefor firstly requires the production of an optimal alignment, taking into consideration gap penalties. A suitable computer program for carrying out such an alignment is the GCG Wisconsin Bestfit package (University of Wisconsin, U.S.A.; Devereux). Examples of other software than can perform sequence comparisons include, but are not limited to, the BLAST package, FASTA (Atschul et al. (1990) J. Mol. Biol. 403-410 (Atschul)) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

The five BLAST programs available at http://www.ncbi.nlm.nih.gov perform the following tasks:
**blastp** - compares an amino acid query sequence against a protein sequence database.
**blastn** - compares a nucleotide query sequence against a nucleotide sequence database.
**blastx** - compares the six-frame conceptual translation products of a nucleotide query sequence (both strands) against a protein sequence database.
**tblastn** - compares a protein query sequence against a nucleotide sequence database dynamically translated in all six reading frames (both strands).
**tblastx** - compares the six-frame translations of a nucleotide query sequence against the six-frame translations of a nucleotide sequence database.
BLAST uses the following search parameters:
HISTOGRAM - Display a histogram of scores for each search; default is yes. (See parameter H in the BLAST Manual).
DESCRIPTIONS - Restricts the number of short descriptions of matching sequences reported to the number specified; default limit is 100 descriptions. (See parameter V in the manual page).
EXPECT - The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF - Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
MATRIX - Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.
STRAND - Restrict a TBLASTN search to just the top or bottom strand of the database sequences; or restrict a BLASTN, BLASTX or TBLASTX search to just reading frames on the top or bottom strand of the query sequence.
FILTER - Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi - Causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST.

In some aspects of the present invention, no gap penalties are used when determining sequence identity.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

### Polynucleotide Hybridisation

The present invention also encompasses nucleotide sequences that are capable of hybridising selectively to the reference sequences, or any variant, fragment or derivative thereof, or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction (PCR) technologies.

Nucleotide sequences useful in the invention capable of selectively hybridising to the nucleotide sequences presented herein, or to their complement, will be generally at least 75%, preferably at least 85 or 90% and more preferably at least 95% or 98% homologous to the corresponding nucleotide sequences presented herein over a region of at least 20, preferably at least 25 or 30, for instance at least 40, 60 or 100 or more contiguous nucleotides. Preferred nucleotide sequences of the invention will comprise regions homologous to the nucleotide sequence, preferably at least 80 or 90% and more preferably at least 95% homologous to the nucleotide sequence.

The term "selectively hybridizable" means that the nucleotide sequence used as a probe is used under conditions where a target nucleotide sequence of the invention is found to hybridize to the probe at a level significantly above background. The background hybridization may occur because of other nucleotide sequences present, for example, in the cDNA or genomic DNA library being screened. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical nucleotide sequences while an intermediate (or low) stringency hybridization can be used to identify or detect similar or related polynucleotide sequences.

In a preferred aspect, the present invention covers nucleotide sequences that can hybridise to the nucleotide sequence of the present invention under stringent conditions (e.g. 65°C and 0.1xSSC {1xSSC = 0.15 M NaCl, 0.015 M Na₃ Citrate pH 7.0). Where the nucleotide sequence of the invention is double-stranded, both strands of the duplex, either individually or in combination, are encompassed by the present invention. Where the nucleotide sequence is single-stranded, it is to be understood that the complementary sequence of that nucleotide sequence is also included within the scope of the present invention.

Stringency of hybridisation refers to conditions under which polynucleic acids hybrids are stable. Such conditions are evident to those of ordinary skill in the field. As known to those of skill in the art, the stability of hybrids is reflected in the melting temperature (Tm) of the hybrid which decreases approximately 1 to 1.5°C with every 1% decrease in sequence homology. In general, the stability of a hybrid is a function of sodium ion concentration and temperature. Typically, the hybridisation reaction is performed under conditions of higher stringency, followed by washes of varying stringency.

As used herein, high stringency preferably refers to conditions that permit hybridisation of only those nucleic acid sequences that form stable hybrids in 1 M Na+ at 65-68 °C. High stringency conditions can be provided, for example, by hybridisation in an aqueous solution containing 6x SSC, 5x Denhardt's, 1 % SDS (sodium dodecyl sulphate), 0.1 Na+ pyrophosphate and 0.1 mg/ml denatured salmon sperm DNA as non specific competitor. Following hybridisation, high stringency washing may be done in several steps, with a final wash (about 30 min) at the hybridisation temperature in 0.2 - 0.1x SSC, 0.1 % SDS. It is understood that these conditions may be adapted and duplicated using a variety of buffers, e.g. formamide-based buffers, and temperatures. Denhardt's solution and SSC are well known to those of skill in the art as are other suitable hybridisation buffers (see, e.g. Sambrook, et al., eds. (1989) Molecular Cloning: A Laboratory Manual, Cold Spring Harbor Laboratory Press, New York or Ausubel, et al., eds. (1990) Current Protocols in Molecular Biology, John Wiley & Sons, Inc.). Optimal hybridisation conditions have to be determined empirically, as the length and the GC content of the hybridising pair also play a role.

Nucleotide sequences can be obtained in a number of ways. Variants of the sequences described herein may be obtained for example by probing DNA libraries made from a range of sources. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of the reference nucleotide sequence under conditions of medium to high stringency. Similar considerations apply to obtaining species homologues and allelic variants of the amino acid and/or nucleotide sequences useful in the present invention.

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used. The primers used in degenerate PCR will contain one or more degenerate positions and will be used at stringency conditions lower than those used for cloning sequences with single sequence primers against known sequences.

Alternatively, such nucleotide sequences may be obtained by site directed mutagenesis of characterised sequences. This may be useful where for example silent codon changes are required to sequences to optimise codon preferences for a particular host cell in which the nucleotide sequences are being expressed. Other sequence changes may be desired in order to introduce restriction enzyme recognition sites, or to alter the activity of the target protein or protein for T cell signalling modulation encoded by the nucleotide sequences.

The nucleotide sequences such as DNA polynucleotides useful in the invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer nucleotide sequences will generally be produced using recombinant means, for example using a PCR (polymerase chain reaction) cloning techniques. This will involve making a pair of primers (e.g. of about 15 to 30 nucleotides) flanking a region of the targeting sequence which it is desired to clone, bringing the primers into contact with mRNA or cDNA obtained from an animal or human cell, performing a polymerase chain reaction (PCR) under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

### Transfection and Expression

For recombinant production, host cells can be genetically engineered to incorporate expression systems or polynucleotides of the invention. Introduction of a polynucleotide into the host cell can be effected by methods described in many standard laboratory manuals, such as Davis *et al* and Sambrook *et al,* such as calcium phosphate transfection, DEAE-dextran mediated transfection, transvection, microinjection, cationic lipid-mediated transfection, electroporation, transduction, scrape loading, ballistic introduction and infection. In will be appreciated that such methods can be employed *in vitro* or *in vivo* as drug delivery systems.

Representative examples of appropriate hosts include bacterial cells, such as streptococci, staphylococci, *E. coli,* streptomyces and *Bacillus subtilis* cells; fungal cells, such as yeast cells and *Aspergillus* cells; insect cells such as *Drosophila* S2 and *Spodoptera* Sf9 cells; animal cells such as CHO, COS, NSO, HeLa, C127, 3T3, BHK, 293 and Bowes melanoma cells; and plant cells.

Proteins or polypeptides may be in the form of the "mature" protein or may be a part of a larger protein such as a fusion protein or precursor. For example, it is often advantageous to include an additional amino acid sequence which contains secretory or leader sequences or pro-sequences (such as a HIS oligomer, immunoglobulin Fc, glutathione S-transferase, FLAG etc) to aid in purification. Likewise such an additional sequence may sometimes be desirable to provide added stability during recombinant production. In such cases the additional sequence may be cleaved (eg chemically or enzymatically) to yield the final product. In some cases, however, the additional sequence may also confer a desirable pharmacological profile (as in the case of IgFc fusion proteins) in which case it may be preferred that the additional sequence is not removed so that it is present in the final product as administered.

A great variety of expression systems can be used to produce a polypeptide useful in the present invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al.*

For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Active agents for use in the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding protein may be employed to regenerate active conformation when the polypeptide is denatured during isolation and/or purification.

### Chemical Coupling

Chemically coupled sequences can be prepared from individual proteins sequences and coupled using known chemically coupling techniques. The conjugate can be assembled using conventional solution- or solid-phase peptide synthesis methods, affording a fully protected precursor with only the terminal amino group in deprotected reactive form. This function can then be reacted directly with a protein for Notch signalling modulation or a suitable reactive derivative thereof. Alternatively, this amino group may be converted into a different functional group suitable for reaction with a cargo moiety or a linker. Thus, e.g. reaction of the amino group with succinic anhydride will provide a selectively addressable carboxyl group, while further peptide chain extension with a cysteine derivative will result in a selectively addressable thiol group. Once a suitable selectively addressable functional group has been obtained in the delivery vector precursor, a protein for Notch signalling modulation or a derivative thereof may be attached through e.g. amide, ester, or disulphide bond formation. Cross-linking reagents which can be utilized are discussed, for example, in Neans, G.E. and Feeney, R.E., Chemical Modification of Proteins, Holden-Day, 1974, pp. 39-43.

As discussed above the target protein and protein for T cell signalling modulation may be linked directly or indirectly via a cleavable linker moiety. Direct linkage may occur through any convenient functional group on the protein for T cell signalling modulation such as a hydroxy, carboxy or amino group. Indirect linkage which is preferable, will occur through a linking moiety. Suitable linking moieties include bi- and multifunctional alkyl, aryl, aralkyl or peptidic moieties, alkyl, aryl or aralkyl aldehydes acids esters and anyhdrides, sulphydryl or carboxyl groups, such as maleimido benzoic acid derivatives, maleimido proprionic acid derivatives and succinimido derivatives or may be derived from cyanuric bromide or chloride, carbonyldiimidazole, succinimidyl esters or sulphonic halides and the like. The functional groups on the linker moiety used to form covalent bonds between linker and protein for T cell signalling modulation on the one hand, as well as linker and target protein on the other hand, may be two or more of, e.g., amino, hydrazino, hydroxyl, thiol, maleimido, carbonyl, and carboxyl groups, etc. The linker moiety may include a short sequence of from 1 to 4 amino acid residues that optionally includes a cysteine residue through which the linker moiety bonds to the target protein.

Various preferred features and embodiments of the present invention will now be described in more detail by way of non-limiting examples.

### Polypeptides and Polynucleotides for Notch Signalling Transduction

The Notch signalling pathway directs binary cell fate decisions in the embryo. Notch was first described in *Drosophila* as a transmembrane protein that functions as a receptor for two different ligands, Delta and Serrate. Vertebrates express multiple Notch receptors and ligands (discussed below). At least four Notch receptors (Notch-1, Notch-2, Notch-3 and Notch-4) have been identified to date in human cells (see for example GenBank Accession Nos. AF308602, AF308601 and U95299 - *Homo sapiens*).

Notch proteins are synthesized as single polypeptide precursors that undergo cleavage via a Furin-like convertase that yields two polypeptide chains that are further processed to form the mature receptor. The Notch receptor present in the plasma membrane comprises a heterodimer of two Notch proteolytic cleavage products, one comprising an N-terminal fragment consisting of a portion of the extracellular domain, the transmembrane domain and the intracellular domain, and the other comprising the majority of the extracellular domain. The proteolytic cleavage step of Notch to activate the receptor occurs in the Golgi apparatus and is mediated by a furin-like convertase.

Notch receptors are inserted into the membrane as disulphide-linked heterodimeric molecules consisting of an extracellular domain containing up to 36 epidermal growth factor (EGF-like repeats [Notch 1/2 = 36, Notch 3 = 34 and Notch 4 = 29], 3 Cysteine Rich Repeats (Lin-Notch (L/N) repeats) and a transmembrane subunit that contains the cytoplasmic domain. The cytoplasmic domain of Notch contains six ankyrin-like repeats, a polyglutamine stretch (OPA) and a PEST sequence. A further domain termed RAM23 lies proximal to the ankyrin repeats and is involved in binding to a transcription factor, known as Suppressor of Hairless [Su(H)] in *Drosophila* and CBF1 in vertebrates (Tamura K, et al. (1995) Curr. Biol. 5:1416-1423). The Notch ligands also display multiple EGF-like repeats in their extracellular domains together with a cysteine-rich DSL (Delta-Serrate Lag2) domain that is characteristic of all Notch ligands (Artavarus-Tsakonas S, et al. (1995) Science 268:225-232; Artavanis-Tsakonas S, et al. (1999) Science 284:770-776).

The Notch receptor is activated by binding of extracellular ligands, such as Delta, Serrate and Scabrous, to the EGF-like repeats of Notch's extracellular domain. Delta may require cleavage for activation. It is cleaved by the ADAM disintegrin metalloprotease Kuzbanian at the cell surface, the cleavage event releasing a soluble and active form of Delta. An oncogenic variant of the human Notch-1 protein, also known as TAN-1, which has a truncated extracellular domain, is constitutively active and has been found to be involved in T-cell lymphoblastic leukemias.

The cdc10/ankyrin intracellular-domain repeats mediate physical interaction with intracellular signal transduction proteins. Most notably, the cdc10/ankyrin repeats interact with Suppressor of Hairless [Su(H)]. Su(H) is the *Drosophila* homologue of C-promoter binding factor-1 [CBF-1], a mammalian DNA binding protein involved in the Epstein-Barr virus-induced immortalization of B-cells. It has been demonstrated that, at least in cultured cells, Su(H) associates with the cdc 10/ankyrin repeats in the cytoplasm and translocates into the nucleus upon the interaction of the Notch receptor with its ligand Delta on adjacent cells. Su(H) includes responsive elements found in the promoters of several genes and has been found to be a critical downstream protein in the Notch signalling pathway. The involvement of Su(H) in transcription is thought to be modulated by Hairless.

The intracellular domain of Notch (NotchIC) also has a direct nuclear function (Lieber, T. et al. (1993) Genes Dev 7(10):1949-65). Recent studies have indeed shown that Notch activation requires that the six cdc10/ankyrin repeats of the Notch intracellular domain reach the nucleus and participate in transcriptional activation. The site of proteolytic cleavage on the intracellular tail of Notch has been identified between gly1743 and val1744 (termed site 3, or S3) (Schroeter, E.H. et al. (1998) Nature 393(6683):382-6). It is thought that the proteolytic cleavage step that releases the cdc10/ankyrin repeats for nuclear entry is dependent on Presenilin activity.

The intracellular domain has been shown to accumulate in the nucleus where it forms a transcriptional activator complex with the CSL family protein CBF1 (suppressor of hairless, Su(H) in *Drosophila,* Lag-2 in C. *elegans*) (Schroeter, E.H. et al. (1998) Nature 393(6683):382-6; Struhl, G. et al. (1998) Cell 93(4):649-60). The NotchIC-CBF1 complexes then activate target genes, such as the bHLH proteins HES (hairy-enhancer of split like) 1 and 5 (Weinmaster G. (2000) Curr. Opin. Genet. Dev. 10:363-369). This nuclear function of Notch has also been shown for the mammalian Notch homologue (Lu, F. M. et al. (1996) Proc Natl Acad Sci 93(11):5663-7).

S3 processing occurs only in response to binding of Notch ligands Delta or Serrate/Jagged. The post-translational modification of the nascent Notch receptor in the Golgi (Munro S, Freeman M. (2000) Curr. Biol. 10:813-820; Ju BJ, et al. (2000) Nature 405:191-195) appears, at least in part, to control which of the two types of ligand is expressed on a cell surface. The Notch receptor is modified on its extracellular domain by Fringe, a glycosyl transferase enzyme that binds to the Lin/Notch motif. Fringe modifies Notch by adding O-linked fucose groups to the EGF-like repeats (Moloney DJ, et al. (2000) Nature 406:369-375; Brucker K, et al. (2000) Nature 406:411-415). This modification by Fringe does not prevent ligand binding, but may influence ligand induced conformational changes in Notch. Furthermore, recent studies suggest that the action of Fringe modifies Notch to prevent it from interacting functionally with Serrate/Jagged ligands but allow it to preferentially bind Delta (Panin VM, et al. (1997) Nature 387:908-912; Hicks C, et al. (2000) Nat. Cell. Biol. 2:515-520). Although *Drosophila* has a single Fringe gene, vertebrates are known to express multiple genes (Radical, Manic and Lunatic Fringes) (Irvine KD (1999) Curr. Opin. Genet. Devel. 9:434-441).

Signal transduction from the Notch receptor can occur via two different pathways. The better defined pathway involves proteolytic cleavage of the intracellular domain of Notch (Notch IC) that translocates to the nucleus and forms a transcriptional activator complex with the CSL family protein CBF1 (suppressor of Hairless, Su(H) in *Drosophila,* Lag-2 in C. *elegans).* NotchIC-CBF1 complexes then activate target genes, such as the bHLH proteins HES (hairy-enhancer of split like) 1 and 5. Notch can also signal in a CBF1-independent manner that involves the cytoplasmic zinc finger containing protein Deltex. Unlike CBF1, Deltex does not move to the nucleus following Notch activation but instead can interact with Grb2 and modulate the Ras-JNK signalling pathway.

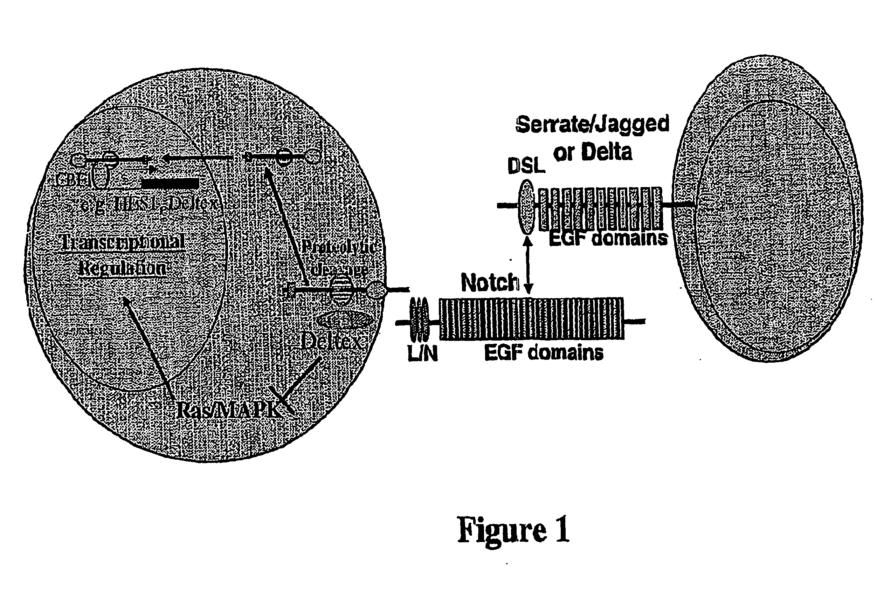

Thus, signal transduction from the Notch receptor can occur via two different pathways both of which are illustrated in Figure 1. Target genes of the Notch signalling pathway include Deltex, genes of the Hes family (Hes-1 in particular), Enhancer of Split [E(spl)] complex genes, IL-10, CD-23, CD-4 and DII-1.

Deltex, an intracellular docking protein, replaces Su(H) as it leaves its site of interaction with the intracellular tail of Notch. Deltex is a cytoplasmic protein containing a zinc-finger (Artavanis-Tsakonas; Osborne B, Miele L. (1999) Immunity 11:653-663). It interacts with the ankyrin repeats of the Notch intracellular domain. Studies indicate that Deltex promotes Notch pathway activation by interacting with Grb2 and modulating the Ras-JNK signalling pathway (Matsuno K, et al. (1998) Nat. Genet. 19:74-78; Matsuno, K. et al. (1995) Development 121(8 :2633-44). Deltex also acts as a docking protein which prevents Su(H) from binding to the intracellular tail of Notch (Matsuno). Thus, Su(H) is released into the nucleus where it acts as a transcriptional modulator. Recent evidence also suggests that, in a vertebrate B-cell system, Deltex, rather than the Su(H) homologue CBF1, is responsible for inhibiting E47 function (Ordentlich et al. (1998) Mol. Cell. Biol. 18:2230-2239). Expression of Deltex is upregulated as a result of Notch activation in a positive feedback loop. The sequence of Homo sapiens Deltex (DTX1) mRNA may be found in GenBank Accession No. AF053700.

Hes-1 (Hairy-enhancer of Split-1) (Takebayashi K. et al. (1994) J Biol Chem 269(7):150-6) is a transcriptional factor with a basic helix-loop-helix structure. It binds to an important functional site in the CD4 silencer leading to repression of CD4 gene expression. Thus, Hes-1 is strongly involved in the determination of T-cell fate. Other genes from the Hes family include Hes-5 (mammalian Enhancer of Split homologue), the expression of which is also upregulated by Notch activation, and Hes-3. Expression of Hes-1 is upregulated as a result of Notch activation. The sequence of Mus musculus Hes-1 can be found in GenBank Accession No. D 16464.

The E(spl) gene complex [E(spl)-C] (Leimeister C. et al. (1999) Mech Dev 85(1-2) :173-7) comprises seven genes of which only E(spl) and Groucho show visible phenotypes when mutant. E(spl) was named after its ability to enhance Split mutations, Split being another name for Notch. Indeed, E(spl)-C genes repress Delta through regulation of achaete-scute complex gene expression. Expression of E(spl) is upregulated as a result of Notch activation.

IL-10 (interleukin-10) is a factor produced by Th2 helper T-cells. It is a co-regulator of mast cell growth and shows extensive homology with the Epstein-Barr bcrfi gene. Although it is not known to be a direct downstream target of the Notch signalling pathway, its expression has been found to be strongly upregulated coincident with Notch activation. The mRNA sequence of IL-10 may be found in GenBank ref. No. GI1041812.

CD-23 is the human leukocyte differentiation antigen CD23 (FCE2) which is a key molecule for B-cell activation and growth. It is the low-affinity receptor for IgE. Furthermore, the truncated molecule can be secreted, then functioning as a potent mitogenic growth factor. Although it is not thought to be a direct downstream target of the Notch signalling pathway, its expression has been found to be strongly upregulated coincident with Notch activation. The sequence for CD-23 may be found in GenBank ref. No. GI1783344.

Dlx-1 (distalless-1) (McGuinness T. Et al (1996) Genomics 35(3):473-85) expression is downregulated as a result of Notch activation. Sequences for Dlx genes may be found in GenBank Accession Nos. U51000-3.

CD-4 expression is downregulated as a result of Notch activation. A sequence for the CD-4 antigen may be found in GenBank Accession No. XM006966.

Other genes involved in the Notch signaling pathway, such as Numb, Mastermind and Dsh, and all genes the expression of which is modulated by Notch activation, are included in the scope of this invention.

### Polypeptides and Polynucleotides for Notch Signalling Activation

Preferred agents for activating Notch signalling include Notch ligands.

Particular examples of mammalian Notch ligands identified to date include the Delta family, for example Delta or Delta-like 1 (Genbank Accession No. AF003522 - *Homo sapiens*)*,* Delta-3 (Genbank Accession No. AF084576 - *Rattus norvegicus*) and Delta-like *3 (Mus musculus*) (Genbank Accession No. NM_016941 - *Homo sapiens*) and US 6121045 (Millennium), Delta-4 (Genbank Accession Nos. AB043894 and AF 253468 - *Homo sapiens*) and the Serrate family, for example Serrate-1 and Serrate-2 (WO97/01571, WO96/27610 and WO92/19734), Jagged-1 (Genbank Accession No. U73936 - *Homo sapiens*) and Jagged-2 (Genbank Accession No. AF029778 - *Homo sapiens*)*,* and LAG-2. Homology between family members is extensive.

Further homologues of known mammalian Notch ligands may be identified using standard techniques. By a "homologue" it is meant a gene product that exhibits sequence homology, either amino acid or nucleic acid sequence homology, to any one of the known Notch ligands, for example as mentioned above. Typically, a homologue of a known Notch ligand will be at least 20%, preferably at least 30%, identical at the amino acid level to the corresponding known Notch ligand over a sequence of at least 10, preferably at least 20, preferably at least 50, suitably at least 100 amino acids, or over the entire length of the Notch ligand. Techniques and software for calculating sequence homology between two or more amino acid or nucleic acid sequences are well known in the art (see for example http://www.ncbi.nim.nih.gov and Ausubel et al., Current Protocols in Molecular Biology (1995), John Wiley & Sons, Inc.)

Notch ligands identified to date have a diagnostic DSL domain (D. *Delta, S. Serrate,* L*.* Lag2) comprising 20 to 22 amino acids at the amino terminus of the protein and up to 14 or more EGF-like repeats on the extracellular surface. It is therefore preferred that homologues of Notch ligands also comprise a DSL domain and up to 14 or more EGF-like repeats on the extracellular surface.

In addition, suitable homologues will be capable of binding to a Notch receptor. Binding may be assessed by a variety of techniques known in the art including *in vitro* binding assays.

Homologues of Notch ligands can be identified in a number of ways, for example by probing genomic or cDNA libraries with probes comprising all or part of a nucleic acid encoding a Notch ligand under conditions of medium to high stringency (for example 0.03M sodium chloride and 0.03M sodium citrate at from about 50°C to about 60°C). Alternatively, homologues may also be obtained using degenerate PCR which will generally use primers designed to target sequences within the variants and homologues encoding conserved amino acid sequences. The primers will contain one or more degenerate positions and will be used at stringency conditions lower than those used for cloning sequences with single sequence primers against known sequences.

Other substances capable of activating the Notch signalling pathway include compounds capable of upregulating Notch ligand expression including polypeptides that bind to and reduce or neutralise the activity of bone morphogenetic proteins (BMPs). Binding of extracellular BMPs (Wilson and Hemmati-Brivanlou (1997) Neuron 18:699-710; Hemmati-Brivanlou and Melton (1997) Cell 88:13-17) to their receptors leads to down-regulated Delta transcription due to the inhibition of the expression of transcription factors of the achaete/scute complex. This complex is believed to be directly involved in the regulation of Delta expression. Thus, any substance that inhibits BMP expression and/or inhibits the binding of BMPs to their receptors may be capable of producing an increase in the expression of Notch ligands such as Delta and/or Serrate. Particular examples of such inhibitors include Noggin (Valenzuela et al. (1995) J. Neurosci. 15:6077-6084), Chordin (Sasai et al. (1994) Cell 79:779-790), Follistatin (Iemura et al. (1998) PNAS 95:9337-9345), Xnr3, and derivatives and variants thereof Noggin and Chordin bind to BMPs thereby preventing activation of their signalling cascade which leads to decreased Delta transcription. Consequently, increasing Noggin and Chordin levels may lead to increased Notch ligand, in particular Delta, expression.

Furthermore, any substance that upregulates expression of transcription factors of the achaete/scute complex may also upregulate Notch ligand expression.

Other suitable substances that may be used to upregulate Notch ligand expression include transforming growth factors such as members of the fibroblast growth factor (FGF) family. The FGF may be a mammalian basic FGF, acidic FGF or another member of the FGF family such as an FGF-1, FGF-2, FGF-3, FGF-4, FGF-5, FGF-6, FGF-7. Preferably the FGF is not acidic FGF (FGF-1: Zhao et al. (1995) J. Immunol. 155:3904-3911). Most preferably, the FGF is a member of the FGF family which acts by stimulating the upregulation of expression of a Serrate polypeptide on APCs. It has been shown that members of the FGF family can upregulate Serrate-1 gene expression in APCs.

Immunosuppressive cytokines may also be used to upregulate Notch ligand expression. Examples include members of the TGF-β family such as TGF-β-1 and TGF-β-2, and interleukins such as IL-4, IL-10, IL-4 and IL-13, and FLT3 ligand. The TGF-β family can upregulate Notch, particularly Notch 1, expression; IL-10 can upregulate Serrate, particularly Serrate 1, expression; IL-10 can upregulate Notch, Delta and Serrate, particularly Notch 2, Notch 4, Delta 1 and Serrate 1, expression; and IL-10 can upregulate Serrate, particularly Serrate 1, expression.

The substance capable of upregulating expression of Notch or a Notch ligand may be selected from polypeptides and fragments thereof, linear peptides, cyclic peptides, including synthetic and natural compounds. The substances capable of upregulating expression of a Notch ligand may be derived from a biological material such as a component of extracellular matrix. Suitable extracellular matrix components are derived from immunologically privileged sites such as the eye. For example aqueous humour or components thereof may be used.

Polypeptide substances such as Noggin, FGFs and TGF-β may be purified from mammalian cells, obtained by recombinant expression in suitable host cells or obtained commercially. Alternatively, nucleic acid constructs encoding the polypeptides may be used. As a further example, overexpression of Notch or Notch ligand, such as Delta or Serrate, may be brought about by introduction of a nucleic acid construct capable of activating the endogenous gene, such as the Serrate or Delta gene. In particular, gene activation can be achieved by the use of homologous recombination to insert a heterologous promoter in place of the natural promoter, such as the Serrate or Delta promoter, in the genome of the target cell.

The activating molecule of the present invention may, in an alternative embodiment, be capable of modifying Notch-protein expression or presentation on the cell membrane or signalling pathways. Agents that enhance the presentation of a fully functional Notch-protein on the target cell surface include matrix metalloproteinases such as the product of the Kuzbanian gene of Drosophila (Dkuz et al (1997) Cell 90: 271-280) and other ADAMALYSIN gene family members.

### Notch ligand domains

As discussed above, Notch ligands typically comprise a number of distinctive domains. Some predicted/potential domain locations for various naturally occurring human Notch ligands (based on amino acid numbering in the precursor proteins) are shown below:

### Human Delta 1

| Component | Amino acids | Proposed function/domain |
|---|---|---|
| SIGNAL | 1-17 | SIGNAL |
| CHAIN | 18-723 | DELTA-LIKE PROTEIN 1 |
| DOMAIN | 18-545 | EXTRACELLULAR |
| TRANSMEM | 546- 568 | TRANSMEMBRANE |
| DOMAIN | 569-723 | CYTOPLASMIC |
| DOMAIN | 159-221 | DSL |
| DOMAIN | 226-254 | EGF-LIKE 1 |
| DOMAIN | 257-285 | EGF-LIKE 2 |
| DOMAIN | 292-325 | EGF-LIKE 3 |
| DOMAIN | 332-363 | EGF-LIKE 4 |
| DOMAIN | 370-402 | EGF-LIKE 5 |
| DOMAIN | 409-440 | EGF-LIKE 6 |
| DOMAIN | 447-478 | EGF-LIKE 7 |
| DOMAIN | 485-516 | EGF-LIKE 8 |

### Human Delta 3

| Component | Amino acids | Proposed function/domain |
|---|---|---|
| DOMAIN | 158-248 | DSL |
| DOMAIN | 278-309 | EGF-LIKE 1 |
| DOMAIN | 316-350 | EGF-LIKE 2 |
| DOMAIN | 357-388 | EGF-LIKE 3 |
| DOMAIN | 395-426 | EGF-LIKE 4 |
| DOMAIN | 433-464 | EGF-LIKE 5 |

### Human Delta 4

| Component | Amino acids | Proposed function/domain |
|---|---|---|
| SIGNAL | 1-26 | SIGNAL |
| CHAIN | 27-685 | DELTA-LIKE PROTEIN 4 |
| DOMAIN | 27-529 | EXTRACELLULAR |
| TRANSMEM | 530-550 | TRANSMEMBRANE |
| DOMAIN | 551-685 | CYTOPLASMIC |
| DOMAIN | 155-217 | DSL |
| DOMAIN | 218-251 | EGF-LIKE 1 |
| DOMAIN | 252-282 | EGF-LIKE 2 |
| DOMAIN | 284-322 | EGF-LIKE 3 |
| DOMAIN | 324-360 | EGF-LIKE 4 |
| DOMAIN | 362-400 | EGF-LIKE 5 |
| DOMAIN | 402-438 | EGF-LIKE 6 |
| DOMAIN | 440-476 | EGF-LIKE 7 |
| DOMAIN | 480-518 | EGF-LIKE 8 |

### Human Jagged 1

| Component | Amino acids | Proposed function/domain |
|---|---|---|
| SIGNAL | 1-33 | SIGNAL |
| CHAIN | 34-1218 | JAGGED 1 |
| DOMAIN | 34-1067 | EXTRACELLULAR |
| TRANSMEM | 1068-1093 | TRANSMEMBRANE |
| DOMAIN | 1094-1218 | CYTOPLASMIC |
| DOMAIN | 167-229 | DSL |
| DOMAIN | 234-262 | EGF-LIKE 1 |
| DOMAIN | 265-293 | EGF-LIKE 2 |
| DOMAIN | 300-333 | EGF-LIKE 3 |
| DOMAIN | 340-371 | EGF-LIKE 4 |
| DOMAIN | 378-409 | EGF-LIKE 5 |
| DOMAIN | 416-447 | EGF-LIKE 6 |
| DOMAIN | 454-484 | EGF-LIKE 7 |
| DOMAIN | 491-522 | EGF-LIKE 8 |
| DOMAIN | 529-560 | EGF-LIKE 9 |
| DOMAIN | 595-626 | EGF-LIKE 10 |
| DOMAIN | 633-664 | EGF-LIKE 11 |
| DOMAIN | 671-702 | EGF-LIKE 12 |
| DOMAIN | 709-740 | EGF-LIKE 13 |
| DOMAIN | 748-779 | EGF-LIKE 14 |
| DOMAIN | 786-817 | EGF-LIKE 15 |
| DOMAIN | 824-855 | EGF-LIKE 16 |
| DOMAIN | 863-917 | VON WILLEBRAND FACTOR C |

### Human Jagged 2

| Component | Amino acids | Proposed function/domain |
|---|---|---|
| SIGNAL | 1-26 | SIGNAL |
| CHAIN | 27-1238 | JAGGED 2 |
| DOMAIN | 27-1080 | EXTRACELLULAR |
| TRANSMEM | 1081-1105 | TRANSMEMBRANE |
| DOMAIN | 1106-1238 | CYTOPLASMIC |
| DOMAIN | 178-240 | DSL |
| DOMAIN | 249-273 | EGF-LIKE 1 |
| DOMAIN | 276-304 | EGF-LIKE 2 |
| DOMAIN | 311-344 | EGF-LIKE 3 |
| DOMAIN | 351-382 | EGF-LIKE 4 |
| DOMAIN | 389-420 | EGF-LIKE 5 |
| DOMAIN | 427-458 | EGF-LIKE 6 |
| DOMAIN | 465-495 | EGF-LIKE 7 |
| DOMAIN | 502-533 | EGF-LIKE 8 |
| DOMAIN | 540-571 | EGF-LIKE 9 |
| DOMAIN | 602-633 | EGF-LIKE 10 |
| DOMAIN | 640-671 | EGF-LIKE 11 |
| DOMAIN | 678-709 | EGF-LIKE 12 |
| DOMAIN | 716-747 | EGF-LIKE 13 |
| DOMAIN | 755-786 | EGF-LIKE 14 |
| DOMAIN | 793-824 | EGF-LIKE 15 |
| DOMAIN | 831-862 | EGF-LIKE 16 |
| DOMAIN | 872-949 | VON WILLEBRAND FACTOR C |

### DSL domain

A typical DSL domain may include most or all of the following consensus amino acid sequence:

Cys Xaa Xaa Xaa Xaa Xaa Xaa Xaa Xaa Cys Xaa Xaa Xaa Cys Xaa Xaa
Xaa Xaa Xaa Xaa Xaa Xaa Xaa Xaa Xaa Cys Xaa Xaa Xaa Xaa Xaa Xaa
Xaa Cys Xaa Xaa Xaa Xaa Xaa Xaa Xaa Xaa Cys

Preferably the DSL domain may include most or all of the following consensus amino acid sequence: 
Cys Xaa Xaa Xaa ARO ARO Xaa Xaa Xaa Cys Xaa Xaa Xaa Cys BAS NOP
BAS ACM ACM Xaa ARO NOP ARO Xaa Xaa Cys Xaa Xaa Xaa NOP Xaa Xaa
Xaa Cys Xaa Xaa NOP ARO Xaa NOP Xaa Xaa Cys
 wherein:
ARO is an aromatic amino acid residue, such as tyrosine, phenylalanine, tryptophan or histidine;
NOP is a non-polar amino acid residue such as glycine, alanine, proline, leucine, isoleucine or valine;
BAS is a basic amino acid residue such as arginine or lysine; and
ACM is an acid or amide amino acid residue such as aspartic acid, glutamic acid, asparagine or glutamine.

Preferably the DSL domain may include most or all of the following consensus amino acid sequence: 
Cys Xaa Xaa Xaa Tyr Tyr Xaa Xaa Xaa Cys Xaa Xaa Xaa Cys Arg Pro
Arg Asx Asp Xaa Phe Gly His Xaa Xaa Cys Xaa Xaa Xaa Gly Xaa Xaa
Xaa Cys Xaa Xaa Gly Trp Xaa Gly Xaa Xaa Cys.
 (wherein Xaa may be any amino acid and Asx is either aspartic acid or asparagine).

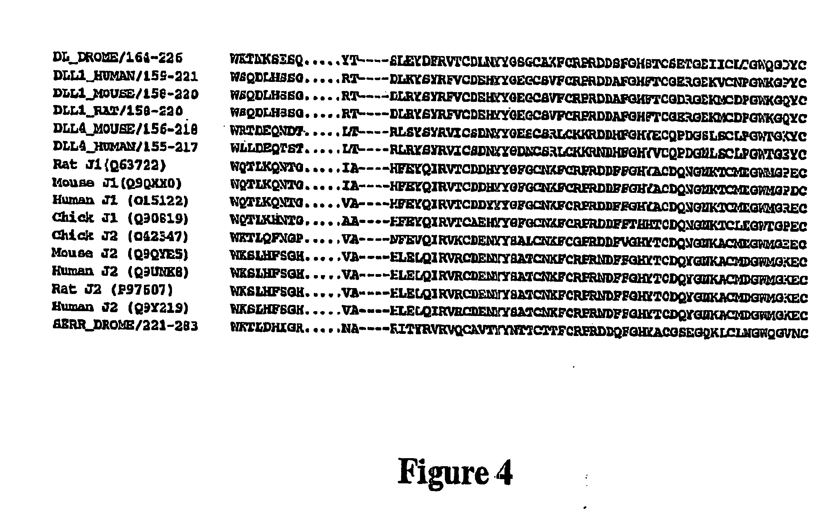

An alignment of DSL domains from Notch ligands from various sources is shown in Figure 4.

The DSL domain used may be derived from any suitable species, including for example Drosophila, Xenopus, rat, mouse or human. Preferably the DSL domain is derived from a vertebrate, preferably a mammalian, preferably a human Notch ligand sequence.

It will be appreciated that the term "DSL domain" as used herein includes sequence variants, fragments, derivatives and mimetics having activity corresponding to naturally occurring domains.

Suitably, for example, a DSL domain for use in the present invention may have at least 30%, preferably at least 50%, preferably at least 60%, preferably at least 70%, preferably at least 80%, preferably at least 90%, preferably at least 95% amino acid sequence identity to the DSL domain of human Jagged 1.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Jagged 2.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 1.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 3.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 4.

### EGF-like domain

The EGF-like motif has been found in a variety of proteins, as well as EGF and Notch and Notch ligands, including those involved in the blood clotting cascade (Furie and Furie, 1988, Cell 53: 505-518). For example, this motif has been found in extracellular proteins such as the blood clotting factors IX and X (Rees et al., 1988, EMBO J. 7:2053-2061; Furie and Furie, 1988, Cell 53: 505-518), in other Drosophila genes (Knust et al., 1987 EMBO J. 761-766; Rothberg et al., 1988, Cell 55:1047-1059), and in some cell-surface receptor proteins, such as thrombomodulin (Suzuki et al., 1987, EMBO J. 6:1891-1897) and LDL receptor (Sudhof et al., 1985, Science 228:815-822). A protein binding site has been mapped to the EGF repeat domain in thrombomodulin and urokinase (Kurosawa et al., 1988, J. Biol. Chem 263:5993-5996; Appella et al., 1987, J. Biol. Chem. 262:4437-4440).

As reported by PROSITE a typical EGF domain may include six cysteine residues which have been shown (in EGF) to be involved in disulfide bonds. The main structure is proposed, but not necessarily required, to be a two-stranded beta-sheet followed by a loop to a C-terminal short two-stranded sheet. Subdomains between the conserved cysteines strongly vary in length as shown in the following schematic representation of a typical EGF-like domain: 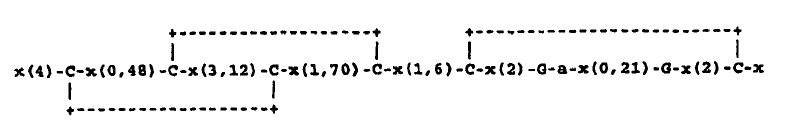 wherein:
'C': conserved cysteine involved in a disulfide bond.
'G': often conserved glycine
'a': often conserved aromatic amino acid
'x': any residue

The region between the 5th and 6th cysteine contains two conserved glycines of which at least one is normally present in most EGF-like domains.

The EGF-like domain used may be derived from any suitable species, including for example Drosophila, Xenopus, rat, mouse or human. Preferably the EGF-like domain is derived from a vertebrate, preferably a mammalian, preferably a human Notch ligand sequence.

It will be appreciated that the term "EGF domain" as used herein includes sequence variants, fragments, derivatives and mimetics having activity corresponding to naturally occurring domains.

Suitably, for example, an EGF-like domain for use in the present invention may have at least 30%, preferably at least 50%, preferably at least 60%, preferably at least 70%, preferably at least 80%, preferably at least 90%, preferably at least 95% amino acid sequence identity to an EGF-like domain of human Jagged 1.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Jagged 2.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 1.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 3.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 4.

As a practical matter, whether any particular amino acid sequence is at least X% identical to another sequence can be determined conventionally using known computer programs. For example, the best overall match between a query sequence and a subject sequence, also referred to as a global sequence alignment, can be determined using a program such as the FASTDB computer program based on the algorithm of Brutlag et al. (Comp. App. Biosci. (1990) 6:237-245). In a sequence alignment the query and subject sequences are either both nucleotide sequences or both amino acid sequences. The result of the global sequence alignment is given as percent identity. Preferably the required sequence homology, similarity or identity occurs over a stretch of at least 30, preferably at least 50, preferably at least 100 amino acid or nucleotide residues and/or over substantially the entire length of the reference sequence.

The term "Notch ligand N-terminal domain" means the part of a Notch ligand sequence from the N-terminus to the start of the DSL domain. It will be appreciated that this term includes sequence variants, fragments, derivatives and mimetics having activity corresponding to naturally occurring domains.

The term "transmembrane domain" includes a domain which is retained within a cell membrane, which preferably anchors the protein or polypeptide to the membrane when expressed.

The term "membrane binding domain" includes a domain which binds to a cell membrane without necessarily passing through it, or passing entirely through it. The term "heterologous amino acid sequence" or "heterologous nucleotide sequence" as used herein means a sequence which is not found in the native Notch ligand or its coding sequence.

Whether an agent can be used for activating a Notch receptor may be determined using suitable screening assays, for example, as described in our co-pending International Patent Application claiming priority from GB 0118153.6 (WO 03/012441, Lorantis, eg Example 8) and the Examples herein.

Activation of Notch signalling may also be achieved by repressing inhibitors of the Notch signalling pathway. As such, polypeptides for Notch signalling activation will include molecules capable of repressing any Notch signalling inhibitors. Preferably the molecule will be a polypeptide, or a polynucleotide encoding such a polypeptide, that decreases or interferes with the production or activity of compounds that are capable of producing an decrease in the expression or activity of Notch, Notch ligands, or any downstream components of the Notch signalling pathway. In a preferred embodiment, the molecules will be capable of repressing polypeptides of the Toll-like receptor protein family and growth factors such as the bone morphogenetic protein (BMP), BMP receptors and activins, derivatives, fragments, variants and homologues thereof.

### Polypeptides and Polynucleotides for Notch Signalling Inhibition

Substances that may be used to modulate Notch signalling by inhibiting Notch ligand expression include nucleic acid sequences encoding polypeptides that affect the expression of genes encoding Notch ligands. For instance, for Delta expression, binding of extracellular BMPs (bone morphogenetic proteins, Wilson and Hemmati-Brivanlou; Hemmati-Brivanlou and Melton) to their receptors leads to down-regulated Delta transcription due to the inhibition of the expression of transcription factors of the achaete/scute complex. This complex is believed to be directly involved in the regulation of Delta expression. Thus, any polypeptide that upregulates BMP expression and/or stimulates the binding of BMPs to their receptors may be capable of producing a decrease in the expression of Notch ligands such as Delta and/or Serrate. Examples may include nucleic acids encoding BMPs themselves. Furthermore, any substance that inhibits expression of transcription factors of the achaete/scute complex may also downregulate Notch ligand expression.

Members of the BMP family include BMP1 to BMP6, BMP7 also called OP1, OP2 (BMP8) and others. BMPs belong to the transforming growth factor beta (TGF-beta) superfamily, which includes, in addition to the TGF-betas, activins/inhibins (e.g., alpha-inhibin), mullerian inhibiting substance, and glial cell line-derived neurotrophic factor.

Other examples of polypeptides that inhibit the expression of Delta and/or Serrate include the Toll-like receptor (Medzhitov) or any other receptors linked to the innate immune system (for example CD14, complement receptors, scavenger receptors or defensin proteins), and other polypeptides that decrease or interfere with the production of Noggin (Valenzuela), Chordin (Sasai), Follistatin (Iemura), Xnr3, and derivatives and variants thereof. Noggin and Chordin bind to BMPs thereby preventing activation of their signalling cascade which leads to decreased Delta transcription. Consequently, reducing Noggin and Chordin levels may lead to decrease Notch ligand, in particular Delta, expression.

In more detail, in Drosophila, the Toll transmembrane receptor plays a central role in the signalling pathways that control amongst other things the innate nonspecific immune response. This Toll-mediated immune response reflects an ancestral conserved signalling system that has homologous components in a wide range of organisms. Human Toll homologues have been identified amongst the Toll-like receptor (TLR) genes and Toll/interleukin-1 receptor-like (TIL) genes and contain the characteristic Toll motifs: an extracellular leucine-rich repeat domain and a cytoplasmic interleukin-1 receptor-like region. The Toll-like receptor genes (including TIL genes) now include TLR4, TIL3, TIL4, and 4 other identified TLR genes.

Other suitable sequences that may be used to downregulate Notch ligand expression include those encoding immune costimulatory molecules (for example CD80, CD86, ICOS, SLAM) and other accessory molecules that are associated with immune potentiation (for example CD2, LFA-1).

Other suitable substances that may be used to downregulate Notch ligand expression include nucleic acids that inhibit the effect of transforming growth factors such as members of the fibroblast growth factor (FGF) family. The FGF may be a mammalian basic FGF, acidic FGF or another member of the FGF family such as an FGF-1, FGF-2, FGF-3, FGF-4, FGF-5, FGF-6, FGF-7. Preferably the FGF is not acidic FGF (FGF-1; Zhao *et al.,* 1995). Most preferably, the FGF is a member of the FGF family which acts by stimulating the upregulation of expression of a Serrate polypeptide on APCs. It has been shown that members of the FGF family can upregulate Serrate-1 gene expression in APCs.

Suitable nucleic acid sequences may include anti-sense constructs, for example nucleic acid sequences encoding antisense Notch ligand constructs as well as antisense constructs designed to reduce or inhibit the expression of upregulators of Notch ligand expression (see above). The antisense nucleic acid may be an oligonucleotide such as a synthetic single-stranded DNA. However, more preferably, the antisense is an antisense RNA produced in the patient's own cells as a result of introduction of a genetic vector. The vector is responsible for production of antisense RNA of the desired specificity on introduction of the vector into a host cell.

In one embodiment, a nucleic acid sequence for use in the present invention is capable of inhibiting Serrate and Delta, preferably Serrate 1 and Serrate 2 as well as Delta 1, Delta 3 and Delta 4 expression in APCs such as dendritic cells. In particular, the nucleic acid sequence may be capable of inhibiting Serrate expression but not Delta expression, or Delta but not Serrate expression in APCs or T cells. Alternatively, the nucleic acid sequence for use in the present invention may be capable of inhibiting Delta expression in T cells such as CD4⁺ helper T cells or other cells of the immune system that express Delta (for example in response to stimulation of cell surface receptors). In particular, the nucleic acid sequence may be capable of inhibiting Delta expression but not Serrate expression in T cells. In a particularly preferred embodiment, the nucleic acid sequence is capable of inhibiting Notch ligand expression in both T cells and APC, for example Serrate expression in APCs and Delta expression in T cells.

Preferred suitable substances that may be used to downregulate Notch ligand expression include growth factors and cytokines. More preferably soluble protein growth factors may be used to inhibit Notch or Notch ligand expression. For instance, Notch ligand expression may be reduced or inhibited by the addition of BMPs or activins (a member of the TGF-β superfamily). In addition, T cells, APCs or tumour cells could be cultured in the presence of inflammatory type cytokines including IL-12, IFN-γ, IL-18, TNF-α, either alone or in combination with BMPs.

Molecules for inhibition of Notch signalling will also include polypeptides, or polynucleotides which encode therefore, capable of modifying Notch-protein expression or presentation on the cell membrane or signalling pathways. Molecules that reduce or interfere with its presentation as a fully functional cell membrane protein may include MMP inhibitors such as hydroxymate-based inhibitors.

Other substances which may be used to reduce interaction between Notch and Notch ligands are exogenous Notch or Notch ligands or functional derivatives thereof. Such Notch ligand derivatives would preferably have the DSL domain at the N-terminus and up to about 14 or more, for example between about 1 to 8 EGF-like repeats on the extracellular surface. A peptide corresponding to the Delta/Serrate/LAG-2 domain of hJagged1 and supernatants from COS cells expressing a soluble form of the extracellular portion of hJagged1 was found to mimic the effect of Jagged 1 in inhibiting Notch 1 (Li et al. (1998) Immunity 8(1):43-55).

Other Notch signalling pathway antagonists include antibodies which inhibit interactions between components of the Notch signalling pathway, e.g. antibodies to Notch ligands. Whether a substance can be used for modulating Notch-Notch ligand expression may be determined using suitable screening assays.

### Monitoring of Notch Signalling: Screens and Assays

Notch signalling can be monitored either through protein assays or through nucleic acid assays. Activation of the Notch receptor leads to the proteolytic cleavage of its cytoplasmic domain and the translocation thereof into the cell nucleus. The "detectable signal" referred to herein may be any detectable manifestation attributable to the presence of the cleaved intracellular domain of Notch. Thus, increased Notch signalling can be assessed at the protein level by measuring intracellular concentrations of the cleaved Notch domain. Activation of the Notch receptor also catalyses a series of downstream reactions leading to changes in the levels of expression of certain well defined genes. Thus, increased Notch signalling can be assessed at the nucleic acid level by say measuring intracellular concentrations of specific mRNAs. In one preferred embodiment of the present invention, the assay is a protein assay. In another preferred embodiment of the present invention, the assay is a nucleic acid assay.

The advantage of using a nucleic acid assay is that they are sensitive and that small samples can be analysed.

The intracellular concentration of a particular mRNA, measured at any given time, reflects the level of expression of the corresponding gene at that time. Thus, levels of mRNA of downstream target genes of the Notch signalling pathway can be measured in an indirect assay of the T-cells of the immune system. In particular, an increase in levels of Deltex, Hes-1 and/or IL-10 mRNA may, for instance, indicate induced anergy while an increase in levels of Dll-1 or IFN-γ mRNA, or in the levels of mRNA encoding cytokines such as IL-2, IL-5 and IL-13, may indicate improved responsiveness.

Various nucleic acid assays are known. Any convention technique which is known or which is subsequently disclosed may be employed. Examples of suitable nucleic acid assay are mentioned below and include amplification, PCR, RT-PCR, RNase protection, blotting, spectrometry, reporter gene assays, gene chip arrays and other hybridization methods.

In particular, gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe. Those skilled in the art will readily envisage how these methods may be modified, if desired.

PCR was originally developed as a means of amplifying DNA from an impure sample. The technique is based on a temperature cycle which repeatedly heats and cools the reaction solution allowing primers to anneal to target sequences and extension of those primers for the formation of duplicate daughter strands. RT-PCR uses an RNA template for generation of a first strand cDNA with a reverse transcriptase. The cDNA is then amplified according to standard PCR protocol. Repeated cycles of synthesis and denaturation result in an exponential increase in the number of copies of the target DNA produced. However, as reaction components become limiting, the rate of amplification decreases until a plateau is reached and there is little or no net increase in PCR product. The higher the starting copy number of the nucleic acid target, the sooner this "end-point" is reached.

Real-time PCR uses probes labeled with a fluorescent tag or fluorescent dyes and differs from end-point PCR for quantitative assays in that it is used to detect PCR products as they accumulate rather than for the measurement of product accumulation after a fixed number of cycles. The reactions are characterized by the point in time during cycling when amplification of a target sequence is first detected through a significant increase in fluorescence.

The ribonuclease protection (RNase protection) assay is an extremely sensitive technique for the quantitation of specific RNAs in solution. The ribonuclease protection assay can be performed on total cellular RNA or poly(A)-selected mRNA as a target. The sensitivity of the ribonuclease protection assay derives from the use of a complementary *in vitro* transcript probe which is radiolabeled to high specific activity. The probe and target RNA are hybridized in solution, after which the mixture is diluted and treated with ribonuclease (RNase) to degrade all remaining single-stranded RNA. The hybridized portion of the probe will be protected from digestion and can be visualized via electrophoresis of the mixture on a denaturing polyacrylamide gel followed by autoradiography. Since the protected fragments are analyzed by high resolution polyacrylamide gel electrophoresis, the ribonuclease protection assay can be employed to accurately map mRNA features. If the probe is hybridized at a molar excess with respect to the target RNA, then the resulting signal will be directly proportional to the amount of complementary RNA in the sample.

Gene expression may also be detected using a reporter system. Such a reporter system may comprise a readily identifiable marker under the control of an expression system, e.g. of the gene being monitored. Fluorescent markers, which can be detected and sorted by FACS, are preferred. Especially preferred are GFP and luciferase. Another type of preferred reporter is cell surface markers, i.e. proteins expressed on the cell surface and therefore easily identifiable.

In general, reporter constructs useful for detecting Notch signalling by expression of a reporter gene may be constructed according to the general teaching of Sambrook et al (1989). Typically, constructs according to the invention comprise a promoter by the gene of interest, and a coding sequence encoding the desired reporter constructs, for example of GFP or luciferase. Vectors encoding GFP and luciferase are known in the art and available commercially.

Sorting of cells, based upon detection of expression of genes, may be performed by any technique known in the art, as exemplified above. For example, cells may be sorted by flow cytometry or FACS. For a general reference, see Flow Cytometry and Cell Sorting: A Laboratory Manual (1992) A. Radbruch (Ed.), Springer Laboratory, New York.

Flow cytometry is a powerful method for studying and purifying cells. It has found wide application, particularly in immunology and cell biology: however, the capabilities of the FACS can be applied in many other fields of biology. The acronym F.A.C.S. stands for Fluorescence Activated Cell Sorting, and is used interchangeably with "flow cytometry". The principle of FACS is that individual cells, held in a thin stream of fluid, are passed through one or more laser beams, causing light to be scattered and fluorescent dyes to emit light at various frequencies. Photomultiplier tubes (PMT) convert light to electrical signals, which are interpreted by software to generate data about the cells. Subpopulations of cells with defined characteristics can be identified and automatically sorted from the suspension at very high purity (∼100%).

FACS can be used to measure gene expression in cells transfected with recombinant DNA encoding polypeptides. This can be achieved directly, by labelling of the protein product, or indirectly by using a reporter gene in the construct. Examples of reporter genes are β-galactosidase and Green Fluorescent Protein (GFP). β-galactosidase activity can be detected by FACS using fluorogenic substrates such as fluorescein digalactoside (FDG). FDG is introduced into cells by hypotonic shock, and is cleaved by the enzyme to generate a fluorescent product, which is trapped within the cell. One enzyme can therefore generate a large amount of fluorescent product. Cells expressing GFP constructs will fluoresce without the addition of a substrate. Mutants of GFP are available which have different excitation frequencies, but which emit fluorescence in the same channel. In a two-laser FACS machine, it is possible to distinguish cells which are excited by the different lasers and therefore assay two transfections at the same time.

Alternative means of cell sorting may also be employed. For example, the invention comprises the use of nucleic acid probes complementary to mRNA. Such probes can be used to identify cells expressing polypeptides individually, such that they may subsequently be sorted either manually, or using FACS sorting. Nucleic acid probes complementary to mRNA may be prepared according to the teaching set forth above, using the general procedures as described by Sambrook et al (1989).

In a preferred embodiment, the invention comprises the use of an antisense nucleic acid molecule, complementary to a mRNA, conjugated to a fluorophore which may be used in FACS cell sorting.

Methods have also been described for obtaining information about gene expression and identity using so-called gene chip arrays or high density DNA arrays (Chee M. et al. (1996) Science 274:601-614). These high density arrays are particularly useful for diagnostic and prognostic purposes. Use may also be made of In Vivo Expression Technology (IVET) (Camilli et al. (1994) Proc Natl Acad Sci USA 91:2634-2638). IVET identifies genes upregulated during say treatment or disease when compared to laboratory culture.

The advantage of using a protein assay is that Notch activation can be directly measured. Assay techniques that can be used to determine levels of a polypeptide are well known to those skilled in the art. Such assay methods include radioimmunoassays, competitive-binding assays, Western Blot analysis, antibody sandwich assays, antibody detection, FACS and ELISA assays.

As described above the modulator of Notch signalling may also be an immune cell which has been treated to modulate expression or interaction of Notch, a Notch ligand or the Notch signalling pathway. Such cells may readily be prepared, for example, as described in WO 00/36089 in the name of Lorantis Ltd.

### Conjugates

As noted above, the invention further provides a conjugate comprising first and second sequences, wherein the first sequence comprises an allergen or antigenic determinant thereof or a polynucleotide sequence coding for such an allergen or antigenic determinant thereof and the second sequence comprises a polypeptide or polynucleotide for Notch signalling modulation. The conjugates of the present invention may be protein/polypeptide or polynucleotide conjugates.

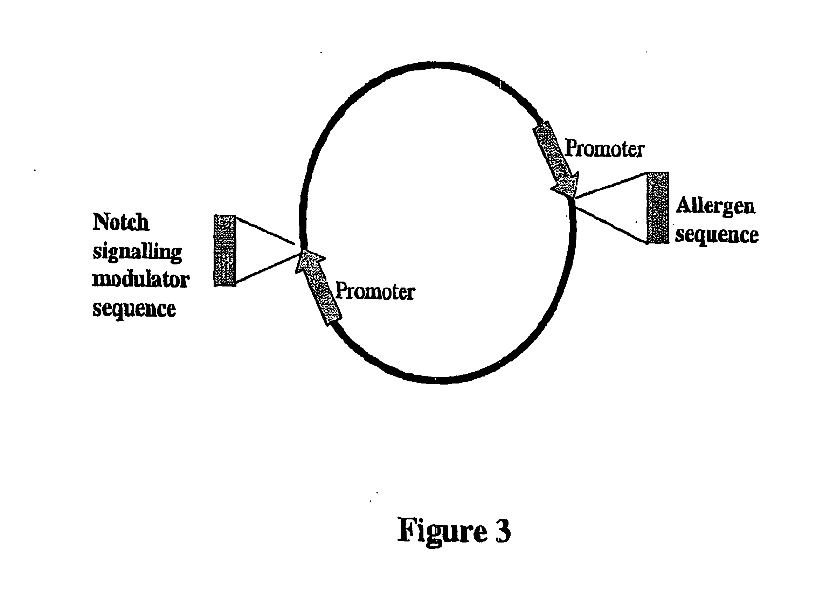

Where the conjugate is a polynucleotide conjugate, it may suitably take the form of a polynucleotide vector such as a plasmid comprising a polynucleotide sequence coding for an allergen or antigenic determinant thereof and a polynucleotide sequence coding for a modulator of the Notch signalling pathway, wherein preferably each sequence is operably linked to regulatory elements necessary for expression in eukaryotic cells. A schematic representation of one such form of vector is shown in Figure 3.

Suitably the polynucleotide sequence coding for the modulator of the Notch signalling pathway may be a nucleotide sequence coding for a Notch ligand such as Deltal, Delta3, Delta4, Jagged1 or Jagged 2, or a biologically active fragment, derivative or homologue of such a sequence. Where intended for human therapy, suitably sequences based on human sequences may be used.

Preferably the polynucleotide sequence coding for the modulator of the Notch signalling pathway may be a nucleotide sequence coding for a Notch ligand DSL domain and at least 1 to 20, suitably at least 2 to 15, suitably at least 2 to 10, for example at least 3 to 8 EGF-like domains. Suitably the DSL and EGF-like domain sequences are or correspond to mammalian sequences. In one embodiment the polynucleotide sequence coding for the modulator of the Notch signalling pathway may further comprise a transmembrane domain (so that the sequence may be expressed on a cell surface, as a membrane protein or polypeptide) and, suitably, a Notch ligand intracellular domain. Preferred sequences include human sequences such as human Delta1, Delta3, Delta4, Jagged1 or Jagged2 sequences.

If desired, the polynucleotide sequence that encodes the allergen or antigenic determinant thereof may further include a nucleotide sequence that encodes a signal sequence which directs trafficking of the allergen or antigenic determinant within a cell to which it is administered. For example, such a signal sequence may direct the allergen or antigenic determinant to be secreted or to be localized to the cytoplasm, the cell membrane, the endoplasmic reticulum, or a lysosome.

Regulatory elements for DNA expression include a promoter and a polyadenylation signal. In addition, other elements, such as a Kozak region, may also be included if desired. Initiation and termination signals are regulatory elements which are often considered part of the coding sequence.

Examples of suitable promoters include but are not limited to promoters from Simian Virus 40 (SV40), Mouse Mammary Tumor Virus (MMTV) promoter, Human Immunodeficiency Virus (HIV) such as the HIV Long Terminal Repeat (LTR) promoter, Moloney virus, ALV, Cytomegalovirus (CMV) such as the CMV immediate early promoter, Epstein Barr Virus (EBV), Rous Sarcoma Virus (RSV) as well as promoters from human genes such as human Actin, human Myosin, human Hemoglobin, human muscle creatine and human metalothionein. Tissue-specific promoters specific for lymphocytes, dendritic cells, skin, brain cells and epithelial cells within the eye are particularly preferred, for example the CD2, CD11c, keratin 14, Wnt-1 and Rhodopsin promoters respectively. Suitably an epithelial cell promoter such as SPC may be used.

Examples of suitable polyadenylation signals include but are not limited to SV40 polyadenylation signals and LTR polyadenylation signals. For example, the SV40 polyadenylation signal used in plasmid pCEP4 (Invitrogen, San Diego Calif.), referred to as the SV40 polyadenylation signal, may be used.

In addition to the regulatory elements required for DNA expression, other elements may also be included in the conjugate. Such additional elements include enhancers which may, for example, be selected from human Actin, human Myosin, human Hemoglobin, human muscle creatine and viral enhancers such as those from CMV, RSV and EBV.

When adminstered to and taken up by a cell, the nucleotide conjugate may for example remain present in the cell as a functioning extrachromosomal molecule and/or integrate into the cell's chromosomal DNA. DNA may be introduced into cells where it remains as separate genetic material in the form of a plasmid or plasmids. Alternatively, linear DNA which can integrate into the chromosome may be introduced into the cell. When introducing DNA into the cell, reagents which promote DNA integration into chromosomes may be added. DNA sequences which are useful to promote integration may also be included in the DNA molecule. Alternatively, RNA may be administered to the cell. It is also possible, for example, to provide the conjugate in the form of a minichromosome including a centromere, telomeres and an origin of replication.

If desired, conjugates may be provided with mammalian origin of replication in order to maintain the construct extrachromosomally and produce multiple copies of the construct in the cell. For example, plasmids pCEP4 and pREP4 from Invitrogen (San Diego, Calif.) contain the Epstein Barr virus origin of replication and nuclear antigen EBNA-1 coding region which produces high copy episomal replication without integration.

In order to maximize protein production, regulatory sequences may be selected which are well suited for gene expression in the type of cells the construct is to be administered to. Moreover, codons may be selected which are most efficiently transcribed in the cell.

Intracellular trafficking signals may also be included as appropriate. Such signals are well known in the art, and include the following:

In some embodiments, the expressed antigen or antigenic determinant may be directed to be secreted by inclusion of an N-terminal hydrophobic sequence. When RNA is translated, the hydrophobic sequence at the N terminal causes the protein to bind to the rough endoplasmic reticulum (RER). The hydrophobic sequence is subsequently clipped off by a protease and the protein is secreted. Thus, if desired, the allergen or antigenic determinant may include an N terminal hydrophobic leader sequence which will direct secretion of the allergen or antigenic determinant when expressed in a cell.

Alternatively, the expressed antigen or antigenic determinant may be directed to be membrane bound by inclusion of an N-terminal hydrophobic sequence and an internal hydrophobic region. As in the secreted forms, when RNA is translated, the hydrophobic sequences causes the protein to bind to the RER. The N terminal hydrophobic sequence is subsequently clipped off by a protease. The protein follows the same secretion pathway but the internal hydrophobic sequence prevents secretion and the protein becomes membrane bound. Thus, if desired, the expressed allergen or antigenic determinant may include an N terminal hydrophobic leader sequence and an internal hydrophobic sequence which will result in the allergen or antigenic determinant, or part thereof, becoming membrane bound when expressed in a cell.

In some alternative embodiments, the expressed antigen or antigenic determinant may be directed to be localized in the cytosol by omitting an N-terminal hydrophobic sequence. When RNA is translated, the protein does not bind to the rough endoplasmic reticulum and the protein becomes cytosolic. Thus, if desired, the expressed antigen or antigenic determinant is free of an N terminal hydrophobic leader sequence so that it becomes cytosolic when expressed in a cell.

In some alternative embodiments, the expressed antigen or antigenic determinant is localized in the lysosome by inclusion of a sequence (such as DKQTLL) which directs localization to lysosomes. Thus, if desired, the allergen or antigenic determinant may include a sequence (such as DKQTLL) so that it is directed to the lysosome when expressed in the cell.

In some embodiments, expressed allergens or antigenic determinants are directed to be localized from the Golgi body back to the ER by including a sequence (such as KDEL) at the C terminal which directs localization to the ER. One example of such an "ER recycling signal" is reported to be the C terminal sequence of the E19 protein from adenovirus. That protein is localized to the ER where it binds to the MHCs and effectively keeps them from loading proteins which are presented by the MHC at the surface where they complex with T cell receptors as part of immune response induction. The E109 protein is a hexapeptide DEKKMP.

Depending upon the type of immune response sought to be modulated, different intracellular localization may be desirable. In the case of Class I immune responses, proteins synthesized within a cell are degraded and transported into the ER where they are loaded onto MHCs which then move to the cell surface and complex with T cell receptors of CD8⁺ T cells. This action encourages CTL responses. In the case of Class II immune responses, proteins are complexed with antigen presenting cells (APCs) which interact with CD4⁺T cells, engaging helper T cells including those associated with antibody responses.

In order to enhance Class I immune responses, localization of proteins to the cytosol or ER allows for such proteins to be more accessible to the Class I pathway.

In order to enhance Class II immune responses, localization of proteins to the transmembrane or lysosomes, or secretion of the protein allows such proteins to be more accessible to the Class II pathway.

Further examples of localization leaders are provided, for example, in Biocca, S. et al. 1990 EMBO J. 9:101-108.

In some embodiments, nucleotide conjugates may code for lysosomal targeting doublets at the C terminal tail of the expressed antigen or antigenic determinant. By including the doublets LL and/or YQ and/or QY the expressed antigen or antigenic determinant is directed to a lysosome.

### Facilitating Agents

In some embodiments, polynucleotides may be delivered in conjunction with administration of a facilitating agent. Facilitating agents which are administered in conjunction with nucleic acid molecules may be administered as a mixture with the nucleic acid molecule or administered separately simultaneously, before or after administration of nucleic acid molecules. Examples of facilitators include benzoic acid esters, anilides, amidines, urethans and the hydrochloride salts thereof such as those of the family of local anesthetics.

Examples of esters include: benzoic acid esters such as piperocaine, meprylcaine and isobucaine; para-aminobenzoic acid esters such as procaine, tetracaine, butethamine, propoxycaine and chloroprocaine; meta-aminobenzoic acid esters including metabuthamine and primacaine; and para-ethoxybenzoic acid esters such as parethoxycaine. Examples of anilides include lidocaine, etidocaine, mepivacaine, bupivacaine, pyrrocaine and prilocaine. Other examples of such compounds include dibucaine, benzocaine, dyclonine, pramoxine, proparacaine, butacaine, benoxinate, carbocaine, methyl bupivacaine, butasin picrate, phenacaine, diothan, luccaine, intracaine, nupercaine, metabutoxycaine, piridocaine, biphenamine and the botanically-derived bicyclics such as cocaine, cinnamoylcocaine, truxilline and cocaethylene and all such compounds complexed with hydrochloride.

The facilitating agent may be administered prior to, simultaneously with or subsequent to the genetic construct. The facilitating agent and the genetic construct may be formulated in the same composition.

Bupivacaine-HCl is chemically designated as 2-piperidinecarboxamide, 1-butyl-N-(2,6-dimethylphenyl)-monohydrochloride, monohydrate and is widely available commercially for pharmaceutical uses from many sources including from Astra Pharmaceutical Products Inc. (Westboro, Mass.) and Sanofi Winthrop Pharmaceuticals (New York, N.Y.), Eastman Kodak (Rochester, N.Y.). Bupivacaine is commercially formulated with and without methylparaben and with or without epinephrine. Any such formulation may be used. It is commercially available for pharmaceutical use in concentration of 0.25%, 0.5% and 0.75% which may be used on the invention. Alternative concentrations, particularly those between 0.05% -1.0% which elicit desirable effects may be prepared if desired. Suitably, for example, about 250µg to about 10 mg of bupivacaine may be administered.

### Antigens and Allergens

An allergen suitable for use in the present invention may be any substance that can be recognised by the immune system, and is generally recognised by an antigen receptor. Preferably the antigen used in the present invention is an immunogen. An allergic response occurs when the host is re-exposed to an antigen that it has encountered previously.

The immune response to antigen is generally either cell mediated (T cell mediated killing) or humoral (antibody production via recognition of whole antigen). The pattern of cytokine production by TH cells involved in an immune response can influence which of these response types predominates: cell mediated immunity (TH1) is characterised by high IL-2 and IFNγ but low IL-4 production, whereas in humoral immunity (TH2) the pattern is low IL-2 and IFNγ but high IL-4, IL-5 and IL-13. Since the secretory pattern is modulated at the level of the secondary lymphoid organ or cells, then pharmacological manipulation of the specific TH cytokine pattern can influence the type and extent of the immune response generated.

The TH1-TH2 balance refers to the relative representation of the two different forms of helper T cells. The two forms have large scale and opposing effects on the immune system. If an immune response favours TH1 cells, then these cells will drive a cellular response, whereas TH2 cells will drive an antibody-dominated response. The type of antibodies responsible for some allergic reactions is induced by TH2 cells.

The antigen or allergen used in the present invention may be a peptide, polypeptide, carbohydrate, protein, glycoprotein, synthetic organic molecule or more complex material containing multiple antigenic epitopes such as a protein complex, cell-membrane preparation, whole cells (viable or non-viable cells), bacterial cells or virus/viral component.

### Treatable Conditions

Preferably the modulation of the immune system is effected by control of immune cell, preferably T-cell, preferably peripheral T-cell, activity.

Suitably the modulation of the immune system comprises reducing an immune response to an allergen or antigenic determinant thereof.

Suitably the modulation of the immune system comprises promoting immune tolerance to an allergen or antigenic determinant thereof.

In one embodiment, the modulation of the immune system comprises reducing the activity of effector T- cells, for example helper (T_{H}) or cytotoxic (T_{C}) T-cells. Preferably, the reduction of activity is with respect to effector T-cells specific for an allergen. Preferably, the activity of effector T-cells specific for an allergen is reduced more than the activity of effector T-cells of other specificities.

Alternatively or in addition, the modulation of the immune system comprises increasing the activity of regulatory (also called suppressor) T- cells, for example Tr1 or Th3 T-cells. Preferably, the increase of activity is with respect to regulatory T-cells specific for an allergen. Preferably, the activity of regulatory T-cells specific for an allergen is increased more than the activity of regulatory T-cells of other specificities.

The present invention may be used for preventing and treating all forms of allergy and allergic disorder, including without limitation: ophthalmic allergic disorders, including allergic conjunctivitis, vernal conjunctivitis, vernal keratoconjunctivitis, and giant papillary conjunctivitis; nasal allergic disorders, including allergic rhinitis and sinusitis; otic allergic disorders, including eustachian tube itching; allergic disorders of the upper and lower airways, including intrinsic and extrinsic asthma; allergic disorders of the skin, including dermatitis, eczema and urticaria; and allergic disorders of the gastrointestinal tract.

### Administration

Suitably the active agents are administered in combination with a pharmaceutically acceptable carrier or diluent The pharmaceutically acceptable carrier or diluent may be, for example, sterile isotonic saline solutions, or other isotonic solutions such as phosphate-buffered saline. The conjugates of the present invention may be admixed with any suitable binder(s), lubricant(s), suspending agent(s), coating agent(s), solubilising agent(s).

It will be appreciated that in one embodiment the therapeutic agents used in the present invention may be administered directly to patients *in vivo.* Alternatively or in addition, the agents may be administered to cells such as T cells and/or APCs in an *ex vivo* manner. For example, leukocytes such as T cells or APCs may be obtained from a patient or donor in known manner, treated/incubated *ex vivo* in the manner of the present invention, and then administered to a patient.

Pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

Alternatively or in addition, active agents may be administered by inhalation, intranasally or in the form of aerosol, or in the form of a suppository or pessary, or they may be applied topically in the form of a lotion, solution, cream, ointment or dusting powder. An alternative means of transdermal administration is by use of a skin patch. For example, they can be incorporated into a cream consisting of an aqueous emulsion of polyethylene glycols or liquid paraffin. They can also be incorporated, at a concentration of between 1 and 10% by weight, into an ointment consisting of a white wax or white soft paraffin base together with such stabilisers and preservatives as may be required.

For some applications, active agents may be administered orally in the form of tablets containing excipients such as starch or lactose, or in capsules or ovules either alone or in admixture with excipients, or in the form of elixirs, solutions or suspensions containing flavouring or colouring agents.

Active agents such as polynucleotides and proteins/polypeptides may also be administered by viral or non-viral techniques. Viral delivery mechanisms include but are not limited to adenoviral vectors, adeno-associated viral (AAV) vectors, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof. The routes for such delivery mechanisms include but are not limited to mucosal, nasal, oral, parenteral, gastrointestinal, topical, or sublingual routes. Active agents may be adminstered by conventional DNA delivery techniques, such as DNA vaccination etc., or injected or otherwise delivered with needleless systems, such as ballistic delivery on particles coated with the DNA for delivery to the epidermis or other sites such as mucosal surfaces.

In general, a therapeutically effective oral or intravenous dose is likely to range from 0.01 to 50 mg/kg body weight of the subject to be treated, preferably 0.1 to 20 mg/kg. The conjugate may also be administered by intravenous infusion, at a dose which is likely to range from 0.001-10 mg/kg/hr.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Tablets or capsules of the conjugates may be administered singly or two or more at a time, as appropriate. It is also possible to administer the conjugates in sustained release formulations.

Active agents may also be injected parenterally, for example intracavemosally, intravenously, intramuscularly or subcutaneously

For parenteral administration, active agents may be used in the form of a sterile aqueous solution which may contain other substances, for example enough salts or monosaccharides to make the solution isotonic with blood.

For buccal or sublingual administration, agents may be administered in the form of tablets or lozenges which can be formulated in a conventional manner.

For oral, parenteral, buccal and sublingual administration to subjects (such as patients), the dosage level of active agents and their pharmaceutically acceptable salts and solvates may typically be from 10 to 500 mg (in single or divided doses). Thus, and by way of example, tablets or capsules may contain from 5 to 100 mg of active agent for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

The routes of administration and dosages described are intended only as a guide since a skilled practitioner will be able to determine readily the optimum route of administration and dosage for any particular patient depending on, for example, the age, weight and condition of the patient.

The term treatment or therapy as used herein should be taken to encompass diagnostic and prophylatic applications.

The treatment of the present invention includes both human and veterinary applications.

Where treated *ex-vivo*, modified cells of the present invention are preferably administered to a host by direct injection into the lymph nodes of the patient. Typically from 10⁴ to 10⁸ treated cells, preferably from 10⁵ to 10⁷ cells, more preferably about 10⁶ cells are administered to the patient. Preferably, the cells will be taken from an enriched cell population.

As used herein, the term "enriched" as applied to the cell populations of the invention refers to a more homogeneous population of cells which have fewer other cells with which they are naturally associated. An enriched population of cells can be achieved by several methods known in the art. For example, an enriched population of T-cells can be obtained using immunoaffinity chromatography using monoclonal antibodies specific for determinants found only on T-cells.

Enriched populations can also be obtained from mixed cell suspensions by positive selection (collecting only the desired cells) or negative selection (removing the undesirable cells). The technology for capturing specific cells on affinity materials is well known in the art (Wigzel, et al., J. Exp. Med., 128:23, 1969; Mage, et al., J. Imnmunol. Meth., 15:47, 1977; Wysocki, et al., Proc. Natl. Acad. Sci. U.S.A., 75:2844, 1978; Schrempf-Decker, et al., J. Immunol Meth., 32:285, 1980; Muller-Sieburg, et al., Cell, 44:653, 1986).

Monoclonal antibodies against antigens specific for mature, differentiated cells have been used in a variety of negative selection strategies to remove undesired cells, for example, to deplete T-cells or malignant cells from allogeneic or autologous marrow grafts, respectively (Gee, et al., J.N.C.I. 80:154, 1988). Purification of human hematopoietic cells by negative selection with monoclonal antibodies and immunomagnetic microspheres can be accomplished using multiple monoclonal antibodies (Griffin, et al., Blood, 63:904, 1984).

Procedures for separation of cells may include magnetic separation, using antibodycoated magnetic beads, affinity chromatography, cytotoxic agents joined to a monoclonal antibody or used in conjunction with a monoclonal antibody, for example, complement and cytotoxins, and "panning" with antibodies attached to a solid matrix, for example, plate, or other convenient technique. Techniques providing accurate separation include fluorescence activated cell sorters, which can have varying degrees of sophistication, for example, a plurality of color channels, low angle and obtuse light scattering detecting channels, impedance channels, etc.

The present invention also provides pharmaceutical kits useful, for example, in the treatment or prevention of allergy, which comprise one or more containers containing a pharmaceutical composition comprising a therapeutically effective amount of a modulator of Notch signalling and one or more containers containing a pharmaceutical composition comprising an allergen or a polynucleotide coding for an allergen or antigenic determinant thereof. Such kits may further include, if desired, one or more of various conventional pharmaceutical kit components, such as, for example, containers with one or more pharmaceutically acceptable carriers, additional containers, etc., as will be readily apparent to those skilled in the art. Instructions, either as inserts or as labels, indicating quantities of the components to be administered, guidelines for administration, and/or guidelines for mixing the components, may also be included if required.

The agents of the present invention can be administered by any suitable means including, but not limited to, for example, oral, rectal, nasal, topical (including transdermal, aerosol, buccal and sublingual), vaginal and parenteral (including subcutaneous, intramuscular, intravenous and intradermal) routes of administration. The modulator of Notch signalling and the allergen or antigenic determinant thereof or polynucleotide coding for the allergen or antigenic determinant thereof may be administered by the same or separate routes. For example, the modulator of Notch signalling may be administered systemically whilst the allergen or antigenic determinant thereof or polynucleotide coding for the allergen or antigenic determinant thereof may be administered locally, or both agents may be administered systemically or both agents may be administered locally.

Alternatively or in addition, one, both or more agents may be administered directly to an organ or tissue which is subject to allergic reaction.

It will be appreciated that it may be appropriate to administer more than one dose of either the modulator of Notch signalling and/or the allergen or antigenic determinant thereof or polynucleotide coding for the allergen or antigenic determinant thereof.

By "simultaneously" is meant that the modulator of the Notch signalling pathway and the allergen or antigenic determinant thereof or polynucleotide coding for the allergen or antigenic determinant thereof are administered at substantially the same time, and suitably together in the same formulation.

By "contemporaneously" it is meant that the modulator of the Notch signalling pathway and the allergen or antigenic determinant thereof or the polynucleotide coding for the allergen or antigenic determinant thereof, or biologically active derivative, homologue or variant thereof are administered closely in time, e.g., the allergen or antigenic determinant, coding polynucleotide or biologically active derivative, homologue or variant thereof is administered within from about one minute to within about one day before or after the modulator of the Notch signalling pathway is administered. Any contemporaneous time is useful. However, it will often be the case that when not administered simultaneously, the modulator of the Notch signalling pathway and the allergen or antigenic determinant, coding polynucleotide or biologically active derivative, homologue or variant thereof will be administered within about one minute to within about eight hours, and preferably within less than about one to about four hours. When administered contemporaneously, the modulator of the Notch signalling pathway and the allergen or antigenic determinant, coding polynucleotide or biologically active derivative, homologue or variant thereof are preferably administered at the same site on the patient/subject. The term "same site" includes the exact location, but can be within about 0.5 to about 15 centimetres, preferably from within about 0.5 to about 5 centimetres.

The term "separately" as used herein means that the modulator of the Notch signalling pathway and the allergen or antigenic determinant, coding polynucleotide or biologically active derivative, homologue or variant thereof are administered at an interval, for example at an interval of about a day to several weeks or months. The active agents may be administered in either order.

Likewise, the modulator of the Notch signalling pathway may be administered more frequently than the allergen or antigenic determinant, coding polynucleotide or biologically active derivative, homologue or variant thereof or *vice versa.*

The term "sequentially" as used herein means that the modulator of the Notch signalling pathway and the allergen or antigenic determinant, coding polynucleotide or biologically active derivative, homologue or variant thereof are administered in sequence, for example at an interval or intervals of minutes, hours, days or weeks. If appropriate the active agents may be administered in a regular repeating cycle.

### Coated Particle Preparations

As disclosed for example in US Patent Publication No 20020165176 (Powderject), particle-mediated methods for delivering nucleic acid preparations are known in the art. Thus, once prepared and suitably purified, nucleic acid molecules can be coated onto carrier particles (e.g., core carriers) using a variety of techniques known in the art. Carrier particles are selected from materials which have a suitable density in the range of particle sizes typically used for intracellular delivery from a particle-mediated delivery device. The optimum carrier particle size will, of course, depend on the diameter of the target cells. Alternatively, colloidal gold particles can be used wherein the coated colloidal gold is administered (e.g., injected) into tissue (e.g., skin or muscle) and subsequently taken-up by immune-competent cells.

For example, tungsten, gold, platinum and iridium carrier particles can be used. Tungsten and gold particles are preferred. Tungsten particles are readily available in average sizes of for example from 0.5 to 2.0 micrometres in diameter. Although such particles have optimal density for use in particle acceleration delivery methods, and allow highly efficient coating with DNA, tungsten may potentially be toxic to certain cell types. Gold particles or microcrystalline gold (e.g., gold powder A1570, available from Engelhard Corp., East Newark, N.J.) may also be used. Gold particles provide uniformity in size (available from Alpha Chemicals in particle sizes of 1-3 micrometres, or available from Degussa, South Plainfield, N.J. in a range of particle sizes including 0.95 micrometres) and reduced toxicity. Microcrystalline gold provides a diverse particle size distribution, typically in the range of 0.1-5 micrometres. However, the irregular surface area of microcrystalline gold provides for highly efficient coating with nucleic acids.

A number of methods are known and have been described for coating or precipitating DNA or RNA onto gold or tungsten particles. Typically such methods generally combine a predetermined amount of gold or tungsten with plasmid DNA, CaCl₂ and spermidine. The resulting solution is preferably vortexed continually during the coating procedure to ensure uniformity of the reaction mixture. After precipitation of the nucleic acid, the coated particles can be transferred to suitable membranes and allowed to dry prior to use, coated onto surfaces of a sample module or cassette, or loaded into a delivery cassette for use in particular particle-mediated delivery instruments.

Following their formation, carrier particles coated with the nucleic acid preparations can be delivered to a subject using particle-mediated delivery techniques.

Various particle acceleration devices suitable for particle-mediated delivery are known in the art. Current device designs typically employ an explosive, electric or gaseous discharge to propel coated carrier particles toward target cells. The coated carrier particles can themselves be releasably attached to a movable carrier sheet, or removably attached to a surface along which a gas stream passes, lifting the particles from the surface and accelerating them toward the target. An example of a gaseous discharge device is described in U.S. Pat. No. 5,204,253. An explosive-type device is described in U.S. Pat. No. 4,945,050. One example of an electric discharge-type particle acceleration apparatus is described in U.S. Pat. No. 5,120,657. Another electric discharge apparatus suitable for use herein is described in U.S. Pat. No. 5,149,655.

If desired, these particle acceleration devices can be provided in a preloaded condition containing a suitable dosage of the coated carrier particles. The loaded syringe can for example be packaged in a sealed container.

The coated particles are administered to the subject to be treated in a manner compatible with the dosage formulation, and in an amount that will be effective to bring about a desired effect. The amount of the composition to be delivered which, in the case of nucleic acid molecules is generally in the range of from 0.001 to 1000 micrograms, more preferably 0.01 to 10.0 micrograms of nucleic acid molecule per dose, depends on the subject to be treated. The exact amount necessary will vary depending on the age and general condition of the individual being immunized and the particular nucleotide sequence selected, as well as other factors. An appropriate effective amount can be readily determined by one of skill in the art upon reading the instant specification.

The formulated compositions may be prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be densified using the techniques described in commonly owned International Publication No. WO 97/48485.

These methods can be used to obtain nucleic acid particles having a size ranging for example from about 0.01 to about 250 micrometres, preferably about 10 to about 150 micrometres, and most preferably about 20 to about 60 micrometres; and a particle density ranging for example from about 0.1 to about 25 g/cm3, and a bulk density of about 0.5 to about 3.0 g/cm3, or greater.

Similarly, particles having a size ranging for example from about 0.1 to about 250 micrometres, preferably about 0.1 to about 150 micrometres, and most preferably about 20 to about 60 micrometres; a particle density ranging for example from about 0.1 to about 25 g/cm3, and a bulk density of preferably about 0.5 to about 3.0 g/cm3, and most preferably about 0.8 to about 1.5 g/cm3 can be obtained.

Single unit dosages or multidose containers, in which the particles may be packaged prior to use, can comprise a hermetically sealed container enclosing a suitable amount of the particles. The particulate compositions can be packaged as a sterile formulation, and the hermetically sealed container can thus be designed to preserve sterility of the formulation until use in the methods of the invention. If desired, the containers can be adapted for direct use in a needleless syringe system. Such containers can take the form of capsules, foil pouches, sachets, cassettes, and the like.

Following their formation, the particulate composition (e.g., powder) can be delivered transdermally to the subject's tissue using a suitable transdermal delivery technique. Various particle acceleration devices suitable for transdermal delivery of the substance of interest are known in the art. A particularly preferred transdermal delivery system employs a needleless syringe to fire solid drug-containing particles in controlled doses into and through intact skin and tissue. See, e.g., U.S. Pat. No. 5,630,796 to Bellhouse et al. which describes a needleless syringe (also known as "the PowderJect^{™} needleless syringe device"). Other needleless syringe configurations are well known in the art.

The particulate compositions can be administered using a transdermal delivery technique. Preferably, the particulate compositions will be delivered via a powder injection method, e.g., delivered from a needleless syringe system such as those described in International Patent Publication Nos. WO 94/24263, WO 96/04947, WO 96/12513, and WO 96/20022,. Delivery of particles from such needleless syringe systems is typically practised with particles having an approximate size generally ranging for example from 0.1 to 250 micrometres, preferably ranging from about 10-70 micrometres. Particles larger than about 250 micrometres can also be delivered from the devices, with the upper limitation being the point at which the size of the particles would cause untoward damage to the skin cells. The actual distance which the delivered particles will penetrate a target surface depends upon particle size (e.g., the nominal particle diameter assuming a roughly spherical particle geometry), particle density, the initial velocity at which the particle impacts the surface, and the density and kinematic viscosity of the targeted skin tissue. In this regard, optimal particle densities for use in needleless injection generally range between about 0.1 and 25 g/cm3, preferably between about 0.9 and 1.5 g/cm3, and injection velocities generally range between about 100 and 3,000 m/sec, or greater. With appropriate gas pressure, particles having an average diameter of for example 10-70 micrometres can be accelerated through the nozzle at velocities approaching the supersonic speeds of a driving gas flow.

If desired, these needleless syringe systems can be provided in a preloaded condition containing a suitable dosage of the particles comprising the antigen of interest and/or the selected adjuvant. The loaded syringe may suitably be packaged in a hermetically sealed container.

Compositions containing a therapeutically effective amount of the powdered molecules described herein can be delivered to any suitable target tissue, suitably via the above-described needleless syringes. For example, the compositions may suitably be delivered to muscle, skin, brain, lung, liver, spleen, bone marrow, thymus, heart, lymph, blood, bone cartilage, pancreas, kidney, gall bladder, stomach, intestine, testis, ovary, uterus, rectum, nervous system, eye, gland and connective tissues. For nucleic acid molecules, delivery is preferably to, and the molecules expressed in, terminally differentiated cells; however, the molecules can also be delivered to non-differentiated, or partially differentiated cells such as stem cells of blood and skin fibroblasts.

The powdered compositions are administered to the subject to be treated in a manner compatible with the dosage formulation, and in an amount that will be prophylactically and/or therapeutically effective. The amount of the composition to be delivered, generally in the range of from 0.5 micrograms/kg to 100 micrograms/kg of nucleic acid molecule per dose, depends on the subject to be treated. The exact amount necessary will vary depending on the age and general condition of the individual to be treated, the severity of the condition being treated, the particular preparation delivered, the site of administration, as well as other factors. An appropriate effective amount can be readily determined by one of skill in the art.

### Antigen Presenting Cells

Where required, antigen-presenting cells (APCs) may be "professional" antigen presenting cells or may be another cell that may be induced to present antigen to T cells. Alternatively a APC precursor may be used which differentiates or is activated under the conditions of culture to produce an APC. An APC for use in the *ex vivo* methods of the invention is typically isolated from a tumour or peripheral blood found within the body of a patient. Preferably the APC or precursor is of human origin. However, where APCs are used in preliminary *in vitro* screening procedures to identify and test suitable nucleic acid sequences, APCs from any suitable source, such as a healthy patient, may be used.

APCs include dendritic cells (DCs) such as interdigitating DCs or follicular DCs, Langerhans cells, PBMCs, macrophages, B-lymphocytes, or other cell types such as epithelial cells, fibroblasts or endothelial cells, activated or engineered by transfection to express a MHC molecule (Class I or II) on their surfaces. Precursors of APCs include CD34⁺ cells, monocytes, fibroblasts and endothelial cells. The APCs or precursors may be modified by the culture conditions or may be genetically modified, for instance by transfection of one or more genes encoding proteins which play a role in antigen presentation and/or in combination of selected cytokine genes which would promote to immune potentiation (for example IL-2, IL-12, IFN-γ, TNF-α, IL-18 etc.). Such proteins include MHC molecules (Class I or Class II), CD80, CD86, or CD40. Most preferably DCs or DC-precursors are included as a source of APCs.

Dendritic cells (DCs) can be isolated/prepared by a number of means, for example they can either be purified directly from peripheral blood, or generated from CD34⁺ precursor cells for example after mobilisation into peripheral blood by treatment with GM-CSF, or directly from bone morrow. From peripheral blood, adherent precursors can be treated with a GM-CSF/IL-4 mixture (Inaba K, et al. (1992) J. Exp. Med. 175: 1157-1167 (Inaba)), or from bone marrow, non-adherent CD34⁺ cells can be treated with GM-CSF and TNF-a (Caux C, et al. (1992) Nature 360: 258-261 (Caux)). DCs can also be routinely prepared from the peripheral blood of human volunteers, similarly to the method of Sallusto and Lanzavecchia (Sallusto F and Lanzavecchia A (1994) J. Exp. Med. 179: 1109-1118) using purified peripheral blood mononucleocytes (PBMCs) and treating 2 hour adherent cells with GM-CSF and IL-4. If required, these may be depleted of CD19⁺ B cells and CD3⁺, CD2⁺ T cells using magnetic beads (Coffin RS, et al. (1998) Gene Therapy 5: 718-722 (Coffin)). Culture conditions may include other cytokines such as GM-CSF or IL-4 for the maintenance and, or activity of the dendritic cells or other antigen presenting cells.

Thus, it will be understood that the term "antigen presenting cell or the like" are used herein is not intended to be limited to APCs. The skilled man will understand that any vehicle capable of presenting to the T cell population may be used, for the sake of convenience the term APCs is used to refer to all these. As indicated above, preferred examples of suitable APCs include dendritic cells, L cells, hybridomas, fibroblasts, lymphomas, macrophages, B cells or synthetic APCs such as lipid membranes.

### T cells

Where required, T cells from any suitable source, such as a healthy patient, may be used and may be obtained from blood or another source (such as lymph nodes, spleen, or bone marrow). They may optionally be enriched or purified by standard procedures. The T cells may be used in combination with other immune cells, obtained from the same or a different individual. Alternatively whole blood may be used or leukocyte enriched blood or purified white blood cells as a source of T cells and other cell types. It is particularly preferred to use helper T cells (CD4⁺). Alternatively other T cells such as CD8⁺ cells may be used. It may also be convenient to use cell lines such as T cell hybridomas.

### Introduction of nucleic acid sequences into APCs and T-cells

T-cells and APCs as described above are cultured in a suitable culture medium such as DMEM or other defined media, optionally in the presence of fetal calf serum.

Polypeptide substances may be administered to T-cells and/or APCs by introducing nucleic acid constructs/viral vectors encoding the polypeptide into cells under conditions that allow for expression of the polypeptide in the T-cell and/or APC. Similarly, nucleic acid constructs encoding antisense constructs may be introduced into the T-cells and/or APCs by transfection, viral infection or viral transduction.

In a preferred embodiment, nucleotide sequences will be operably linked to control sequences, including promoters/enhancers and other expression regulation signals. The term "operably linked" means that the components described are in a relationship permitting them to function in their intended manner. A regulatory sequence "operably linked" to a coding sequence is peferably ligated in such a way that expression of the coding sequence is achieved under condition compatible with the control sequences.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of a-actin, b-actin, tubulin) or, alternatively, a tissue-specific manner (such as promoters of the genes for pyruvate kinase). Tissue-specific promoters specific for lymphocytes, dendritic cells, skin, brain cells and epithelial cells within the eye are particularly preferred, for example the CD2, CD11c, keratin 14, Wnt-1 and Rhodopsin promoters respectively. Preferably the epithelial cell promoter SPC is used. They may also be promoters that respond to specific stimuli, for example promoters that bind steroid hormone receptors. Viral promoters may also be used, for example the Moloney murine leukaemia virus long terminal repeat (MMLV LTR) promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated.

Any of the above promoters may be modified by the addition of further regulatory sequences, for example enhancer sequences. Chimeric promoters may also be used comprising sequence elements from two or more different promoters.

Alternatively (or in addition), the regulatory sequences may be cell specific such that the gene of interest is only expressed in cells of use in the present invention. Such cells include, for example, APCs and T-cells.

The cells may be prepared for administration to a patient or incubated with T-cells *in vitro* (*ex vivo*)*.*

### Tolerisation assays

Any of the assays described above (see "Assays") can be adapted to monitor or to detect reduced reactivity and promotion of tolerance in immune cells for use in clinical applications. Such assays will involve, for example, detecting increased Notch-ligand expression or activity in host cells or monitoring Notch cleavage in donor cells. Further methods of monitoring immune cell activity are set out below.

Immune cell activity may be monitored by any suitable method known to those skilled in the art. For example, cytotoxic activity may be monitored. Natural killer (NK) cells will demonstrate enhanced cytotoxic activity after activation. Therefore any drop in or stabilisation of cytotoxicity will be an indication of reduced reactivity.

Once activated, leukocytes express a variety of new cell surface antigens. NK cells, for example, will express transferrin receptor, HLA-DR and the CD25 IL-2 receptor after activation. Reduced reactivity may therefore be assayed by monitoring expression of these antigens.

Hara et al. Human T-cell Activation: III, Rapid Induction of a Phosphorylated 28 kD/32kD Disulfide linked Early Activation Antigen (EA-1) by 12-0-tetradecanoyl Phorbol-13-Acetate, Mitogens and Antigens, J. Exp. Med., 164:1988 (1986), and Cosulich et al. Functional Characterization of an Antigen (MLR3) Involved in an Early Step of T-Cell Activation, PNAS, 84:4205 (1987), have described cell surface antigens that are expressed on T-cells shortly after activation. These antigens, EA-1 and MLR3 respectively, are glycoproteins having major components of 28kD and 32kD. EA-1 and MLR3 are not HLA class II antigens and an MLR3 Mab will block IL-1 binding. These antigens appear on activated T-cells within 18 hours and can therefore be used to monitor immune cell reactivity.

Additionally, leukocyte reactivity may be monitored as described in EP 0325489.

Briefly this is accomplished using a monoclonal antibody ("Anti-Leu23") which interacts with a cellular antigen recognised by the monoclonal antibody produced by the hybridoma designated as ATCC No. HB-9627.

Anti-Leu 23 recognises a cell surface antigen on activated and antigen stimulated leukocytes. On activated NK cells, the antigen, Leu 23, is expressed within 4 hours after activation and continues to be expressed as late as 72 hours after activation. Leu 23 is a disulfide-linked homodimer composed of 24 kD subunits with at least two N-linked carbohydrates.

Because the appearance of Leu 23 on NK cells correlates with the development of cytotoxicity and because the appearance of Leu 23 on certain T-cells correlates with stimulation of the T-cell antigen receptor complex, Anti-Leu 23 is useful in monitoring the reactivity of leukocytes.

Further details of techniques for the monitoring of immune cell reactivity may be found in: 'The Natural Killer Cell' Lewis C. E. and J. O'D. McGee 1992. Oxford University Press; Trinchieri G. 'Biology of Natural Killer Cells' Adv. Immunol. 1989 vol 47 pp187-376; 'Cytokines of the Immune Response' Chapter 7 in "Handbook of Immune Response Genes". Mak T.W. and J.J.L. Simard 1998.

### Preparation of Primed APCs and Lymphocytes

According to one aspect of the invention immune cells may be used to present antigens or allergens or antigenic determinants thereof and/or may be treated to modulate expression or interaction of Notch, a Notch ligand or the Notch signalling pathway. Thus, for example, Antigen Presenting Cells (APCs) may be cultured in a suitable culture medium such as DMEM or other defined media, optionally in the presence of a serum such as fetal calf serum. Optimum cytokine concentrations may be determined by nitration. One or more substances capable of up-regulating or down-regulating the Notch signalling pathway are then typically added to the culture medium together with the antigen or antigenic determinant of interest. The antigen or antigenic determinant may be added before, after or at substantially the same time as the substance(s). Cells are typically incubated with the substance(s) and antigen for at least one hour, preferably at least 3 hours, at 37°C. If required, a small aliquot of cells may be tested for modulated target gene expression as described above. Alternatively, cell activity may be measured by the inhibition of T cell activation by monitoring surface markers, cytokine secretion or proliferation as described in WO98/20142.

As discussed above, polypeptide substances may be administered to APCs by introducing nucleic acid constructs/viral vectors encoding the polypeptide into cells under conditions that allow for expression of the polypeptides in the APC. Similarly, nucleic acid constructs encoding antigens may be introduced into the APCs by transfection, viral infection or viral transduction. The resulting APCs that show increased levels of a Notch signalling are now ready for use.

### Preparation of Regulatory T cells (and B cells) ex vivo

The techniques described below are described in relation to T cells, but are equally applicable to B cells. The techniques employed are essentially identical to that described for APCs alone except that T cells are generally co-cultured with the APCs. However, it may be preferred to prepare primed APCs first and then incubate them with T cells. For example, once the primed APCs have been prepared, they may be pelleted and washed with PBS before being resuspended in fresh culture medium. Once primed APCs have been prepared, it is not always necessary to administer any substances to the T cell since the primed APC is itself capable of inducing immunotolerance leading to increased Notch or Notch ligand expression in the T cell, presumably via Notch/Notch ligand interactions between the primed APC and T cell.

Incubations will typically be for at least 1 hour, preferably at least 3, 6 , 12, 24, 36 or more hours, in suitable culture medium at 37°C. Induction of immunotolerance may be determined, for example, by subsequently challenging T cells with antigen and measuring IL-2 production compared with control cells not exposed to APCs.

Primed T cells or B cells may also be used to induce immunotolerance in other T cells or B cells in the absence of APCs using similar culture techniques and incubation times.

Various preferred features and embodiments of the present invention will now be described in more detail by way of non-limiting examples.

### Example 1

### hDelta1-IgG4Fc Fusion Protein

A fusion protein comprising the extracellular domain of human Deltal fused to the Fc domain of human IgG4 ("hDeltal-IgG4Fc") was prepared by inserting a nucleotide sequence coding for the extracellular domain of human Deltal (see, eg Genbank Accession No AF003522) into the expression vector pCONγ (Lonza Biologics, Slough, UK) and expressing the resulting construct in CHO cells. The amino acid sequence of the resulting expressed fusion protein was as follows:

MGSRCALALAVLSALLCQVWSSGVFELKLQEFVNKKGLLGNRNCCRGGAGPPP
CACRTFFRVCLKHYQASVSPEPPCTYGSAVTPVLGVDSFSLPDGGGADSAFSNPI
RFPFGFTWPGTFSLIIEALHTDSPDDLATENPERLISRLATQRHLTVGEEWSQDLH
SSGRTDLKYSYRFVCDEHYYGEGCSVFCRPRDDAFGHFTCGERGEKVCNPGWK
GPYCTEPICLPGCDEQHGFCDKPGECKCRVGWQGRYCDECIRYPGCLHGTCQQP
WQCNCQEGWGGLFCNQDLNYCTHHKPCKNGATCTNTGQGSYTCSCRPGYTGA
TCELGIDECDPSPCKNGGSCTDLENSYSCTCPPGFYGKICELSAMTCADGPCFNG
GRCSDSPDGGYSCRCPVGYSGFNCEKKIDYCSSSPCSNGAKCVDLGDAYLCRCQ
AGFSGRHCDDNVDDCASSPCANGGTCRDGVNDFSCTCPPGYTGRNCSAPVSRCE
HAPCHNGATCHERGHGYVCECARGYGGPNCQFLLPELPPGPAVVDLTEKLEAST
KGPSVFPLAPCSRSTSESTAALGCLVKDYFPEPVTVSWNSGALTSGVHTFPAVLQ
SSGLYSLSSVVTVPSSSLGTKTYTCNVDHKPSNTKVDKRVESKYGPPCPSCPAPEF
LGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEKTISKAKGQ

PREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLS LGK

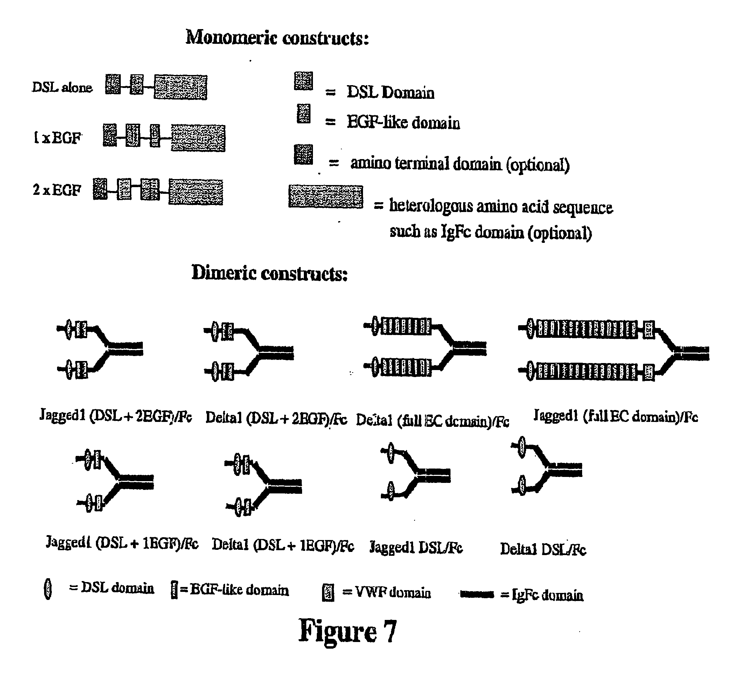

Wherein the first underlined sequence is the signal peptide (cleaved from the mature protein) and the second underlined sequence is the IgG4 Fc sequence. The protein normally exists as a dimer linked by disulphide bonds (see eg schematic representation in Figure 7).

### Example 2

### Dynabeads ELISA Assay Method For Detecting Notch Signalling Activity

### (i) CD4+ cell purification

Spleens were removed from mice (variously Balb/c females, 8-10 weeks, C57B/6 females, 8-10 weeks, D011.10 transgenic females, 8-10 weeks) and passed through a 0.2µM cell strainer into 20ml R10F medium (R10F-RPMI 1640 media (Gibco Cat No 22409) plus 2mM L-glutamine, 50µg/ml Penicillin, 50µg/ml Streptomycin, 5 x 10⁻⁵ M β-mercapto-ethanol in 10% fetal calf serum). The cell suspension was spun (1150rpm 5min) and the media removed.

The cells were incubated for 4 minutes with 5ml ACK lysis buffer (0.15M NH₄Cl, 1.0M KHCO₃, 0.1mM Na₂EDTA in double distilled water) per spleen (to lyse red blood cells). The cells were then washed once with R10F medium and counted. CD4+- cells were purified from the suspensions by positive selection on a Magnetic Associated Cell Sorter (MACS) column (Miltenyi Biotec, Bisley, UK: Cat No.130-042-401) using CD4 (L3T4) beads (Miltenyi Biotec Cat No 130-049-201), according to the manufacturer's directions.

### (ii) Antibody Coating

96 well flat-bottomed plates were coated with DPBS plus 1µg/ml anti-hamsterIgG antibody (Pharmingen Cat No 554007) plus 1µg/ml anti-IgG4 antibody. 100µl of coating mixture was added per well. Plates were incubated overnight at 4°C then washed with DPBS. Each well then received either 100µl DPBS plus anti-CD3 antibody (1µg/ml) or, 100µl DPBS plus anti-CD3 antibody (1µg/ml) plus hDelta1-IgG4Fc fusion protein (100µg/ml; as described above). The plates were incubated for 2-3 hours at 37°C then washed again with DPBS before cells (prepared as described above) were added.

### (iii) Primary Polyclonal Stimulation and ELISA

CD4+ cells were cultured in 96 well, flat-bottomed plates pre-coated according to (ii) above. Cells were re-suspended, following counting, at 2 x 10⁶ /ml in R10F medium plus 4µg/ml anti-CD28 antibody (Pharmingen, Cat No 553294, Clone No 37.51). 100µl cell suspension was added per well. 100µl of R10F medium was then added to each well to give a final volume of 200µl (2 x 10⁵ cells/well, anti-CD28 final concentration 2µg/ml). The plates were then incubated at 37°C for 72 hours.

125µl supernatant was then removed from each well and stored at -20°C until tested by ELISA for IL-2, IL-10, IFNg and IL-13 using antibody pairs from R & D Systems (Abingdon, UK).

### Example 3

### Constructs for use in Notch signalling activity assay

### A) Construction of Luciferase Reporter Plasmid 10xCBF1-Luc (pLOR91)

An adenovirus major late promoter TATA-box motif with BglII and HindIII cohesive ends was generated as follows:

BglII HindIII
GATCTGGGGGCTATAAAAGGGGGTA
ACCCCCGATATTTTCCCCCATTCGA

This was cloned into plasmid pGL3-Basic (Promega) between the BgiII and HindIII sites to generate plasmid pGL3-AdTATA.

A TP1 promoter sequence (TP1; equivalent to 2 CBF1 repeats) with BamHl and BglII cohesive ends was generated as follows:

BamH1                                                    BglII
5' GATCCCGACTCGTGGGAAAATGGGCGGAAGGGCACCGTGGGAAAATAGTA 3'
   3' GGCTGAGCACCCTTTTACCCGCCTTCCCGTGGCACCCTTTTATCATCTAG 5'

This sequence was pentamerised by repeated insertion into a BglII site and the resulting TP1 pentamer (equivalent to 10 CBF1 repeats) was inserted into pGL3-AdTATA at the BglII site to generate plasmid pLOR91.

### B) Generation of a stable CHO cell reporter cell line expressing full length Notch2 and the 10xCBF1-Luc reporter cassette

A cDNA clone spanning the complete coding sequence of the human Notch2 gene (see, eg GenBank Accession No AF315356) was constructed as follows. A 3' cDNA fragment encoding the entire intracellular domain and a portion of the extracellular domain was isolated from a human placental cDNA library (OriGene Technologies Ltd., USA) using a PCR-based screening strategy. The remaining 5' coding sequence was isolated using a RACE (Rapid Amplification of cDNA Ends) strategy and ligated onto the existing 3' fragment using a unique restriction site common to both fragments (Cla I). The resulting full-length cDNA was then cloned into the mammalian expression vector pcDNA3.1-V5-HisA (Invitrogen) without a stop codon to generate plasmid pLOR92. When expressed in mammalian cells, pLOR92 thus expresses the full-length human Notch2 protein with V5 and His tags at the 3' end of the intracellular domain.

Wild-type CHO-K1 cells (eg see ATCC No CCL 61) were transfected with pLOR92 (pcDNA3.1-FLNotch2-V5-His) using Lipfectamine 2000^{™} (Invitrogen) to generate a stable CHO cell clone expressing full length human Notch2 (N2). Transfectant clones were selected in Dulbecco's Modified Eagle Medium (DMEM) plus 10% heat inactivated fetal calf serum ((HI)FCS) plus glutamine plus Penicillin-Streptomycin (P/S) plus 1 mg/ml G418 (Geneticin^{™} - Invitrogen) in 96-well plates using limiting dilution. Individual colonies were expanded in DMEM plus 10%(HI)FCS plus glutamine plus P/S plus 0.5 mg/ml G418. Clones were tested for expression of N2 by Western blots of cell lysates using an anti-V5 monoclonal antibody (Invitrogen). Positive clones were then tested by transient transfection with the reporter vector pLOR91 (10xCBF1-Luc) and co-culture with a stable CHO cell clone (CHO-Delta) expressing full length human delta-like ligand 1 (DLL1; eg see GenBank Accession No AF196571). (CHO-Delta was prepared in the same way as the CHO Notch 2 clone, but with human DLL1 used in place of Notch 2. A strongly positive clone was selected by Western blots of cell lysates with anti-V5 mAb.)

One CHO-N2 stable clone, N27, was found to give high levels of induction when transiently transfected with pLOR91 (10xCBF1-Luc) and co-cultured with the stable CHO cell clone expressing full length human DLL1 (CHO-Deltal). A hygromycin gene cassette (obtainable from pcDNA3.1/hygro, Invitrogen) was inserted into pLOR91 (10xCBF1-Luc) using BamH1 and Sal1 and this vector (10xCBF1-Luc-hygro) was transfected into the CHO-N2 stable clone (N27) using Lipfectamine 2000 (Invitrogen). Transfectant clones were selected in DMEM plus 10%(HI)FCS plus glutamine plus P/S plus 0.4 mg/ml hygromycin B (Invitrogen) plus 0.5 mg/ml G418 (Invitrogen) in 96-well plates using limiting dilution. Individual colonies were expanded in DMEM plus 10%(HI)FCS plus glutamine plus P/S + 0.2 mg/ml hygromycin B plus 0.5 mg/ml G418 (Invitrogen).

Clones were tested by co-culture with a stable CHO cell clone expressing FL human DLL1. Three stable reporter cell lines were produced N27#11, N27#17 and N27#36. N27#11 was selected for further use because of its low background signal in the absence of Notch signalling, and hence high fold induction when signalling is initiated. Assays were set up in 96-well plates with 2 x 10⁴ N27#11 cells per well in 100 µl per well of DMEM plus 10%(HI)FCS plus glutamine plus P/S.

### Example 4

### Luciferase assay for detecting Notch signalling activity

hDelta1-IgG4Fc fusion protein (Example 1) was immobilised on Streptavidin-Dynabeads (CELLection Biotin Binder Dynabeads [Cat. No. 115.21] at 4.0 x 10⁸ beads/ml from Dynal (UK) Ltd; "beads") in combination with biotinylated α-IgG-4 (clone JDC14 at 0.5 mg/ml from Pharmingen [Cat. No. 555879]) as follows:
1 x 10⁷ beads (25 µl of beads at 4.0 x 10⁸ beads/ml) and 2 µg biotinylated α-IgG-4 was used for each sample assayed. PBS was added to the beads to 1 ml and the mixture was spun down at 13,000 rpm for 1 minute. Following washing with a further 1 ml of PBS the mixture was spun down again. The beads were then resuspended in a final volume of 100 µl of PBS containing the biotinylated α-IgG-4 in a sterile Eppendorf tube and placed on a shaker at room temperature for 30 minutes. PBS to was added to 1 ml and the mixture was spun down at 13,000 rpm for 1 minute and then washed twice more with 1 ml of PBS.

The mixture was then spun down at 13,000 rpm for 1 minute and the beads were resupsended in 50 µl PBS per sample. 50 µl of biotinylated α-IgG-4 -coated beads were added to each sample and the mixture was incubated on a rotary shaker at 4 °C overnight. The tube was then spun at 1000 rpm for 5 minutes at room temperature.
The beads then were washed with 10 ml of PBS, spun down, resupended in 1 ml of PBS, transferred to a sterile Eppendorf tube, washed with a further 2 x 1 ml of PBS, spun down and resuspended in a final volume of 100 µl of DMEM plus 10%(HI)FCS plus glutamine plus P/S, i.e. at 1.0 x 10⁵ beads/µl.

Stable N27#11 cells (T₈₀ flask)were removed using 0.02% EDTA solution (Sigma), spun down and resuspended in 10 ml DMEM plus 10%(HI)FCS plus glutamine plus P/S. 10 µl of cells were counted and the cell density was adjusted to 1.0 x 10⁵ cells/ml with fresh DMEM plus 10%(HI)FCS plus glutamine plus P/S. 1.0 x 10⁵ of the cells were plated out per well of a 24-well plate in a 1 ml volume of DMEM plus 10%(HI)FCS plus glutamine plus P/S and cells were placed in an incubator to settle down for at least 30 minutes.

20 µl of beads were then added in duplicate to a pair of wells to give 2.0 x 10⁶ beads /well (100 beads / cell). The plate was left in a CO₂ incubator overnight.
Supernatant was then removed from all the wells, 100 µl of SteadyGlo^{™} luciferase assay reagent (Promega) was added and the resulting mixture left at room temperature for 5 minutes.

The mixture was then pipetted up and down 2 times to ensure cell lysis and the contents from each well were transferred to a 96 well plate (with V-shaped wells) and spun in a plate holder for 5 minutes at 1000 rpm at room temperature.

175 µl of cleared supernatant was then transferred to a white 96-well plate (Nunc) leaving the beads pellet behind.

Luminescence was then read in a TopCount^{™} (Packard) counter.

### Example 5

### Reporter Assay using Jurkat cell line

As Jurkat cells cannot be cloned by simple limiting dilution a methylcellulose-containing medium (ClonaCell^{™} TCS) was used with these cells.

Jurkat E6.1 cells (lymphoblast cell line; ATCC No TIB-152) were cloned using ClonaCell^{™} Transfected Cell Selection (TCS) medium (StemCell Technologies, Vancouver, Canada and Meylan, France) according to the manufacturer's guidelines. Plasmid pLOR92 (prepared as described above) was electroporated into the Jurkat E6.1 cells with a Biorad Gene Pulser II electroporator as follows:
Actively dividing cells were spun down and resuspended in ice-cold RPMI medium containing 10% heat-inactivated FCS plus glutamine plus penicillin/streptomycin (complete RPMI) at 2.0 x 10⁷ cells per ml. After 10 min on ice, 0.5 ml of cells (ie 1 x 10⁷ cells) was placed into a pre-cooled 4 mm electroporation cuvette containing 20 µg of plasmid DNA (Endo-free Maxiprep DNA dissolved in sterile water). The cells were electroporated at 300 v and 950 µF and then quickly removed into 0.5 ml of warmed complete RPMI medium in an Eppendorf tube. The cells were spun for at 3000 rpm for 1 min in a microfuge and placed at 37 °C for 15 min to recover from being electroporated.
The supernatant was then removed and the cells were plated out into a well of a 6-well dish in 4 ml of complete RPMI and left at 37 °C for 48 h to allow for expression of the antibiotic resistance marker.

After 48 h the cells were spun down and resupended into 10 ml fresh complete RPMI. This was then divided into 10 x 15 ml Falcon tubes and 8 ml of pre-warmed ClonaCell-TCS medium was added followed by 1 ml of a 10 x final concentration of the antibiotic being used for selection. For G418 selection the final concentration of G418 was 1 mg/ml so a 10 mg/ml solution in RPMI was prepared and 1 ml of this was added to each tube. The tubes were mixed well by inversion and allowed to settle for 15 min at room temperature before being plated out into 10 cm tissue culture dishes. These were then placed in a CO2 incubator for 14 days when that were examined for visible colonies.

Macroscopically visible colonies were picked off the plates and these colonies were expanded through 96-well plates to 24-well plates to T25 flasks - in complete RPMI containing 1 mg/ml G418.

The resulting clones were each transiently transfected with pLOR91 using Lipofectamine 2000 reagent (according to manufacturer's protocol) and then plated out onto a 96-well plate containing plate-bound immobilised hDeltal-IgG4Fc. A well-performing clone (#24) was selected and used for luciferase assays.

### Example 6

A series of truncations of sequences for modulating Notch signalling, based on human Deltal comprising varying numbers of EGF repeats, was prepared as follows:

### A) Delta 1 DSL domain plus EGF repeats 1-2

A human Delta 1 (DLL-1) deletion coding for the DSL domain and the first two only of the naturally occurring EGF repeats (ie omitting EGF repeats 3 to 8 inclusive) was generated by PCR from a DLL-1 extracellular (EC) domain/V5His clone (for the sequence of the human DLL-1 EC domain see Figures and, for example, Genbank Accession No. AF003522) using a primer pair as follows:
DLacl3: CACCAT GGGCAG TCGGTG CGCGCT GG;
   and
DLLld3-8: GTAGTT CAGGTC CTGGTT GCAG
PCR conditions were:
   1 cycle at 95°C/3 minutes;
   18 cycles of (95°C/1 minute, 60°C/1 minute, 72°C/2 minutes); and
   1 cycle at 72°C/2 minutes.

The DNA was then isolated from a 1% agarose gel in 1 x U/V-Safe TAE (Tris/acetate/EDTA) buffer (MWG-Biotech, Ebersberg, Germany) and used as a template for PCR with the following primers:
FcDL.4: CACCAT GGGCAG TCGGTG CGCGCT GG ; and
FcDLLd3-8:
   GGATAT GGGCCC TTGGTG GAAGCG TAGTTC AGGTCC TGGTTG CAG
PCR conditions were:
   1 cycle at 94°C/3 minutes;
   18 cycles of (94°C/1 minute, 68°C/1 minute, 72°C/2 minutes); and
   1 cycle at 72°C/10 minutes.

The fragment was ligated into pCRbluntII.TOPO (Invitrogen) and cloned in TOP10 cells (Invitrogen). Plasmid DNA was generated using a Qiagen Minprep kit (QIAprep^{™}) according to the manufacturer's instructions and the identity of the PCR products was confirmed by sequencing.

An IgFc fusion vector pCONγ (Lonza Biologics, UK) was cut with ApaI and HindIII then treated with shrimp alkaline phosphatase (Roche) and gel purified.

The DLL-1 deletions cloned in pCRbluntn were cut with HindIII (and EcoRV to aid later selection of the desired DNA product) followed by ApaI partial restriction. The sequences were then gel purified and ligated into the pCONγ vector which was cloned into TOP10 cells.

Plasmid DNA was generated using a Qiagen Minprep kit (QIAprep^{™}) according to the manufacturer's instructions.

The resulting construct (pCONγ hDLL1 EGF1-2) coded for the following DLL-1 amino acid sequence fused to the IgG Fc domain encoded by the pCONγ vector. 
MGSRCALALAVLSALLCQVWSSGVFELKLQEFVNKKGLLGNRNCCRGGAGPPPCACR
TFFRVCLKHYQASVSPEPPCTYGSAVTPVLGVDSFSLPDGGGADSAFSNPIRFPFGF
TWPGTFSLIIEALHTDSPDDLATENPERLISRLATQRHLTVGEEWSQDLHSSGRTDL
KYSYRFVCDEHYYGEGCSVFCRPRDDAFGHFTCGERGEKVCNPGWKGPYCTEPICLP
GCDEQHGFCDKPGECKCRVGWQGRYCDECIRYPGCLHGTCQQPWQCNCQEGWGGLFC
NQDLNY
 (wherein the emboldened portion of the sequence which is single underlined is the DSL domain and the emboldened portions of the sequence which are double underlined are EGF repeats 1 and 2 respectively).

### B) Delta 1 DSL domain plus EGF repeats 1-3

A human Delta 1 (DLL-1) deletion coding for the DSL domain and the first three only of the naturally occurring EGF repeats (ie omitting EGF repeats 4 to 8 inclusive) was generated by PCR from a DLL-1 DSL plus EGF repeats 1-4 clone using a primer pair as follows:
DLacl3: CACCATGGGCAGTCGGTGCGCGCTGG ; and 
FcDLLd4-8: GGA TAT GGG CCC TTG GTG GAA GCC TCG TCA ATC CCC AGC
TCG CAG
PCR conditions were:
   1cycle at 94°C/3 minutes;
   18 cycles of (94°C/1 minute, 68°C/1 minute, 72°C/2.5 minutes); and
   1 cycle at 72°C/10 minutes

The DNA was then isolated from a 1% agarose gel in 1 x U/V-Safe TAE (Tris/acetate/EDTA) buffer (MWG-Biotech, Ebersberg, Germany) and ligated into pCRbluntII.TOPO and cloned in TOP10 cells (Invitrogen). Plasmid DNA was generated using a Qiagen Minprep kit (QIAprep^{™}) according to the manufacturer's instructions and the identity of the PCR products was confirmed by sequencing.

An IgFc fusion vector pCONγ (Lonza Biologics, UK) was cut with ApaI and HindIII then treated with shrimp alkaline phosphatase (Roche) and gel purified.

The DLL-1 deletions cloned in pCRbluntII were cut with HindIII followed by ApaI partial restriction. The sequences were then gel purified and ligated into the pCONγ vector which was cloned into TOP 10 cells.

Plasmid DNA was generated using a Qiagen Minprep kit (QIAprep^{™}) according to the manufacturer's instructions and the identity of the PCR products was confirmed by sequencing.

The resulting construct (pCONγ hDLL1 EGF1-3) coded for the following DLL-1 sequence fused to the IgG Fc domain coded by the pCONγ vector. 
MGSRCALALAVLSALLCQVWSSGVFELKLQEFVNKKGLLGNRNCCRGGAGPPPCACR
TFFRVCLKHYQASVSPEPPCTYGSAVTPVLGVDSFSLPDGGGADSAFSNPIRFPFGF
TWPGTFSLIIEALHTDSPDDLATENPERLISRLATQRHLTVGEEWSQDLHSSGRTDL
KYSYRFVCDEHYYGEGCSVFCRPRDDAFGHFTCGERGEKVCNPGWKGPYCTEPICLP
GCDEQHGFCDKPGECKCRVGWQGRYCDECIRYPGCLHGTCQQPWQCNCQEGWGGLFC
NQDLNYCTHHKPCKNGATCTNTGQGSYTCSCRPGYTGATCELGIDE
 (wherein the emboldened portion of the sequence which is single underlined is the DSL domain and the emboldened portions of the sequence which are double underlined are EGF repeats 1 to 3 respectively).

### C) Delta 1 DSL domain plus EGF repeats 1-4

A human Delta 1 (DLL-1) deletion coding for the DSL domain and the first four only of the naturally occurring EGF repeats (ie omitting EGF repeats 5 to 8 inclusive) was generated by PCR from a DLL-1 EC domain/V5His clone using a primer pair as follows:
DLacl3: CACCAT GGGCAG TCGGTG CGCGCT GG
   and
DLLld5-8: GGTCAT GGCACT CAATTC ACAG
PCR conditions were:
   1 cycle at 95°C/3 minutes;
   18 cycles of (95°C/1 minute, 60°C/1 minute, 72°C/2.5 minutes); and
   1 cycle at 72°C/10 minutes.

The DNA was then isolated from a 1% agarose gel in 1 x U/V-Safe TAE (Tris/acetate/EDTA) buffer (MWG-Biotech, Ebersberg, Germany) and used as a template for PCR using the following primers:
FcDL.4: CACCAT GGGCAG TCGGTG CGCGCT GG; and
FcDLLd5-8:
   GGATAT GGGCCC TTGGTG GAAGCG GTCATG GCACTC AATTCA CAG
PCR conditions were:
   1 cycle at 94°C/3 minutes;
   18 cycles of (94°C/1 minute, 68°C/1 minute, 72°C/2.5 minutes); and
   1 cycle at 72°C/10 minutes.

The fragment was ligated into pCRbluntII.TOPO and cloned in TOP10 cells (Invitrogen). Plasmid DNA was generated using a Qiagen Minprep kit (QIAprep^{™}) according to the manufacturer's instructions and the identity of the PCR products was confirmed by sequencing.

An IgFc fusion vector pCONγ (Lonza Biologics, UK) was cut with ApaI and HindIII then treated with shrimp alkaline phosphatase (Roche) and gel purified.

The DLL-1 deletions cloned in pCRbluntII were cut with HindIII (and EcoRV to aid later selection of the desired DNA product) followed by ApaI partial restriction. The sequences were then gel purified and ligated into the pCONγ vector which was cloned into TOP 10 cells.

Plasmid DNA was generated using a Qiagen Minprep kit (QIAprep^{™}) according to the manufacturer's instructions and the identity of the PCR products was confirmed by sequencing.

The resulting construct (pCONγ hDLL1 EGF1-4) coded for the following DLL-1 sequence fused to the IgG Fc domain coded by the pCONγ vector. 
MGSRCALALAVLSALLCQVWSSGVFELKLQEFVNKKGLLGNRNCCRGGAGPPPCACR
TFFRVCLKHYQASVSPEPPCTYGSAVTPVLGVDSFSLPDGGGADSAFSNPIRFPFGF
TWPGTFSLIIEALHTDSPDDLATENPERLISRLATQRHLTVGEEWSQDLHSSGRTDL
KYSYRFVCDEHYYGEGCSVFCRPRDDAFGHFTCGERGEKVCNPGWKGPYCTEPICLP
GCDEQHGFCDKPGECKCRVGWQGRYCDECIRYPGCLHGTCQQPWQCNCQEGWGGLFC
NQDLNYCTHHKPCKNGATCTNTGQGSYTCSCRPGYTGATCELGIDECDPSPCKNGGS
CTDLENSYSCTCPPGFYGKICELSAMT
 (wherein the emboldened portion of the sequence which is single underlined is the DSL domain and the emboldened portions of the sequence which are double underlined are EGF repeats 1 to 4 respectively).

### D) Delta 1 DSL domain plus EGF repeats 1-7

A human Delta 1 (DLL-1) deletion coding for the DSL domain and the first seven of the naturally occurring EGF repeats (ie omitting EGF repeat 8) was generated by PCR from a DLL-1 EC domain/V5His clone using a primer pair as follows:
DLacl3: CACCAT GGGCAG TCGGTG CGCGCT GG and
DLL1d8: CCTGCT GACGGG GGCACT GCAGTT C
PCR conditions were:
   1 cycle at 95°C/3 minutes;
   18 cycles of (95°C/1 minute, 68°C/1 minute, 72°C/3 minutes); and
   1 cycle at 72°C/10 minutes.

The DNA was then isolated from a 1% agarose gel in 1 x U/V-Safe TAE (Tris/acetate/EDTA) buffer (MWG-Biotech, Ebersberg, Germany) and used as a template for PCR using the following primers:
FcDL.4: CACCAT GGGCAG TCGGTG CGCGCT GG and
FCDLLd8: GGATAT GGGCCC TTGGTG GAAGCC CTGCTG ACGGGG GCACTG CAGTTC
PCR conditions were:
   1 cycle at 94°C/3 minutes;
   18 cycles of (94°C/1minute, 68°C/1minute, 72°C/3minutes); and
   1 cycle at 72°C/10 minutes.

The fragment was ligated into pCRbluntII.TOPO and cloned in TOP10 cells (Invitrogen). Plasmid DNA was generated using a Qiagen Minprep kit (QIAprep^{™}) according to the manufacturer's instructions and the identity of the PCR products was confirmed by sequencing.

An IgFc fusion vector pCONγ (Lonza Biologics, UK) was cut with ApaI and HindIII then treated with shrimp alkaline phosphatase (Roche) and gel purified.

The DLL-1 deletions cloned in pCRbluntII were cut with HindIII (and EcoRV to aid later selection of the desired DNA product) followed by ApaI partial restriction. The sequences were then gel purified and ligated into the pCONγ vector which was cloned into TOP10 cells.

Plasmid DNA was generated using a Qiagen Minprep kit (QIAprep^{™}) according to the manufacturer's instructions and the PCR products were sequenced.

The resulting construct (pCONγ hDLL1 EGF1-7) coded for the following DLL-1 sequence fused to the IgG Fc domain coded by the pCONγ vector. 
MGSRCALALAVLSALLCQVWSSGVFELKLQEFVNKKGLLGNRNCCRGGAGPPPCACR
TFFRVCLKHYQASVSPEPPCTYGSAVTPVLGVDSFSLPDGGGADSAFSNPIRFPFGF
TWPGTFSLIIEALHTDSPDDLATENPERLISRLATQRHLTVGEEWSQDLHSSGRTDL
KYSYRFVCDEHYYGEGCSVFCRPRDDAFGHFTCGERGEKVCNPGWKGPYCTEPICLP
GCDEQHGFCDKPGECKCRVGWQGRYCDECIRYPGCLHGTCQQPWQCNCQEGWGGLFC
NQDLNYCTHHKPCKNGATCTNTGQGSYTCSCRPGYTGATCELGIDECDPSPCKNGGS
CTDLENSYSCTCPPGFYGKICELSAMTCADGPCFNGGRCSDSPDGGYSCRCPVGYSG
FNCEKKIDYCSSSPCSNGAKCVDLGDAYLCRCQAGFSGRHCDDNVDDCASSPCANGG
TCRDGVNDFSCTCPPGYTGRNCSAPVSR
 (wherein the emboldened portion of the sequence which is single underlined is the DSL domain and the emboldened portions of the sequence which are double underlined are EGF repeats 1 to 7 respectively).

### E) Transfection and Expression

### i) Transfection and expression of constructs of constructs A, C and D

Cos 1 cells were separately transfected with each of the expression constructs from A, C and D above (viz pCONγ hDLL1 EGF1-2, pCONγ hDLL1 EGF1-4, pCONγ hDLL1 EGF1-7) and pCONγ control as follows:

In each case 3x10⁶ cells were plated in a 10cm dish in Dulbecco's Modified Eagle's Medium (DMEM) + 10% Fetal Calf Serum (FCS) and cells were left to adhere to the plate overnight. The cell monolayer was washed twice with 5 ml phosphate-buffered saline (PBS) and cells left in 8 ml OPTIMEM^{™} medium (Gibco/Invitrogen). 12 µg of the relevant construct DNA was diluted into 810 µl OPTIMEM medium and 14 µl Lipofectamine2000^{™} cationic lipid transfection reagent (Invitrogen) was diluted in 810 µl OPTIMEM medium. The DNA-containing and Lipofectamine2000 reagent-containing solutions were then mixed and incubated at room temperature for a minimum of 20 minutes, and then added to the cells ensuring an even distribution of the transfection mix within the dish. The cells were incubated with the transfection reagent for 6 hours before the media was removed and replaced with 20 ml DMEM + 10% FCS.
Supernatant containing secreted protein was collected from the cells after 5 days and dead cells suspended in the supernatant were removed by centrifugation (4,500 rpm for 5 minutes). The resulting expression products were designated: hDLL1 EGF1-2 Fc (from pCONγ hDLL1 EGF1-2), hDLL1 EGF1-4 Fc (from pCONγ hDLL1 EGF1-4) and hDLL1 EGF1-7 Fc (from pCONγ hDLL1 EGF1-7).

Expression of the Fc fusion proteins was assessed by western blot. The protein in 10 µl of supernatant was separated by 13% SDS-PAGE and blotted by semi dry apparatus on to Hybond^{™}-ECL (Amersham Pharmacia Biotech) nitrocellulose membrane (17 V for 28 minutes). The presence of Fc fusion proteins was detected by Western blot using JDC14 anti-human IgG4 antibody diluted 1:500 in blocking solution (5% non-fat Milk solids in Tris-buffered saline with Tween 20 surfactant; TBS-T). The blot was incubated in this solution for 1 hour before being washed in TBS-T. After 3 washes of 5 minutes each, the presence of mouse anti-human IgG4 antibodies was detected using anti mouse IgG-HPRT conjugate antiserum diluted 1:10,000 in blocking solution. The blot was incubated in this solution for 1 hour before being washed in TBS-T (3 washes of 5 minutes each). The presence of Fc fusion proteins was then visualised using ECL^{™} detection reagent (Amersham Pharmacia Biotech).

The amount of protein present in 10 ml supernatant was assessed by comparing to Kappa chain standards containing 10 ng (7), 30ng (8) and 100 ng (9) protein.

### ii) Transfection and expression of constructs of construct B

Cos 1 cells were transfected with the expression construct from B above (viz pCONγ hDLL1 EGF1-3) as follows:
7.1x10⁵ cells were plated in a T25 flask in Dulbecco's Modified Eagle's Medium (DMEM) + 10% Fetal Calf Serum (FCS) and cells were left to adhere to the plate overnight. The cell monolayer was washed twice with 5 ml phosphate-buffered saline (PBS) and cells left in 1.14 ml OPTIMEM^{™} medium (Gibco/Invitrogen). 2.85 µg of the relevant construct DNA was diluted into 143 µl OPTIMEM medium and 14.3 µl Lipofectamine2000^{™} cationic lipid transfection reagent (Invitrogen) was diluted in 129 µl OPTIMEM medium and incubated at room temperature for 45 minutes. The DNA-containing and Lipofectamine2000 reagent-containing solutions were then mixed and incubated at room temperature for 15 minutes, and then added to the cells ensuring an even distribution of the transfection mix within the flask. The cells were incubated with the transfection reagent for 18 hours before the media was removed and replaced with 3 ml DMEM + 10% FCS. Supernatant containing secreted protein was collected from the cells after 4 days and dead cells suspended in the supernatant were removed by centrifugation (1,200 rpm for 5 minutes). The resulting expression product was designated: hDLL1 EGF1-3 Fc (from pCONγ hDLL1 EGF1-3).

### Example 7

### i) Preparation of modulator of Notch signalling in form of Notch ligand Extracellular domain fragment with free Cysteine tail for polymer coupling

A protein fragment comprising amino acids 1 to 332 (ie comprising DSL domain plus first 3 EGF repeats) of human Delta 1 (DLL-1; for sequence see GenBank Accession No AF003522) and ending with a free cysteine residue ("D1E3Cys") was prepared as follows:

A template containing the entire coding sequence for the extracellular (EC) domain of human DLL-1 (with two silent mutations) was prepared by a PCR cloning strategy from a placental cDNA library made from placental polyA+ RNA (Clontech; cat no 6518-1) and combined with a C-terminal V5HIS tag in a pCDNA3.1 plasmid (Invitrogen, UK) The template was cut HindIII to PmeI to provide a fragment coding for the EC domain and this was used as a template for PCR using primers as follows:
5'-primer: CAC CAT GGG CAG TCG GTG CGC GCT GG
3'-primer: GTC TAC GTT TAA ACT TAA CAC TCG TCA ATC CCC AGC TCG CAG GTG
PCR was carried out using Pfu turbo polymerase (Stratagene, La Jolla, CA, US) with cycling conditions as follows: 95C 5min, 95C 1min, 45-69C 1min, 72C 1min for 25 cycles, 72C 10min.

The products at 58C, 62C & 67C were purified from 1% agarose gel in 1 x TAE using a Qiagen gel extraction kit according to the manufacturer's instructions, ligated into pCRIIblunt vector (InVitrogen TOPO-blunt kit) and then transformed into TOP10 cells (InVitrogen). The resulting clone sequence was verified, and only the original two silent mutations were found to be present in the parental clone.

The resulting sequence coding for "D1E3Cys" was excised using PmeI and HindIII, purified on 1% agarose gel, 1x TAE using a Qiagen gel extraction kit and ligated into pCDNA3.1V5HIS (Invitrogen) between the PmeI and HindIII sites, thereby eliminating the V5HIS sequence. The resulting DNA was transformed into TOP10 cells. The resulting clone sequence was verified at the 3'-ligation site.

The D1E3Cys-coding fragment was excised from the pCDNA3.1 plasmid using PmeI and HindIII. A pEE14.4 vector plasmid (Lonza Biologics, UK) was then restricted using EcoRI, and the 5'-overhangs were filled in using Klenow fragment polymerase. The vector DNA was cleaned on a Qiagen PCR purification column, restricted using HindIII, then treated with Shrimp Alkaline Phosphatase (Roche). The pEE14.4 vector and DIE3cys fragments were purified on 1% agarose gel in 1 x TAE using a Qiagen gel extraction kit prior to ligation (T4 ligase) to give plasmid pEE14.4 DLLΔ4-8cys. The resulting clone sequence was verified.

The DIE3Cys coding sequence is as follows:

1 atgggcagtc ggtgcgcgct ggccctggcg gtgctctcgg ccttgctgtg

51 tcaggtctgg agctctgggg tgttcgaact gaagctgcag gagttcgtca
101 acaagaaggg gctgctgggg aaccgcaact gctgccgcgg gggcgcgggg
151 ccaccgccgt gcgcctgccg gaccttcttc cgcgtgtgcc tcaagcacta
201 ccaggccagc gtgtcccccg agccgccctg cacctacggc agcgccgtca
251 cccccgtgct gggcgtcgac tccttcagtc tgcccgacgg cgggggcgcc
301 gactccgcgt tcagcaaccc catccgcttc cccttcggct tcacctggcc
351 gggcaccttc tctctgatta ttgaagctct ccacacagat tctcctgatg
401 acctcgcaac agaaaaccca gaaagactca tcagccgcct ggccacccag
451 aggcacctga cggtgggcga ggagtggtcc caggacctgc acagcagcgg
501 ccgcacggac ctcaagtact cctaccgctt cgtgtgtgac gaacactact
551 acggagaggg ctgctccgtt ttctgccgtc cccgggacga tgccttcggc
601 cacttcacct gtggggagcg tggggagaaa gtgtgcaacc ctggctggaa
651 agggccctac tgcacagagc cgatctgcct gcctggatgt gatgagcagc
701 atggattttg tgacaaacca ggggaatgca agtgcagagt gggctggcag
751 ggccggtact gtgacgagtg tatccgctat ccaggctgtc tccatggcac
801 ctgccagcag ccctggcagt gcaactgcca ggaaggctgg gggggccttt
851 tctgcaacca ggacctgaac tactgcacac accataagcc ctgcaagaat
901 ggagccacct gcaccaacac gggccagggg agctacactt gctcttgccg
951 gcctgggtac acaggtgcca cctgcgagct ggggattgac gagtgttaa

The DNA was prepared for stable cell line transfection/selection in a Lonza GS system using a Qiagen endofree maxi-prep kit.

### ii) Expression of D1E3Cys

### Linearisation of DNA

The pEE14.4 DLLΔ4-8cys plasmid DNA from (i) above was linearised by restriction enzyme digestion with PvuI, and then cleaned up using phenol chloroform isoamyl alcohol (IAA), followed by ethanol precipitation. Plasmid DNA was checked on an agarose gel for linearisation, and spec'd at 260/280nm for quantity and quality of prep.

### Transfection

CHO-K1 cells were seeded into 6 wells at 7.5 x 10⁵ cells per well in 3ml media (DMEM 10% ECS) 24hrs prior to transfection, giving 95% confluency on the day of transfection. Lipofectamine 2000 was used to transect the cells using 5ug of linearised DNA. The transfection mix was left on the cell sheet for 5 ½ hours before replacing with 3ml semi-selective media (DMEM, 10% dFCS, GS) for overnight incubation.

At 24 hours post-transfection the media was changed to full selective media (DMEM (Dulbecco's Modified Eagle Medium), 10%dFCS (fetal calf serum), GS (glutamine synthase), 25uM L-MSX (methionine sulphoximine)) and incubated further.

Cells were plated into 96 wells at 10⁵ cells per well on days 4 and 15 after transfection.

96 well plates were screened under a microscope for growth 2 weeks post clonal plating. Single colonies were identified and scored for % confluency. When colony size was >30% media was removed and screened for expression by dot blot against anti-human-Delta-1 antisera. High positives were confirmed by the presence of a 36kDa band reactive to anti-human-Delta-1 antisera in PAGE Western blot of media.

Cells were expanded by passaging from 96 well to 6 well to T25 flask before freezing. The fastest growing positive clone (LC09 0001) was expanded for protein expression.

### D1E3Cys expression and purification

T500 flasks were seeded with 1 x 10⁷ cells in 80ml of selective media. After 4 days incubation the media was removed, cell sheet rinsed with DPBS and 150ml of 325 media with GS supplement added to each flask. Flasks were incubated for 7 further days before harvesting. Harvest media was filtered through a 0.65- 0.45um filter to clarify prior to freezing. Frozen harvests were purified by FPLC as follows:

Frozen harvest was thawed and filtered. A 17ml Q Sepharose column was equilibrated in 0.1M Tris pH8 buffer, for 10 column volumes. The harvest was loaded onto the column using a P1 pump set at 3ml/min, the flowthrough was collected into a separate container (this is a reverse purification - a lot of the BSA contaminant binds to the Q Sepharose FF and our target protein does not and hence remains in the flowthrough). The flowthrough was concentrated in a TFF rig using a 10kDa cut off filter cartridge, during concentration it was washed 3 x with 0.1M Sodium phosphate pH 7 buffer. The 500ml was concentrated down to 35ml, to a final concentration of 3mg/ml.

### Samples were run on SDS PAGE reduced and non-reduced.

The amino acid sequence of the resulting expressed DIE3Cys protein was as follows: 
MGSRCALALAVLSALLCQVWSSGVFELKLQEFVNKKGLLGNRNCCRGGAGPPPCACRTF
FRVCLKHYQASVSPEPPCTYGSAVTPVLGVDSFSLPDGGGADSAFSNPIRFPFGFTWPG
TFSLIIEALHTDSPDDLATENPERLISRLATQRHLTVGEEWSQDLHSSGRTDLKYSYRF
VCDEHYYGEGCSVFCRPRDDAFGHFTCGERGEKVCNPGWKGPYCTEPICLPGCDEQHGF
CDKPGECKCRVGWQGRYCDECIRYPGCLHGTCQQPWQCNCQEGWGGLFCNQDLNYCTHH
KPCKNGATCTNTGQGSYTCSCRPGYTGATCELGIDEC
 (wherein the sequence in italics is the leader peptide, the underlined sequence is the DSL domain, the bold sequences are the three EGF repeats, and the terminal Cys residue is shown bold underlined).

### iii) Reduction of D1E3cys Protein

40µg D1E3Cys protein from (ii) above was made up to 100µl to include 100mM sodium phosphate pH 7.0 and 5mM EDTA. 2 volumes of immobilised TCEP (tris[2-carboxyethyl]phosphine hydrochloride; Pierce, Rockford, IL, US, Cat No: 77712; previously washed 3 times 1ml 100mM sodium phosphate pH 7.0) were added and the mixture was incubated for 30 minutes at room temperature, with rotating.

The resin was pelleted at room temperature in a microfuge (13,000 revs/min, 5 minutes) and the supernatant was transferred to a clean Eppendorf tube and stored on ice. Protein concentration was measured by Warburg-Christian method.

This fragment is linked to a polymer such as dextran or PEG as described above to provide the final conjugate.

### Example 8

### Coupling of DIE3cys to Amino-Dextran to provide conjugate

### i) Purification of expressed DIE3Cys by HIC

Harvests from Example 7 above were purified using Hydrophobic Interaction Chromatography (HIC), the eiuate was then concentrated and buffer exchanged using centrifugal concentrators according to the manufacturers' instructions. The purity of the product was determined by SDS PAGE.

### ii) Maleimide substitution of amino-dextran (polymer activation)

Amino-dextran of molecular mass 500,000 Da (dextran, amino, 98 moles amine/mole; Molecular Probes, ref D-7144), 3.2 mg/ml, was derivatised/activated with sulfo-SMCC (sulfosuccinimidyl 4-[N-maleimidomethyl]-cyclohexane-1-carboxylate; Pierce, ref 22322) at 73 moles sulfo-SMCC per mole amino-dextran in 100mM sodium phosphate pH8.0 for 1h, 22°C.

The amino content of the dextran and the level of maleimide substitution was measured using a Ninhydrin assay. Aliquots of dextran derivative or B-alanine (Sigma, A-7752) were made to 50 µl in 100mM sodium phosphate pH7.0 and diluted in water to 250 µl. Ninhydrin reagent solution (Sigma, N1632) was added, 1 vol., and samples heated 100°C, 15 min. After cooling on ice 1 vol. 50% ethanol was added, mixed and the solution clarified by centrifugation. Absorbance was recorded at 570nm.

The resulting maleimido-dextran was purified and concentrated by buffer exchange using Vivaspin 6ml concentrators (VivaScience, VS0612) and 3 x 5ml, 100mM sodium phosphate pH7.0.

The concentration of dextran was measured using an ethanol precipitation/turbidity assay. Aliqouts of dextran derivative were made to 50 µl in 100mM sodium phosphate pH7.0. Water was added to make 500 µl final volume, dextran was precipitated by the addition of 1 vol. absolute ethanol and absorbance was recorded at 600nm.

### iii) Partial reduction of D1E3cys

D1E3cys protein (purified as in (i) above) at 1 mg/ml in 100mM sodium phosphate pH7.0 was reduced using TCEP.HCl (Tris(2-carboxyethyl)phosphine hydrochloride; Pierce, 20490) at a 10-fold molar excess of reducing agent for 1h at 22°C. The protein was purified by buffer exchange using Sephadex G-25, PD-10 columns (Amersham biosciences, 17-0851-01) into 100mM sodium phosphate pH7.0 followed by concentration in Vivaspin 6ml concentrators. Protein concentration was estimated using the Warburg-Christian A280/A260 method.

The efficiency of reduction can be estimated using the Ellman's assay. The supplied D1E3cys protein has no free thiol groups, whereas partially reduced D1E3cys is predicted to have a single free thiol group per mole of protein. Using a 96-well microtitre plate, aliqouts of D1E3cys protein or L-cysteine hydrochloride (Sigma, C-1276) were made to 196 ul in 100mM sodium phosphate pH7.0 and 4ul 4 mg/ml Ellman's reagent (in 100mM sodium phosphate pH 7.0) was added. Reactions were incubated for 15 min at 22°C and absorbance was recorded at 405nm.

### iv) Coupling of Reduced D1E3cys to Maleimido-Dextran.

The derivatized maleimido-dextran was added to concentrated, reduced D1E3cys at a 1 : 75 molar ratio of dextran to D1E3cys. Coupling proceeded for 18h, 4°C.

The resulting D1E3cys-dextran polymer (D1E3Cys-dextran conjugate; comprising aminodextrans each coupled to a large number of D1E3Cys proteins via SMCC linkers) was purified by gel permeation chromatography using a Superdex 200 (Amersham. Biosciences, 17-1043-10) column attached to an AKTA purifier FPLC (Amersham. Biosciences) in 100mM sodium phosphate pH7.0. At a flow rate of 1ml/min, 1ml fractions were collected. The protein complex was then concentrated in Vivaspin 6mL concentrators and protein concentration was measured using the Warburg-Christian A280/A260 method.

The complex was analysed on SDS-PAGE gel and screened for endotoxin contamination prior to activity assays *in vitro* and *in vivo* as described below.

### Example 9

### Co-administration of KLH beads and dextran-D1E3cys conjugate in vivo

### i) Coating of beads with KLH

Imject® Mariculture Keyhole Limpet Hemocyanin (mcKLH) in PBS Buffer (lyophilized from PBS) 20mg (Pierce product number 77600) was reconstituted with 2.0ml dH₂O to make a 10mg/ml solution containing PBS, pH 7.2 with proprietary stabilizer.

Surfactant-free White Aldehyde/Sulfate Latex Beads (Interfacial Dynamics corp Portland. or USA batch number 1813) concentration 5.8x10⁸ beads/ml were washed in PBS x3 (spun for 10mins at 13k RT). The beads were then resuspended at 2x10⁸ beads/ml in 500µg/ml mcKLH in PBS and horizontally rotated at 37°C overnight. Beads were then washed again in PBS x3 (spun for 10mins at 13k RT) and resuspended in PBS at the required concentration. Successful coating of the beads was checked by their ability to neutralize an anti-KLH antiserum in an ELISA system.

### ii) in vivo administration with D1E3Cys/dextran conjugate

6-8 weeks old female Balb/c mice were injected s.c. at the base of the tail with 2 x 10⁶ KLH coated beads (prepared as described in (i) above) per mouse. Dextran-D1E3cys conjugate from Example 5 above (250, 50 or 10 µg per mouse), D1E3Cys alone (control) or dextran alone (control) were injected s.c. in a close separate site of the tail base (all agents were administered as aqueous solutions;100 mM sodium phosphate at pH7).

Mice were challenged after 7 days in the right ear with 20 µg of KLH. The increase in ear swelling (right ear - left ear) was measured using a digital calliper.

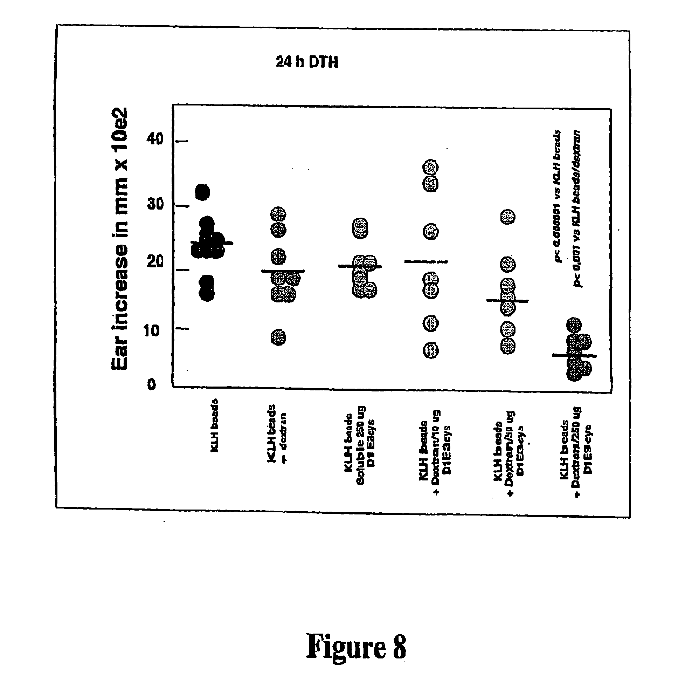

Results are shown in Figure 8. As can be seen, the control groups (KLH beads, KLH beads plus dextran alone and KLH beads plus soluble D1E3Cys alone) showed a similar degree of response. KLH beads plus D1E3cys/dextran conjugate 250 µg showed a significant decreased DTH response at 24 hours.

### Example 10

### Coupling of D1E3cys to iron/dextran microbeads

### i) Purification of expressed D1E3Cys by HIC

D1E3Cys Harvests from Example 8 above were purified using Hydrophobic Interaction Chromatography (HIC), the eluate was then concentrated and buffer exchanged using centrifugal concentrators according to the manufacturers' instructions. The purity of the product was determined by SDS PAGE .

### ii) Partial reduction of D1E3cys

D1E3cys protein (purified as in (i) above) at 1 mg/ml in 100mM sodium phosphate pH7.0 was reduced using TCEP.HCl (Tris(2-carboxyethyl)phosphine hydrochloride; Pierce, 20490) at a 10-fold molar excess of reducing agent for 1h at 22°C. The protein was purified by buffer exchange using Sephadex G-25, PD-10 columns (Amersham Biosciences, 17-0851-01) into 100mM sodium phosphate pH7.0 followed by concentration in Vivaspin 6ml concentrators. Protein concentration was estimated using the Warburg-Christian A280/A260 method.

The efficiency of reduction can be estimated using the Ellman's assay. The supplied D1E3cys protein has no free thiol groups, whereas partially reduced D1E3cys is predicted to have a single free thiol group per mole of protein. Using a 96-well microtitre plate, aliqouts of D1E3cys protein or L-cysteine hydrochloride (Sigma, C-1276) were made to 196 ul in 100mM sodium phosphate pH7.0 and 4ul 4 mg/ml Ellman's reagent (in 100mM sodium phosphate pH 7.0) was added. Reactions were incubated for 15 min at 22°C and absorbance was recorded at 405nm.

### iii) Coupling of Reduced D1E3cys to Beads.

D1E3Cys was coupled to beads from Miltenyi Biotec (Bisley, Surrey, LTK and Auburn, CA, US; eg product reference 130-048-001) by reductive coupling. The beads are superparamagnetic iron-dextran particles with a mean particle diameter of approximately 50 nm.

### Example 11

### Co-administration of KLH beads and D1E3Cys-coupled microbeads in vivo

### i) Coating of beads with KLH

Imject® Mariculture Keyhole Limpet Hemocyanin (mcKLH) in PBS Buffer (lyophilized from PBS) 20mg (Pierce product number 77600) was reconstituted with 2.0ml dH₂O to make a 10mg/ml solution containing PBS, pH 7.2 with proprietary stabilizer.

Surfactant-free White Aldehyde/Sulfate Latex Beads (Interfacial Dynamics corp Portland or USA batch number 1813) concentration 5.8x10⁸ beads/ml were washed in PBS x3 (spun for 10mins at 13k RT). The beads were then resuspended at 2x10⁸ beads/ml in 500µg/ml mcKLH in PBS and horizontally rotated at 37°C overnight. Beads were then washed again in PBS x3 (spun for 10mins at 13k RT) and resuspended in PBS at the required concentration. Successful coating of the beads was checked by their ability to neutralize an anti-KLH antiserum in an ELISA system.

### ii) in vivo administration with D1E3Cys-coupled beads

6-8 weeks old female Balb/c mice were injected s.c. at the base of the tail with 2 x 10⁶ KLH coated beads (prepared as described above) per mouse. Particles bearing modulators of Notch signalling (D1E3cys-coupled beads from Example 10 above; 0.6 or 7 µg protein per mouse); D4E3Cys protein alone (7 µg per mouse; control); Protein G-coupled beads (Miltenyi Cat No 130-071-101; control); or LPS 0.76 ng/mouse in Na₂PO₄ buffer (100 ul) were injected s.c. in a close separate site of the tail base (all agents were administered as aqueous solutions; 100 mM sodium phosphate at pH 7). In each case 8 mice were used in each group and one group was left untreated.

Groups were thus as follows:
**A**: n=8, 2x10⁶ KLH beads/mouse, 100ul s.c.,(1 site 100 ul each) tail base, + 7 ug D1E3cys-coated Miltenyi beads/mouse, 100 ul (1 site 100 ul each) s.c. tail base
**B**: n=8, 2x10⁶ KLH beads/mouse, 100u1 s.c..,(1 site 100 ul each) tail base + 0.6 ug D1E3cys-coated Miltenyi beads/mouse, 100 ul (1 site 100 ul each) s.c. tail base
**C**: n=8,2x10⁶ KLH beads/mouse, 100u1 s.c.,(1 site 100 ul each) tail base, + Protein G-coated Miltenyi beads, 100 ul/mouse (1 site 100 ul each) s.c.tail base
**D**: n=8, 2x10⁶ KLH beads/mouse, 100ul s.c., (1 site 100 ul each) tail base + 7 ug soluble D1E3cys 100 ul (1 site 100 ul each) tail base
**E**: n=8, 2x10⁶KLH beads/mouse, 100ul s.c., (1 site 100 ul each) tail base + LPS 0.76 ng/mouse in Na2PO4 buffer 100 ul (1 site 100 ul each)s.c. tail base
**F**: n=8, 2x10⁶KLH beads/mouse, 100ul s.c., (1 site 100 ul each) tail base + Saline 100 ul (1 site 100 ul each)s.c. tail base
**G**: Untreated

Mice were challenged after 7 days in the right ear with 20 µg of KLH. The increase in ear swelling (right ear - left ear) was measured for the following four days using a digital calliper.

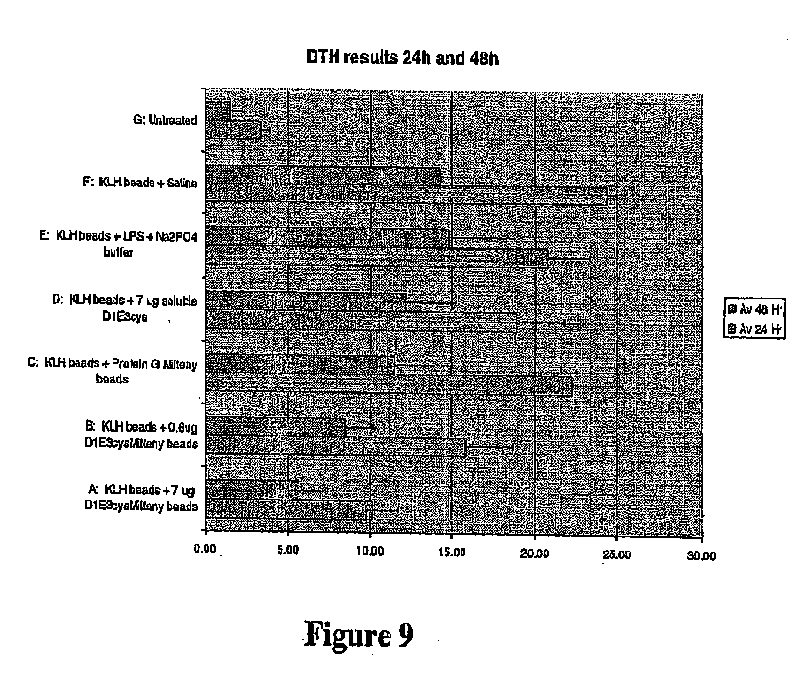

### Results are shown in Figure 9.

### Example 12

### Co-administration of KLH beads and DIE3Cys-coupled microbeads in vivo (bystander effect)

### In vivo administration with D1E3Cys-coupled beads

6-8 weeks old female Balb/c mice were injected s.c. at the base of the tail with 2 x 10⁶ KLH coated beads (prepared as described above) per mouse. Particles bearing modulators of Notch signalling (D1E3cys-coupled beads prepared as described above; 0.6 or 7 µg protein per mouse); D1E3Cys protein alone (7 µg per mouse); Protein G-coupled beads (Miltenyi Cat No 130-071-101; control); or LPS 0.76 ng/mouse in Na₂PO₄ buffer (100 ul) were injected s.c. in a close separate site of the tail base (all agents were administered as aqueous solutions; 100 mM sodium phosphate at pH 7). In each case 8 mice were used in each group and one group was left untreated.

### Groups were thus as follows:

### Untreated:

(8 mice) Untreated

### KLH Only:

(8 mice), 2x10⁶ KLH beads/mouse, 100ul s.c. at tail base + Saline 100 ul (1 site 100 ul each) s.c. tail base

### KLH plus Buffer Control:

(8 mice), 2x10⁶ KLH beads/mouse, 100ul s.c. at tail base + LPS 0.76 ng/mouse in Na2PO4 buffer 100 ul s.c. at tail base

### KLH + D1E3Cys-Beads:

(8 mice), 2x10⁶ KLH beads/mouse, 100ul s.c. at tail base, + 7 ug D1E3cys-coated Miltenyi beads/mouse, 100 ul s.c. tail base

After 14 days, mice were injected s.c. in a close separate site of the tail base with KLH 5 ng + ovalbumin (OVA) 100ug/100ul Saline:CFA (1:1).

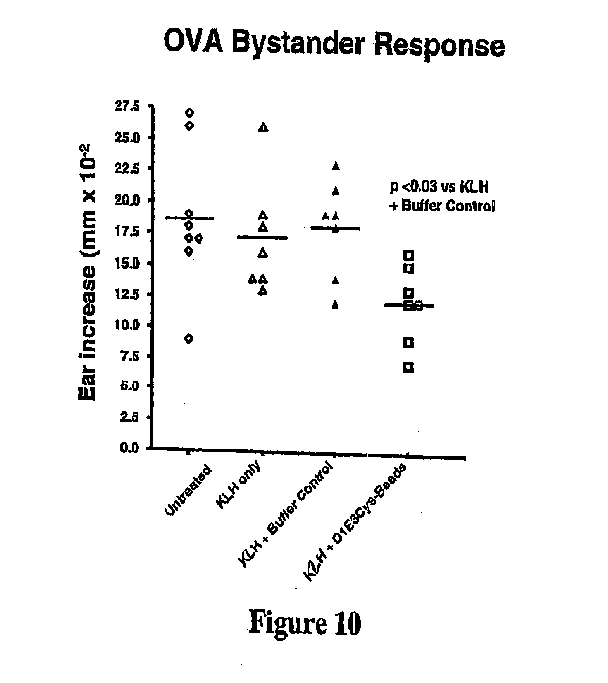

2 weeks later mice were challenged in the right ear with OVA 20 ug/20 ul. The increase in ear swelling (right ear - left ear) was measured for the following four days using a digital calliper. Results are shown in Figure 10.

In this case KLH can be seen as the bystander antigen and OVA as the target antigen. The suppression seen in the mice treated with the D1E3Cys coated beads (modulator of Notch signaling) is indicative of a bystander suppression effect (p < 0.03 vs KLH + buffer, student's t-test).

### References

Altman JD et al Science 1996 274: 94-6.
Artavanis-Tsakonas S, et al. (1995) Science 268:225-232.
Artavanis-Tsakonas S, et al. (1999) Science 284:770-776.
Brucker K, et al. (2000) Nature 406:411-415.
Camilli et al. (1994) Proc Natl Acad Sci USA 91:2634-2638.
Chee M. et al. (1996) Science 274:601-614.
Dkuz et al (1997) Cell 90: 271-280
Hemmati-Brivanlou and Melton (1997) Cell 88:13-17.
Hicks C, et al. (2000) Nat. Cell. Biol. 2:515-520.
Iemura et al. (1998) PNAS 95:9337-9345.
Irvine KD (1999) Curr. Opin. Genet. Devel. 9:434-441.
Ju BJ, et al. (2000) Nature 405:191-195.
Leimeister C. et al. (1999) Mech Dev 85(1-2):173-7.
Li et al. (1998) Immunity 8(1):43-55.
Lieber, T. et al. (1993) Genes Dev 7(10): 1949-65.
Lu, F. M. et al. (1996) Proc Natl Acad Sci 93(11):5663-7.
Matsuno K, et al. (1998) Nat. Genet. 19:74-78.
Matsuno, K. et al. (1995) Development 121(8):2633-44.
McGuinness T. Et al (1996) Genomics 35(3):473-85
Medhzhitov et al. (1997) Nature 388:394-397.
Meuer S. et al (2000) Rapid Cycle Real-time PCR, Springer-Verlag Berlin and Heidelberg GmbH & Co.
Moloney DJ, et al. (2000) Nature 406:369-375.
Munro S, Freeman M. (2000) Curr. Biol. 10:813-820.
Ordentlich et al. (1998) Mol. Cell. Biol. 18:2230-2239.
Osbome B, Miele L. (1999) Immunity 11:653-663.
Panin VM, et al. (1997) Nature 387:908-912.
Sasai et al. (1994) Cell 79:779-790.
Schroeter, E.H. et al. (1998) Nature 393(6683):382-6.
Struhl, G. et al. (1998) Cell 93(4):649-60.
Takebayashi K. et al. (1994) J Biol Chem 269(7):150-6.
Tamura K, et al. (1995) Curr. Biol. 5:1416-1423.
Valenzuela et al. (1995) J. Neurosci. 15:6077-6084.
Weinmaster G. (2000) Curr. Opin. Genet. Dev. 10:363-369. Wilson and Hemmati-Brivanlou (1997) Neuron 18:699-710.
Zhao et al. (1995) J. Immunol. 155:3904-3911.

## Claims

1. A product comprising i) a modulator of the Notch signalling pathway; and ii) an allergen or antigenic determinant thereof, or a polynucleotide coding for an allergen or antigenic determinant thereof; as a combined preparation for simultaneous, contemporaneous, separate or sequential use for modulation of the immune system wherein the modulator of the Notch signalling pathway comprises a Notch ligand comprising an N-terminal domain, a DSL domain and the first 3 only EGF repeats of Deltal, or a polynucleotide coding therefor.

2. A product as claimed in claim 1 for modulation of peripheral T-cell activation.

3. A product as claimed in claim 1 for use in reducing an immune response to an allergen or antigenic determinant thereof.

4. A product as claimed in claim 1 for use in promoting immune tolerance to an allergen or antigenic determinant thereof.

5. A product as claimed in claim 1 for use in the treatment of pollen, mite, cockroach, food, nut, venom, latex, animal dander, drug or insect allergy.

6. A product as claimed in any one of the preceding claims in the form of a pharmaceutical composition.

7. A combination of i) a modulator of the Notch signalling pathway and ii) an allergen or antigenic determinant thereof or a polynucleotide coding for an allergen or antigenic determinant thereof; for simultaneous, contemporaneous, separate or sequential use for the treatment of allergy wherein the modulator of the Notch signalling pathway comprises a Notch ligand comprising an N-terminal domain, a DSL domain and the first 3 only EGF repeats of Deltal, or a polynucleotide coding therefor.

8. A pharmaceutical composition comprising i) a modulator of the Notch signalling pathway, ii) an allergen or antigenic determinant thereof, or a polynucleotide coding for an allergen or antigenic determinant thereof and iii) a pharmaceutically acceptable carrier wherein the modulator of the Notch signalling pathway comprises a Notch ligand comprising an N-terminal domain, a DSL domain and the first 3 only EGF repeats of Deltal, or a polynucleotide coding therefor.

9. The use of a modulator of the Notch signalling pathway in the manufacture of a medicament for the treatment of allergy in simultaneous, contemporaneous, separate or sequential combination with an allergen or antigenic determinant thereof or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

10. The use of a combination of i) a modulator of the Notch signalling pathway; and ii) an allergen or antigenic determinant thereof or a polynucleotide coding for an allergen or antigenic determinant thereof, in the manufacture of a medicament for the treatment of allergy wherein the modulator of the Notch signalling pathway comprises a Notch ligand comprising an N-terminal domain, a DSL domain and the first 3 only EGF repeats of Deltal, or a polynucleotide coding therefor.

11. The use of a modulator of the Notch signalling pathway in the manufacture of a medicament for the treatment of allergy wherein the modulators of the Notch signalling pathway comprises a Notch ligand comprising an N-terminal domain, a DSL domain and the first 3 only EGF repeats of Deltal, or a polynucleotide coding therefor.

12. The use of a modulator of the Notch signalling pathway in the manufacture of a medicament for reducing an immune response to an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

13. The use of a modulator of the Notch signalling pathway in the manufacture of a medicament for promoting tolerance to an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

14. The use of a modulator of the Notch signalling pathway in the manufacture of a medicament for reducing an immune response to an allergen in a mammal in simultaneous, contemporaneous, separate or sequential combination with:
an allergen or antigenic determinant thereof, or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

15. The use of a modulator of the Notch signalling pathway in the manufacture of a medicament for promoting immune tolerance to an allergen or antigenic determinant thereof in a mammal in simultaneous, contemporaneous, separate or sequential combination with an allergen or antigenic determinant thereof, or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

16. A modulator of the Notch signalling pathway for use to treat allergy in simultaneous, contemporaneous, separate or sequential combination with an allergen or antigenic determinant thereof or a polynucleotide coding for an allergen or antigenic determinant thereof, wherein the modulator of the Notch signalling pathway comprises a Notch ligand comprising an N-terminal domain, a DSL domain and the first 3 only EGF repeats of Deltal, or a polynucleotide coding therefor.

17. A combination of i) a modulator of the Notch signalling pathway; and ii) an allergen or antigenic determinant thereof or a polynucleotide coding for an allergen or antigenic determinant thereof for use in the treatment of allergy wherein the modulator of the Notch signalling pathway comprises a Notch ligand comprising an N-terminal domain, a DSL domain and the first 3 only EGF repeats of Deltal, or a polynucleotide coding therefor.

18. A modulator of the Notch signalling pathway for treatment of allergy in simultaneous, contemporaneous, separate or sequential combination with an allergen or antigenic determinant thereof or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

19. A modulator of the Notch signalling pathway for use in the treatment of allergy wherein the modulator of the Notch signalling pathway comprises a Notch ligand comprising an N-terminal domain, a DSL domain and the first 3 only EGF repeats of Deltal, or a polynucleotide coding therefor.

20. A modulator of the Notch signalling pathway for use in reducing an immune response to an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

21. A modulator of the Notch signalling pathway for use in promoting tolerance to an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

22. A modulator of the Notch signalling pathway for use in reducing an immune response to an allergen in a mammal in simultaneous, contemporaneous, separate or sequential combination with an allergen or antigenic determinant thereof, or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

23. A modulator of the Notch signalling pathway for use in promoting immune tolerance to an allergen or antigenic determinant thereof in a mammal in simultaneous, contemporaneous, separate or sequential combination with an allergen or antigenic determinant thereof, or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

24. A pharmaceutical or veterinary kit comprising a modulator of the Notch signalling pathway and an allergen or antigenic determinant thereof or a polynucleotide coding for an allergen or antigenic determinant thereof wherein the modulator of the Notch signalling pathway comprises a Notch ligand comprising an N-terminal domain, a DSL domain and the first 3 only EGF repeats of Deltal, or a polynucleotide coding therefor.

## Patentansprüche

1. Produkt, welches i) einen Modulator des Notch-Signalwegs und ii) ein Allergen oder eine Antigendeterminante davon oder ein für ein Allergen oder eine Antigendeterminante davon kodierendes Polynukleotid umfasst, als kombinierte Präparation zur simultanen, gleichzeitigen, getrennten oder sequentiellen Verwendung zur Modulation des Immunsystems, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

2. Produkt gemäß Anspruch 1 zur Modulation der peripheren T-Zell-Aktivierung.

3. Produkt gemäß Anspruch 1 zur Verwendung bei der Reduzierung einer Immunantwort auf ein Allergen oder eine Antigendeterminante davon.

4. Produkt gemäß Anspruch 1 zur Verwendung bei der Förderung der Immuntoleranz gegen ein Allergen oder eine Antigendeterminante davon.

5. Produkt gemäß Anspruch 1 zur Verwendung bei der Behandlung einer Pollen-, Milben-, Schaben-, Lebensmittel-, Nuss-, Gift-, Latex-, Tierschuppen-, Wirkstoff- oder Insektenallergie.

6. Produkt gemäß einem der vorangehenden Ansprüche in der Form einer pharmazeutischen Zusammensetzung.

7. Kombination von i) einem Modulator des Notch-Signalwegs und ii) einem Allergen oder einer Antigendeterminante davon oder einem für ein Allergen oder eine Antigendeterminante davon kodierenden Polynukleotid, zur simultanen, gleichzeitigen, getrennten oder sequentiellen Verwendung zur Behandlung einer Allergie, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

8. Pharmazeutische Zusammensetzung, welche i) einen Modulator des Notch-Signalwegs, ii) ein Allergen oder eine Antigendeterminante davon oder ein für ein Allergen oder eine Antigendeterminante davon kodierendes Polynukleotid und iii) einen pharmazeutisch verträglichen Träger umfasst, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

9. Verwendung eines Modulators des Notch-Signalwegs bei der Herstellung eines Medikaments zur Behandlung einer Allergie in simultaner, gleichzeitiger, separater oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

10. Verwendung einer Kombination von i) einem Modulator des Notch-Signalwegs und ii) einem Allergen oder einer Antigendeterminante davon oder einem für ein Allergen oder eine Antigendeterminante davon kodierenden Polynukleotid bei der Herstellung eines Medikaments zur Behandlung einer Allergie, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

11. Verwendung eines Modulators des Notch-Signalwegs bei der Herstellung eines Medikaments zur Behandlung einer Allergie, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

12. Verwendung eines Modulators des Notch-Signalwegs bei der Herstellung eines Medikaments zur Reduzierung einer Immunantwort auf ein Allergen oder eine Antigendeterminante davon, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

13. Verwendung eines Modulators des Notch-Signalwegs bei der Herstellung eines Medikaments zur Förderung der Toleranz gegen ein Allergen oder eine Antigendeterminante davon, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

14. Verwendung eines Modulators des Notch-Signalwegs bei der Herstellung eines Medikaments zur Reduzierung einer Immunantwort gegen ein Allergen in einem Säuger, in simultaner, gleichzeitiger, getrennter oder sequentieller Kombination mit einem Allergen oder einer Antigedeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

15. Verwendung eines Modulators des Notch-Signalwegs bei der Herstellung eines Medikaments zur Förderung der Immuntoleranz gegen ein Allergen oder eine Antigendeterminante davon in einem Säuger in simultaner, gleichzeitiger, getrennter oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

16. Modulator des Notch-Signalwegs zur Verwendung bei der Behandlung einer Allergie in simultaner, gleichzeitiger, getrennter oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

17. Kombination von i) einem Modulator des Notch-Signalwegs und ii) einem Allergen oder einer Antigendeterminante davon oder einem für ein Allergen oder eine Antigendeterminante davon kodierenden Polynukleotid zur Verwendung bei der Behandlung einer Allergie, wobei der Modulator des Notch-Signalwegs einen Notch-Liganden umfasst, welcher eine
N-endständige Domäne, eine DSL-Domäne und nur die ersten 3 EGF-Wiederholungen von Delta1 umfasst, oder ein dafür kodierendes Polynukleotid.

18. Modulator des Notch-Signalwegs zur Behandlung einer Allergie in simultaner, gleichzeitiger, getrennter oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

19. Modulator des Notch-Signalwegs zur Verwendung bei der Behandlung einer Allergie, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

20. Modulator des Notch-Signalwegs zur Verwendung bei der Reduzierung einer Immunantwort auf ein Allergen oder eine Antigendeterminante davon, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

21. Modulator des Notch-Signalwegs zur Verwendung bei der Förderung der Toleranz gegen ein Allergen oder eine Antigendeterminante davon, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

22. Modulator des Notch-Signalwegs zur Verwendung bei der Reduzierung einer Immunantwort gegen ein Allergen in einem Säuger in simultaner, gleichzeitiger, getrennter oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

23. Modulator des Notch-Signalwegs zur Verwendung bei der Förderung der Immuntoleranz gegen ein Allergen oder eine Antigendeterminante davon in einem Säuger in simultaner, gleichzeitiger, getrennter oder sequentieller Kombination mit einem Allergen oder einer Antigendeterminante davon oder einem für ein Allergen oder eine Antigendeterminante davon kodierenden Polynukleotid, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

24. Pharmazeutisches oder tierärztliches Kit, welches ein Modulator des Notch-Signalwegs und ein Allergen oder eine Antigendeterminante davon oder ein für ein Allergen oder eine Antigendeterminante davon kodierendes Polynukleotid umfasst, wobei der Modulator des Notch-Signalwegs einen Notch-Liganden umfasst, welcher eine N-endständige Domäne, eine DSL-Domäne und nur die ersten 3 EGF-Wiederholungen von Delta1 umfasst, oder ein dafür kodierendes Polynukleotid.

## Revendications

1. Produit comprenant i) un modulateur de la voie de signalisation Notch ; et ii) un allergène ou un déterminant antigénique de celui-ci, ou un polynucléotide codant pour un allergène ou un déterminant antigénique de celui-ci ; sous la forme d'une préparation combinée pour une utilisation simultanée, contemporaine, séparée ou séquentielle pour la modulation du système immunitaire où le modulateur de la voie de signalisation Notch comprend un ligand de Notch comprenant un domaine N-terminal, un domaine DSL et les 3 premières répétitions seulement de type EGF de Deltal, ou un polynucléotide codant pour celui-ci.

2. Produit selon la revendication 1, pour la modulation de l'activation des cellules T périphériques.

3. Produit selon la revendication 1, pour une utilisation dans la réduction d'une réponse immunitaire à un allergène ou un déterminant antigénique de celui-ci.

4. Produit selon la revendication 1, pour une utilisation destinée à favoriser la tolérance immunitaire à un allergène ou un déterminant antigénique de celui-ci.

5. Produit selon la revendication 1, pour une utilisation dans le traitement d'une allergie aux pollens, aux acariens, aux cafards, alimentaire, aux noix, aux venins, au latex, aux phanères d'animaux, aux médicaments ou aux insectes.

6. Produit selon l'une quelconque des revendications précédentes sous la forme d'une composition pharmaceutique.

7. Combinaison i) d'un modulateur de la voie de signalisation Notch et ii) d'un allergène ou d'un déterminant antigénique de celui-ci ou d'un polynucléotide codant pour un allergène ou un déterminant antigénique de celui-ci ; pour une utilisation simultanée, contemporaine, séparée ou séquentielle pour le traitement d'une allergie où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

8. Composition pharmaceutique comprenant i) un modulateur de la voie de signalisation Notch, ii) un allergène ou un déterminant antigénique de celui-ci ou un polynucléotide codant pour un allergène ou un déterminant antigénique de celui-ci et iii) un support pharmaceutiquement acceptable, où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

9. Utilisation d'un modulateur de la voie de signalisation Notch dans la fabrication d'un médicament destiné au traitement d'une allergie dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1 , ou un polynucléotide codant pour celui-ci.

10. Utilisation d'une combinaison i) d'un modulateur de la voie de signalisation Notch ; et ii) d'un allergène ou d'un déterminant antigénique de celui-ci ou d'un polynucléotide codant pour un allergène ou un déterminant antigénique de celui-ci, dans la fabrication d'un médicament destiné au traitement d'une allergie où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

11. Utilisation d'un modulateur de la voie de signalisation Notch dans la fabrication d'un médicament destiné au traitement d'une allergie où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

12. Utilisation d'un modulateur de la voie de signalisation Notch dans la fabrication d'un médicament destiné à la réduction d'une réponse immunitaire à un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

13. Utilisation d'un modulateur de la voie de signalisation Notch dans la fabrication d'un médicament destiné à favoriser la tolérance à un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

14. Utilisation d'un modulateur de la voie de signalisation Notch dans la fabrication d'un médicament destiné à la réduction d'une réponse immunitaire à un allergène chez un mammifère dans une combinaison simultanée, contemporaine, séparée ou séquentielle avec : un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

15. Utilisation d'un modulateur de la voie de signalisation Notch dans la fabrication d'un médicament destiné à favoriser une tolérance immunitaire à un allergène ou un déterminant antigénique de celui-ci chez un mammifère dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Deltal, ou un polynucléotide codant pour celui-ci.

16. Modulateur de la voie de signalisation Notch pour une utilisation pour traiter une allergie dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

17. Combinaison i) d'un modulateur de la voie de signalisation Notch et ii) d'un allergène ou d'un déterminant antigénique de celui-ci ou d'un polynucléotide codant pour un allergène ou un déterminant antigénique de celui-ci pour une utilisation dans le traitement d'une allergie où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

18. Modulateur de la voie de signalisation Notch pour le traitement d'une allergie dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

19. Modulateur de la voie de signalisation Notch pour une utilisation dans le traitement d'une allergie où le modulateur de la voie de signalisation Notch comprend un ligand de Notch comprenant un domaine N-terminal, un domaine DSL et les 3 premières répétitions seulement de type EGF de Deltal, ou un polynucléotide codant pour celui-ci.

20. Modulateur de la voie de signalisation Notch pour une utilisation dans la réduction d'une réponse immunitaire à un allergène ou un déterminant antigénique
de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

21. Modulateur de la voie de signalisation Notch pour une utilisation destinée à favoriser la tolérance à un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

22. Modulateur de la voie de signalisation Notch pour une utilisation dans la réduction d'une réponse immunitaire à un allergène chez un mammifère dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

23. Modulateur de la voie de signalisation Notch pour une utilisation destinée à favoriser la tolérance immunitaire à un allergène ou un déterminant antigénique de celui-ci chez un mammifère dans une combinaison simultanée, contemporaine, séparée ou séquentielle avec un allergène ou un déterminant antigénique de celui-ci ou un polynucléotide codant pour un allergène ou un déterminant antigénique de celui-ci où le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.

24. Kit pharmaceutique ou vétérinaire comprenant un modulateur de la voie de signalisation Notch et un allergène ou un déterminant antigénique de celui-ci ou un polynucléotide codant pour un allergène ou un déterminant antigénique de celui-ci où
le modulateur de la voie de signalisation Notch comprend un ligand de Notch comprenant un domaine N-terminal, un domaine DSL et les 3 premières répétitions seulement de type EGF de Delta1, ou un polynucléotide codant pour celui-ci.
